# EUROPÄISCHE PATENTSCHRIFT

(11) **EP 1 572 663 B1**
(45) Veröffentlichungstag und Bekanntmachung des Hinweises auf die Patenterteilung: **20.03.2013**
(21) Anmeldenummer: 03795860.0
(22) Anmeldetag: 01.12.2003
(51) Int. Cl.: C07D 231/14, C07D 333/38, C07D 327/06, C07D 307/68, C07D 277/20, C07D 213/82, C07D 207/34, A01N 43/02, A01N 43/08, A01N 43/10, A01N 43/18, A01N 43/56

(54) **BIPHENYLCARBOXAMIDE**
BIPHENYLCARBOXAMIDES
BIPHENYLCARBOXAMIDES

(30) Priorität: 13.12.2002 DE 10258314
(43) Veröffentlichungstag der Anmeldung: 14.09.2005
(73) Patentinhaber: Bayer CropScience AG, 40789 Monheim (DE)
(72) Erfinder: DUNKEL, Ralf, 69001 Lyon (FR); ELBE, Hans-Ludwig, 42329 Wuppertal (DE); RIECK, Heiko, 69110 Ste. Foy les Lyon (FR); WACHENDORFF-NEUMANN, Ulrike, 56555 Neuwied (DE); DAHMEN, Peter, 41470 Neuss (DE); LÖSEL, Peter, 51371 Leverkusen (DE); KUCK, Karl-Heinz, 40764 Langenfeld (DE); MAULER-MACHNIK, Astrid, 42799 Leichlingen (DE); GREUL, Jörg, Nico, 42799 Leichlingen (DE)
(86) Internationale Anmeldenummer: PCT/EP2003/013498
(87) Internationale Veröffentlichungsnummer: WO 2004/054982

(56) Entgegenhaltungen:
- EP-A- 0 579 124
- WO-A-02/08195
- WO-A-02/08197

## Beschreibung

Die vorliegende Erfindung betrifft neue Biphenylcarboxamide, mehrere Verfahren zu deren Herstellung und deren Verwendung zur Bekämpfung von unerwünschten Mikroorganismen.

Es ist bereits bekannt, dass zahlreiche Carboxanilide fungizide Eigenschaften besitzen (vergleiche WO 93/11 117, WO 99/09 013, WO 00/14071, EP-A 0 545 099 und EP-A 0 589 301). Insbesondere sind aus WO 02/08195 und WO 02/08197 Oxyiminomethyl substituierte Carboxamide bekannt. Die Wirksamkeit dieser Stoffe ist gut, lässt aber in manchen Fällen zu wünschen übrig.

Es wurden nun neue Biphenylcarboxamide der Formel (I) 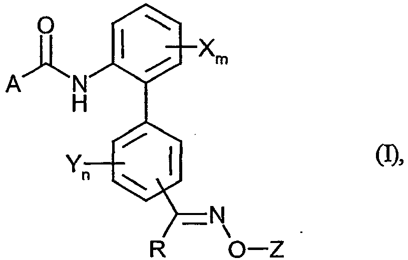 in welcher
- R: für Wasserstoff, C₁-C₆-Alkyl oder C₁-C₃-Halogenalkyl mit jeweils 1 bis 7 Fluor-, Chlor- und/oder Bromatomen steht,
- Z: für C₃-C₈-Alkenyl, C₃-C₈-Alkinyl, C₃-C₈-Halogenalkenyl, C₃-C₈-Halogenalkinyl mit jeweils 1 bis 5 Fluor-, Chlor- und/oder Bromatomen, oder (C₃-C₈-Cycloalkyl)-(C₁-C₄-alkyl) steht,
- X und Y: unabhängig voneinander für Halogen, Cyano, Nitro, C₁-C₈-Alkyl, C₁-C₈-Alkoxy, C₁-C₈-Alkylthio, C₁-C₆-Halogenalkyl, C₁-C₆-Halogenalkoxy oder C₁-C₆-Halogen-alkylthio mit jeweils 1 bis 13 Fluor-, Chlor- und/oder Bromatomen stehen,
- m: für 0, 1, 2, 3 oder 4 steht, wobei X für gleiche oder verschiedene Reste steht, wenn m für 2, 3 oder 4 steht,
- n: für 0, 1, 2, 3 oder 4 steht, wobei Y für gleiche oder verschiedene Reste steht, wenn n für 2, 3 oder 4 steht,
und
- A: für einen Rest der Formel 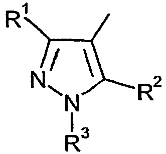 steht, worin
R¹ für Wasserstoff, Cyano, Halogen, Nitro, C₁-C₄-Alkyl, C₃-C₆-Cycloalkyl, C₁-C₄-Alkoxy, C₁-C₄-Alkylthio, Aminocarbonyl, Aminocarbonyl-C₁-C₄-alkyl oder für C₁-C₄-Halogenalkyl, C₁-C₄-Halogenalkoxy, C₁-C₄-Halo-genalkylthio mit jeweils 1 bis 5 Halogenatomen, steht und
R² für Wasserstoff, Halogen, Cyano, C₁-C₄-Alkyl, C₁-C₄-Alkoxy oder C₁-C₄-Alkylthio steht und
R³ für Wasserstoff, C₁-C₄-Alkyl, Hydroxy-C₁-C₄-alkyl, C₂-C₆-Alkenyl, C₃-C₆-Cycloalkyl, C₁-C₄- Alkylthio-C₁-C₄-alkyl, C₁-C₄-Alkox-y-C₁-C₄-alkyl, oder für C₁-C₄-Halogenalkyl, Halogen(C₁-C₄-alkylthio-C₁-C₄-alkyl), Halogen-(C₁-C₄-alkoxy-C₁-C₄-alkyl) mit jeweils 1 bis 5 Halogenatomen oder für Phenyl steht,
oder
- A: für einen Rest der Formel 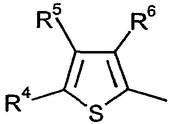 steht, worin
R⁴ und R⁵ unabhängig voneinander für Wasserstoff, Halogen, C₁-C₄-Alkyl oder C₁-C₄-Halogenalkyl mit 1 bis 5 Halogenatomen stehen und
R⁶ für Halogen, Cyano oder C₁-C₄-Alkyl, C₁-C₄-Halogenalkyl, C₁-C₄-Halogenalkoxy mit jeweils 1 bis 5 Halogenatomen steht,
oder
- A: für einen Rest der Formel 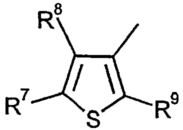 steht, worin
R⁷ und R⁸ unabhängig voneinander für Wasserstoff, Halogen, C₁-C₄-Alkyl oder C₁-C₄-Halogenalkyl mit 1 bis 5 Halogenatomen stehen und
R⁹ für Wasserstoff, Halogen oder C₁-C₄-Alkyl steht,
oder
- A: für einen Rest der Formel 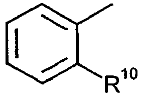 steht, worin
R¹⁰ für Wasserstoff, Halogen, Hydroxy, Cyano, C₁-C₆-Alkyl, C₁-C₄-Halogenalkyl, C₁-C₄-Halogenalkoxy, C₁-C₄-Halogenalkylthio mit jeweils 1 bis 5 Halogenatomen steht,
oder
- A: für einen Rest der Formel 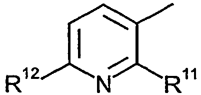 steht, worin
R¹¹ für Halogen, Hydroxy, Cyano, C₁-C₄-Alkyl, C₁-C₄-Alkoxy, C₁-C₄-Alkyl-thio, C₁-C₄-Halogenalkyl, C₁-C₄-Halogenalkoxy, C₁-C₄-Halogenalkylthio mit jeweils 1 bis 5 Halogenatomen steht und
R¹² für Wasserstoff, Halogen, Cyano, C₁-C₄-Alkyl, C₁-C₄-Alkoxy, C₁-C₄-A1-kylthio, C₁-C₄-Alkylsulfinyl, C₁-C₄-Alkylsulfonyl oder für C₁-C₄-Halogenalkyl, C₁-C₄-Halogenalkoxy mit jeweils 1 bis 5 Halogenatomen steht,
oder
- A: für einen Rest der Formel 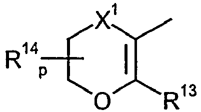 steht, worin
R¹³ für C₁-C₄-Alkyl oder für C₁-C₄-Halogenalkyl mit 1 bis 5 Halogenatomen steht,
R¹⁴ für C₁-C₄-Alkyl steht,
X¹ für S (Schwefel), für SO, SO₂ oder CH₂ steht und
p für 0, 1 oder 2 steht,
oder
- A: für einen Rest der Formel 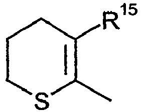 steht, worin
R¹⁵ für C₁-C₄-Alkyl oder für C₁-C₄-Halogenalkyl mit 1 bis 5 Halogenatomen steht,
oder
- A: für einen Rest der Formel 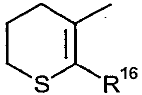 steht, worin
R¹⁶ für C₁-C₄-Alkyl oder für C₁-C₄-Halogenalkyl mit 1 bis 5 Halogenatomen steht,
oder
- A: für einen Rest der Formel 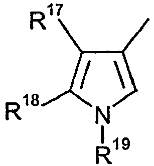 steht, worin
R¹⁷ für Halogen, Cyano, C₁-C₄-Alkyl oder für C₁-C₄-Halogenalkyl mit 1 bis 5 Halogenatomen steht,
R¹⁸ für Wasserstoff, Halogen, C₁-C₄-Alkyl oder für C₁-C₄-Halogenalkyl mit 1 bis 5 Halogenatomen steht,
R¹⁹ für Wasserstoff, Cyano, C₁-C₄-Alkyl, C₁-C₄-Halogenalkyl mit 1 bis 5 Halogenatomen, C₁-C₄-Alkoxy-C₁-C₄-alkyl, Hydroxy-C₁-C₄-alkyl, C₁-C₄-Alkylsulfonyl, Di(C₁-C₄-alkyl)aminosulfonyl, C₁-C₆-Alkylcarbonyl oder für gegebenenfalls substituiertes Phenylsulfonyl oder Benzoyl steht,
oder
- A: für einen Rest der Formel 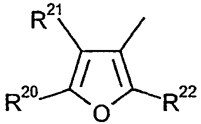 steht, worin
R²⁰ und R²¹ unabhängig voneinander für Wasserstoff, Halogen, Amino, C₁-C₄-Alkyl oder für C₁-C₄-Halogenalkyl mit 1 bis 5 Halogenatomen stehen und
R²² für Wasserstoff, Halogen, C₁-C₄-Alkyl oder für C₁-C₄-Halogenalkyl mit 1 bis 5 Halogenatomen steht,
oder
- A: für einen Rest der Formel 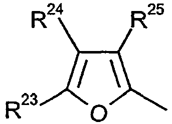 steht, worin
R²³ und R²⁴ unabhängig voneinander für Wasserstoff, Halogen, Amino, Nitro, C₁-C₄-Alkyl oder für C₁-C₄-Halogenalkyl mit 1 bis 5 Halogenatomen stehen und
R²⁵ für Wasserstoff, Halogen, C₁-C₄-Alkyl oder für C₁-C₄-Halogenalkyl mit 1 bis 5 Halogenatomen steht,
oder
- A: für einen Rest der Formel 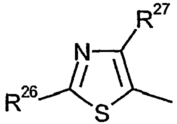 steht, worin
R²⁶ für Wasserstoff, Halogen, Amino, C₁-C₄-Alkylamino, Di-(C₁-C₄-alkyl)amino, Cyano, C₁-C₄-Akyl oder für C₁-C₄-Halogenalkyl mit 1 bis 5 Halogenatomen steht und
R²⁷ für Halogen, C₁-C₄-Alkyl oder C₁-C₄-Halogenalkyl mit 1 bis 5 Halogenatomen steht,
oder
- A: für einen Rest der Formel 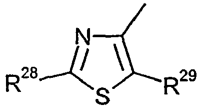 steht, worin
R²⁸ für Wasserstoff, Halogen, Amino, C₁-C₄-Alkylamino, Di-(C₁-C₄-alkyl)amino, Cyano, C₁-C₄-Alkyl oder für C₁-C₄-Halogenalkyl mit 1 bis 5 Halogenatomen steht und
R²⁹ für Halogen, C₁-C₄-Alkyl oder C₁-C₄-Halogenalkyl mit 1 bis 5 Halogenatomen steht,
oder
- A: für einen Rest der Formel 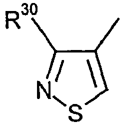 steht, worin
R³⁰ für Halogen, C₁-C₄-Alkyl oder C₁-C₄-Halogenalkyl mit 1 bis 5 Halogenatomen steht,
oder
- A: für einen Rest der Formel 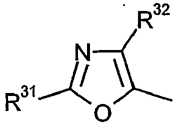 steht, worin
R³¹ für Wasserstoff oder C₁-C₄-Alkyl steht und
R³² für Halogen oder C₁-C₄-Alkyl steht,
oder
- A: für einen Rest der Formel 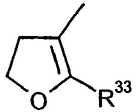 steht, worin
R³³ für C₁-C₄-Alkyl oder C₁-C₄-Halogenalkyl mit 1 bis 5 Halogenatomen steht,
oder
- A: für einen Rest der Formel 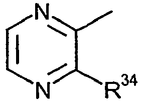 steht, worin
R³⁴ für Wasserstoff, Halogen, C₁-C₄-Alkyl oder C₁-C₂-Halogenalkyl mit 1 bis 5 Halogenatomen steht,
gefunden.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Insbesondere in Bezug auf die Oxim-Doppelbindung treten E/Z-Isomere auf, wenn R für C₁-C₆-Alkyl steht. Diese Isomere liegen in der Regel im Gemisch vor. Die im Folgenden in Strukturformeln angegebene Konfiguration umfasst immer beide Möglichkeiten. Der Einfachheit halber ist jeweils nur ein Isomer abgebildet.

Weiterhin wurde gefunden, dass man Biphenylcarboxamide der Formel (I) erhält, indem man
a) Carbonsäure-Derivate der Formel (II) 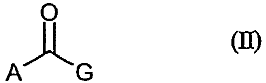 in welcher
   - A: die oben angegebenen Bedeutungen hat und
   - G: für Halogen, Hydroxy oder C₁-C₆-Alkoxy steht,
   mit Anilin-Derivaten der Formel (III) 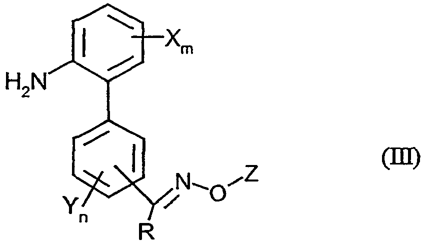 in welcher
   R, Z, X, Y, m und n die oben angegebenen Bedeutungen haben,
   gegebenenfalls in Gegenwart eines Katalysators, gegebenenfalls in Gegenwart eines Säurebindemittels und gegebenenfalls in Gegenwart eines Verdünnungsmittels umsetzt,
   oder
b) Carboxamid-Derivate der Formel (IV) 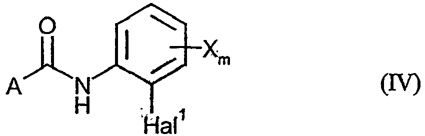 in welcher
   - A, X und m: die oben angegebenen Bedeutungen haben,
   - Hal¹: für Brom oder Iod steht,
   mit Boronsäure-Derivaten der-Formel (V) 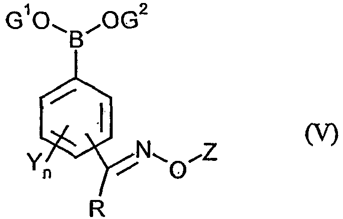 in welcher
   R, Z, Y und n die oben angegebenen Bedeutungen haben und
   G¹ und G² jeweils für Wasserstoff oder zusammen für Tetramethylethylen stehen,
   in Gegenwart eines Katalysators, gegebenenfalls in Gegenwart eines Säurebindemittels und gegebenenfalls in Gegenwart eines Verdünnungsmittels umsetzt,
   oder
c) Carboxamid-Boronsäure-Derivate der Formel (VI) 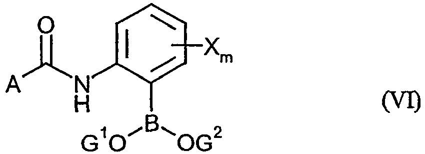 in welcher
   A, X und m die oben angegebenen Bedeutungen haben und
   G¹ und G² jeweils für Wasserstoff oder zusammen für Tetramethylethylen stehen,
   mit Phenyloxim-Derivaten der Formel (VII) 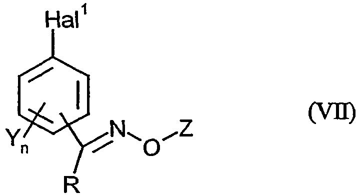 in welcher
   - R, Z, Y und n: die oben angegebenen Bedeutungen haben,
   - Hal¹: für Brom oder Iod steht,
   in Gegenwart eines Katalysators, gegebenenfalls in Gegenwart eines Säurebindemittels und gegebenenfalls in Gegenwart eines Verdünnungsmittels umsetzt, oder
d) Biphenylacyl-Derivate der Formel (VIII) 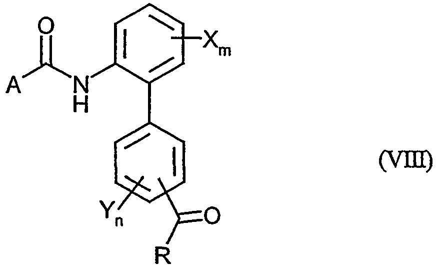 in welcher
   A, R, X, Y, m und n die oben angegebenen Bedeutungen haben,
   mit Hydroxylamin-Derivaten der Formel (IX)

   Z-O-NH₂ x HCl (IX)

   in welcher
   Z die oben angegebenen Bedeutungen hat,
   gegebenenfalls in Gegenwart eines Säurebindemittels und gegebenenfalls in Gegenwart eines Verdünnungsmittels umsetzt,
   oder
e) Hydroxyimino-Derivate der Formel (I-a) 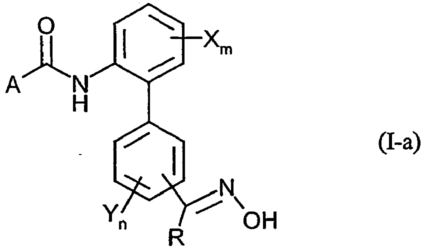 in welcher
   A, R, X, Y, m und n die oben angegebenen Bedeutungen haben,
   mit Verbindungen der Formel (X)

   Z-E (X)

   in welcher
   - Z: die oben angegebenen Bedeutungen hat,
   - E: für Chlor, Brom, Iod, Methansulfonyl oder p-Toluolsulfonyl steht,
   gegebenenfalls in Gegenwart eines Säurebindemittels und gegebenenfalls in Gegenwart eines Verdünnungsmittels umsetzt,
   oder
f) Carboxamid-Derivate der Formel (IV) 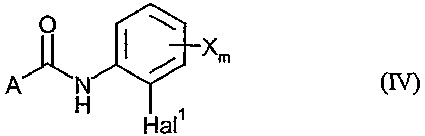 in welcher
   A, X und m die oben angegebenen Bedeutungen haben,
   Hal¹ für Brom oder Iod steht,
   mit Phenyloxim-Derivaten der Formel (VII) 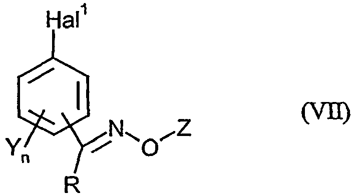 in welcher
   - R, Z, Y und n: die oben angegebenen Bedeutungen haben,
   - Hal¹: für Brom oder Iod steht,
   in Gegenwart eines Palladium- oder Platin-Katalysators und in Gegenwart von 4,4,4',4',5,5,5',5'-Octamethyl-2,2'-bis-1,3,2-dioxaborolan, gegebenenfalls in Gegenwart eines Säurebindemittels und gegebenenfalls in Gegenwart eines Verdünnungsmittels umsetzt.

Schließlich wurde gefunden, dass die neuen Biphenylcarboxamide der Formel (I) sehr gute mikrobizide Eigenschaften besitzen und zur Bekämpfung unerwünschter Mikroorganismen sowohl im Pflanzenschutz als auch im Materialschutz verwendbar sind.

Überraschenderweise zeigen die erfindungsgemäßen Biphenylcarboxamide der Formel (I) eine wesentlich bessere fungizide Wirksamkeit als die konstitutionell ähnlichsten, vorbekannten Wirkstoffe gleicher Wirkungsrichtung.

Die erfindungsgemäßen Biphenylcarboxamide sind durch die Formel (I) allgemein definiert.
- R: steht bevorzugt für Wasserstoff, C₁-C₄-Alkyl oder C₁-C₃-Halogenalkyl mit jeweils 1 bis 7 Fluor-, Chlor- und/oder Bromatomen.
- Z: steht bevorzugt für C₃-C₆-Alkenyl, C₃-C₆-Alkinyl, C₃-C₆-Halogenalkenyl, C₃-C₆-Halogenalkinyl mit jeweils 1 bis 5 Fluor-, Chlor- und/oder Bromatomen, oder (C₃-C₆-Cycloalkyl)-(C₁-C₄-alkyl).
- X und Y: stehen unabhängig voneinander bevorzugt für Fluor, Chlor, Brom, Cyano, Nitro, C₁-C₆-Alkyl, C₁-C₆-Alkoxy, C₁-C₆-Alkylthio, C₁-C₂-Halogenalkyl, C₁-C₂-Halogenalkoxy oder C₁-C₂-Halogenalkylthio mit jeweils 1 bis 5 Fluor-, Chlor und/oder Bromatomen.
- m: steht bevorzugt für 0, 1, 2 oder 3, wobei X für gleiche oder verschiedene Reste steht, wenn m für 2 oder 3 steht.
- n: steht bevorzugt für 0, 1, 2 oder 3, wobei Y für gleiche oder verschiedene Reste steht, wenn n für 2 oder 3 steht.
- A: steht bevorzugt für einen Rest der Formel 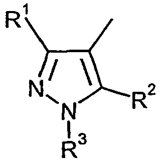 worin
R¹ für Wasserstoff, Cyano, Fluor, Chlor, Brom, Iod, Methyl, Ethyl, Isopropyl, Cyclopropyl, Methoxy, Ethoxy, Methylthio, Ethylthio, Aminocarbonyl, Aminocarbonylmethyl, Aminocarbonylethyl, C₁-C₇-Halogenalkyl C₁-C₂-Halogenalkoxy mit jeweils 1 bis 5 Fluor-, Chlor- und/ oder Bromatomen, Trifluormethylthio oder Difluormethylthio steht,
R² für Wasserstoff, Fluor, Chlor, Brom, Iod, Methyl, Ethyl, Methoxy, Ethoxy, Methylthio oder Ethylthio steht und
R³ für Wasserstoff, Methyl, Ethyl, n-Propyl, Isopropyl, Hydroxymethyl, Hydroxyethyl, Cyclopropyl, Cyclopentyl, Cyclohexyl, C₁-C₂-Halogenalkyl mit 1 bis 5 Fluor-, Chlor und/oder Bromatomen oder für Phenyl steht.
- A: steht außerdem bevorzugt für einen Rest der Formel 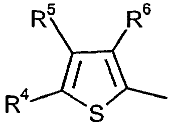 worin
R⁴ und R⁵ unabhängig voneinander für Wasserstoff, Fluor, Chlor, Brom, Methyl, Ethyl oder C₁-C₂-Halogenalkyl mit 1 bis 5 Fluor-, Chlor- und/oder Bromatomen stehen und
R⁶ für Fluor, Chlor, Brom, Iod, Cyano, Methyl, Ethyl, Trifluormethyl oder C₁-C₂-Halogenalkoxy mit 1 bis 5 Fluor-, Chlor- und/oder Bromatomen steht.
- A: steht außerdem bevorzugt für einen Rest der Formel 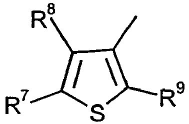 worin
R⁷ und R⁸ unabhängig voneinander für Wasserstoff, Fluor, Chlor, Brom, Methyl, Ethyl oder C₁-C₂-Halogenalkyl mit 1 bis 5 Fluor-, Chlor- und/oder Bromatomen stehen und
R⁹ für Wasserstoff, Fluor, Chlor, Brom, Methyl oder Ethyl steht.
- A: steht außerdem bevorzugt für einen Rest der Formel 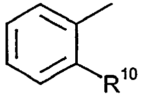 worin
R¹⁰ für Wasserstoff, Fluor, Chlor, Brom, Iod, Hydroxy, Cyano, C₁-C₄-Alkyl, C₁-C₂-Halogenalkyl, C₁-C₂-Halogenalkoxy, C₁-C₂-Halogenalkylthio mit jeweils 1 bis 5 Fluor-, Chlor- und/oder Bromatomen steht.
- A: steht außerdem bevorzugt für einen Rest der Formel 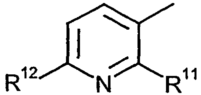 worin
R¹¹ für Fluor, Chlor, Brom, Iod, Hydroxy, Cyano, C₁-C₄-Alkyl, Methoxy, Ethoxy, Methylthio, Ethylthio, C₁-C₂-Halogenalkyl, C₁-C₂-Halogenalkoxy mit jeweils 1 bis 5 Fluor-, Chlor- und/oder Bromatomen, Trifluormethylthio, Difluormethylthio steht und
R¹² für Wasserstoff, Fluor, Chlor, Brom, Iod, Cyano, C₁-C₄-Alkyl, Methoxy, Ethoxy, Methylthio, Ethylthio, C₁-C₂-Alkylsulfinyl, C₁-C₂-Alkylsulfonyl, C₁-C₂-Halogenalkyl, C₁-C₂-Halogenalkoxy mit jeweils 1 bis 5 Fluor-, Chlor- und/oder Bromatomen steht.
- A: steht außerdem bevorzugt für einen Rest der Formel 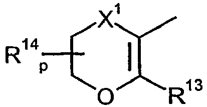 worin
R¹³ für Methyl, Ethyl oder für C₁-C₂-Halogenalkyl mit 1 bis 5 Fluor-, Chlor- und/oder Bromatomen steht und
R¹⁴ für Methyl oder Ethyl steht,
X¹ für S (Schwefel), für SO, SO₂ oder CH₂ steht und
p für 0, 1 oder 2 steht.
- A: steht außerdem bevorzugt für einen Rest der Formel 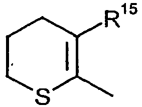 worin
R¹⁵ für Methyl, Ethyl oder für C₁-C₂-Halogenalkyl mit 1 bis 5 Fluor-, Chlor- und/oder Bromatomen steht.
- A: steht außerdem bevorzugt für einen Rest der Formel 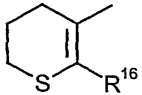 worin
R¹⁶ für Methyl, Ethyl oder für C₁-C₂-Halogenalkyl mit 1 bis 5 Fluor-, Chlor- und/oder Bromatomen steht.
- A: steht außerdem bevorzugt für einen Rest der Formel 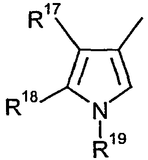 worin
R¹⁷ für Fluor, Chlor, Brom, Cyano, Methyl, Ethyl, Isopropyl oder für C₁-C₂-Halogenalkyl mit 1 bis 5 Fluor-, Chlor- und/oder Bromatomen steht,
R¹⁸ für Wasserstoff, Fluor, Chlor, Brom, Methyl, Ethyl oder für C₁₋C₂-Halogenalkyl mit 1 bis 5 Fluor-, Chlor- und/oder Bromatomen steht und
R¹⁹ für Wasserstoff, Methyl, Ethyl, C₁-C₂-Halogenalkyl mit 1 bis 5 Fluor-, Chlor- und/oder Bromatomen, C₁-C₂-Alkoxy-C₁-C₂-alkyl, Hydroxymethyl, Hydroxyethyl, Methylsulfonyl oder Dimethylaminosulfonyl steht.
- A: steht außerdem bevorzugt für einen Rest der Formel 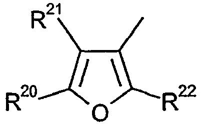 worin
R²⁰ und R²¹ unabhängig voneinander für Wasserstoff, Fluor, Chlor, Brom, Amino, Methyl, Ethyl oder für C₁-C₂-Halogenalkyl mit 1 bis 5 Fluor-, Chlor- und/oder Bromatomen stehen und
R²² für Wasserstoff, Fluor, Chlor, Brom, Methyl, Ethyl oder für C₁-C₂-Halogenalkyl mit 1 bis 5 Fluor-, Chlor- und/oder Bromatomen steht.
- A: steht außerdem bevorzugt für einen Rest der Formel 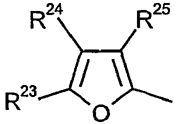 worin
R²³ und R²⁴ unabhängig voneinander für Wasserstoff, Fluor, Chlor, Brom, Amino, Nitro, Methyl, Ethyl oder für C₁-C₂-Halogenalkyl mit 1 bis 5 Fluor-, Chlor- und/oder Bromatomen stehen und
R²⁵ für Wasserstoff, Fluor, Chlor, Brom, Methyl, Ethyl oder für C₁-C₂-Halogenalkyl mit 1 bis 5 Fluor-, Chlor- und/oder Bromatomen steht.
- A: steht außerdem bevorzugt für einen Rest der Formel 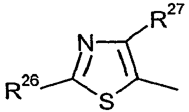 worin
R²⁶ für Wasserstoff, Fluor, Chlor, Brom, Amino, C₁-C₄-Alkylamino, Di-(C₁-C₄-alkyl)amino, Cyano, Methyl, Ethyl oder für C₁-C₂-Halogenalkyl mit 1 bis 5 Fluor-, Chlor- und/oder Bromatomen steht und
R²⁷ für Fluor, Chlor, Brom, Methyl, Ethyl oder C₁-C₂-Halogenalkyl mit 1 bis 5 Fluor-, Chlor- und/oder Bromatomen steht.
- A: steht außerdem bevorzugt für einen Rest der Formel 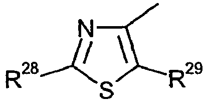 worin
R²⁸ für Wasserstoff, Fluor, Chlor, Brom, Amino, C₁-C₄-Alkylamino, Di-(C₁-C₄-alkyl)amino, Cyano, Methyl, Ethyl oder für C₁-C₂-Halogenalkyl mit 1 bis 5 Fluor-, Chlor- und/oder Bromatomen steht und
R²⁹ für Fluor, Chlor, Brom, Methyl, Ethyl oder C₁-C₂-Halogenalkyl mit 1 bis 5 Fluor-, Chlor- und/oder Bromatomen steht.
- A: steht außerdem bevorzugt für einen Rest der Formel 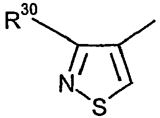 worin
R³⁰ für Fluor, Chlor, Brom, Methyl, Ethyl oder C₁-C₂-Halogenalkyl mit 1 bis 5 Fluor-, Chlor- und/oder Bromatomen steht.
- A: steht außerdem bevorzugt für einen Rest der Formel 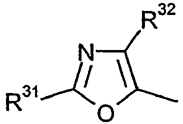 worin
R³¹ für Wasserstoff, Methyl oder Ethyl steht und
R³² für Fluor, Chlor, Brom, Methyl oder Ethyl steht.
- A: steht außerdem bevorzugt für einen Rest der Formel 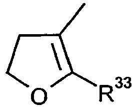 worin
R³³ für Methyl, Ethyl oder C₁-C₂-Halogenalkyl mit 1 bis 5 Fluor-, Chlor- und/oder Bromatomen steht
- A: steht außerdem bevorzugt für einen Rest der Formel 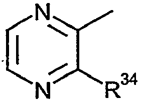 worin
R³⁴ für Wasserstoff, Fluor, Chlor, Brom, Methyl, Ethyl oder Trifluormethyl steht.
- R: steht besonders bevorzugt für Wasserstoff, Methyl, Ethyl, Isopropyl, tert.-Butyl.
- Z: steht besonders bevorzugt für Allyl, 2-Butenyl, 2-Methyl-allyl, 1-Methyl-allyl, 3-Methyl-2-butenyl, Propargyl, 2-Butinyl, 3-Butinyl, 2-Methyl-3-butinyl, 3,3-Difluorallyl, 3,3-Dichlorallyl, Cyclopropylmethyl, Cyclopentylmethyl, Cyclohexylmethyl.
- X und Y: stehen unabhängig voneinander besonders bevorzugt für Fluor, Chlor, Brom, Cyano, Nitro, Methyl, Ethyl, n-Propyl, Isopropyl, n-Butyl, sek.-Butyl, iso-Butyl, tert.-Butyl, Methoxy, Ethoxy, Methylthio, Trichlormethyl, Trifluormethyl, Difluormethyl, Difluorchlormethyl, Difluormethoxy, Trifluormethoxy, Trifluormethylthio, Difluorchlormethylthio.
- m: steht besonders bevorzugt für 0 oder 1.
- n: steht besonders bevorzugt für 0, 1 oder 2, wobei Y für gleiche oder verschiedene Reste steht, wenn n für 2 steht.
- A: steht besonders bevorzugt für einen Rest der Formel 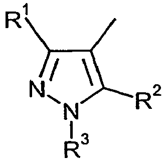 worin
R¹ für Wasserstoff, Fluor, Chlor, Brom, Iod, Methyl, Ethyl, Isopropyl, Cyclopropyl, Methoxy, Ethoxy, Methylthio, Ethylthio, Monofluormethyl, Difluormethyl, Trifluormethyl, Difluorchlormethyl, Trichlormethyl, Trifluormethoxy, Trichlormethoxy, Trifluormethylthio oder Difluormethylthio steht und
R² für Wasserstoff, Fluor, Chlor, Brom, Iod, Methyl, Ethyl, Methoxy, Ethoxy, Methylthio oder Ethylthio steht und
R³ für Wasserstoff, Methyl, Ethyl, Hydroxymethyl, Hydroxyethyl, Trifluormethyl, Difluormethyl oder Phenyl steht.
- A: steht außerdem besonders bevorzugt für einen Rest der Formel 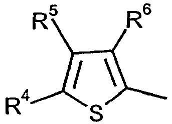 worin
R⁴ und R⁵ unabhängig voneinander für Wasserstoff, Fluor, Chlor, Brom, Methyl, Ethyl, Difluormethyl, Trifluormethyl, Difluorchlormethyl oder Trichlormethyl stehen und
R⁶ für Fluor, Chlor, Brom, Cyano, Methyl, Trifluormethyl, Trifluormethoxy, Difluormethoxy, Difluorchlormethoxy oder Trichlormethoxy steht.
- A: steht außerdem besonders bevorzugt für einen Rest der Formel 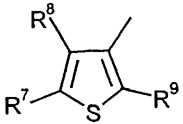 worin
R⁷ und R⁸ unabhängig voneinander für Wasserstoff, Fluor, Chlor, Brom, Methyl, Ethyl, Difluormethyl, Trifluormethyl, Difluorchlormethyl oder Trichlormethyl stehen und
R⁹ für Wasserstoff, Fluor, Chlor, Brom, Methyl oder Ethyl steht.
- A: steht außerdem besonders bevorzugt für einen Rest der Formel 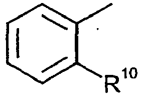 worin
R¹⁰ für Wasserstoff, Fluor, Chlor, Brom, Iod, Hydroxy, Cyano, Methyl, Ethyl, n-Propyl, Isopropyl, n-Butyl, iso-Butyl, sek.-Butyl, tert.-Butyl, Difluormethyl, Trifluormethyl, Difluorchlormethyl, Trichlormethyl, Trifluormethoxy, Difluormethoxy, Difluorchlormethoxy, Trichlormethoxy, Trifluormethylthio, Difluormethylthio, Difluorchlormethylthio oder Trichlormethylthio steht.
- A: steht außerdem besonders bevorzugt für einen Rest der Formel 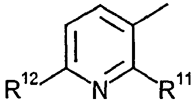 worin
R¹¹ für Fluor, Chlor, Brom, Iod, Hydroxy, Cyano, Methyl, Ethyl, n-Propyl, Isopropyl, n-Butyl, iso-Butyl, sek.-Butyl, tert.-Butyl, Methoxy, Ethoxy, Methylthio, Ethylthio, Trifluormethyl, Difluormethyl, Difluorchlormethyl, Trichlormethyl, Trifluormethoxy, Difluormethoxy, Difluorchlormethoxy, Trichlormethoxy, Difluormethylthio, Trifluormethylthio steht und
R¹² für Wasserstoff, Fluor, Chlor, Brom, Iod, Cyano, Methyl, Ethyl, n-Propyl, Isopropyl, n-Butyl, iso-Butyl, sek.-Butyl, tert.-Butyl, Methoxy, Ethoxy, Methylthio, Ethylthio, Methylsulfinyl, Methylsulfonyl, Trifluormethyl, Difluormethyl, Difluorchlormethyl, Trichlormethyl, Trifluormethoxy, Difluormethoxy, Difluorchlormethoxy oder Trichlormethoxy steht.
- A: steht außerdem besonders bevorzugt für einen Rest der Formel 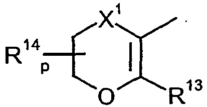 worin
R¹³ für Methyl, Ethyl, Trifluormethyl, Difluormethyl, Difluorchlormethyl oder Trichlormethyl steht und
R¹⁴ für Methyl oder Ethyl steht,
X¹ für S (Schwefel), für SO, SO₂ oder CH₂ steht und
p für 0, 1 oder 2 steht.
- A: steht außerdem besonders bevorzugt für einen Rest der Formel 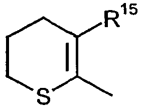 worin
R¹⁵ für Methyl, Ethyl, Trifluormethyl, Difluormethyl, Difluorchlormethyl oder Trichlormethyl steht.
- A: steht außerdem besonders bevorzugt für einen Rest der Formel 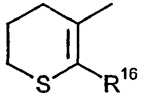 worin
R¹⁶ für Methyl, Ethyl, Trifluormethyl, Difluormethyl, Difluorchlormethyl oder Trichlormethyl steht.
- A: steht außerdem besonders bevorzugt für einen Rest der Formel 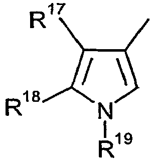 worin
R¹⁷ für Fluor, Chlor, Brom, Cyano, Methyl, Ethyl, Isopropyl, Trifluormethyl, Difluormethyl, Difluorchlormethyl oder Trichlormethyl steht,
R¹⁸ für Wasserstoff, Fluor, Chlor, Brom, Methyl, Ethyl, Trifluormethyl, Difluormethyl oder Trichlormethyl steht und
R¹⁹ für Wasserstoff, Methyl, Ethyl, Trifluormethyl, Methoxymethyl, Ethoxymethyl, Hydroxymethyl oder Hydroxyethyl steht
- A: steht außerdem besonders bevorzugt für einen Rest der Formel 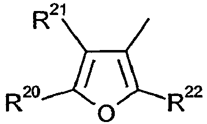 worin
R²⁰ und R²¹ unabhängig voneinander für Wasserstoff, Fluor, Chlor, Brom, Methyl, Ethyl, Trifluormethyl, Difluormethyl, Difluorchlormethyl oder Trichlormethyl stehen und
R²² für Wasserstoff, Fluor, Chlor, Brom, Methyl, Ethyl, Trifluormethyl, Difluormethyl, Difluorchlormethyl oder Trichlormethyl steht.
- A: steht außerdem besonders bevorzugt für einen Rest der Formel 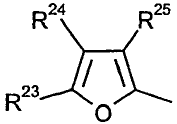 worin
R²³ und R²⁴ unabhängig voneinander für Wasserstoff, Fluor, Chlor, Brom, Nitro, Methyl, Ethyl, Trifluormethyl, Difluormethyl, Difluorchlormethyl oder Trichlormethyl stehen und
R²⁵ für Wasserstoff, Fluor, Chlor, Brom, Methyl, Ethyl, Trifluormethyl, Difluormethyl, Difluorchlormethyl oder Trichlormethyl steht
- A: steht außerdem besonders bevorzugt für einen Rest der Formel 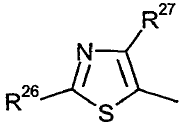 worin
R²⁶ für Wasserstoff, Fluor, Chlor, Brom, Amino, Methylamino, Dimethylamino, Cyano, Methyl, Ethyl, Trifluormethyl, Difluormethyl, Difluorchlormethyl oder Trichlormethyl steht und
R²⁷ für Fluor, Chlor, Brom, Methyl, Ethyl, Trifluormethyl, Difluormethyl, Difluorchlormethyl oder Trichlormethyl steht.
- A: steht außerdem besonders bevorzugt für einen Rest der Formel 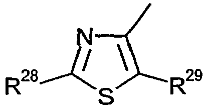 worin
R²⁸ für Wasserstoff, Fluor, Chlor, Brom, Amino, Methylamino, Dimethylamino, Cyano, Methyl, Ethyl, Trifluormethyl, Difluormethyl, Difluorchlormethyl oder Trichlormethyl steht und
R²⁹ für Fluor, Chlor, Brom, Methyl, Ethyl, Trifluormethyl, Difluormethyl, Difluorchlormethyl oder Trichlormethyl steht.
- A: steht außerdem besonders bevorzugt für einen Rest der Formel 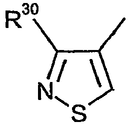 worin
R³⁰ für Fluor, Chlor, Brom, Methyl, Ethyl, Trifluormethyl, Difluormethyl, Difluorchlormethyl oder Trichlormethyl steht.
- A: steht außerdem besonders bevorzugt für einen Rest der Formel 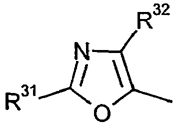 worin
R³¹ für Wasserstoff, Methyl oder Ethyl steht und
R³² für Fluor, Chlor, Brom, Methyl oder Ethyl steht.
- A: steht außerdem besonders bevorzugt für einen Rest der Formel 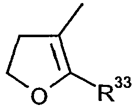 worin
R³³ für Methyl, Ethyl, Trifluormethyl, Difluormethyl, Difluorchlormethyl oder Trichlormethyl steht.
- A: steht außerdem besonders bevorzugt für einen Rest der Formel 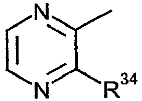 worin
R³⁴ für Wasserstoff, Fluor, Chlor, Brom, Methyl, Ethyl oder Trifluormethyl steht.
- R: steht ganz besonders bevorzugt für Wasserstoff oder Methyl.
- Z: steht ganz besonders bevorzugt für Allyl, 2-Butenyl, 2-Methyl-allyl, 1-Methyl-allyl, 3-Methyl-2-butenyl, Propargyl, 2-Butinyl, 2-Methyl-3-butinyl, 3,3-Difluorallyl, Cyclopropylmethyl.
- X: steht ganz besonders bevorzugt für Fluor oder Methyl.
- Y: steht ganz besonders bevorzugt für Fluor, Chlor, Brom, Cyano, Methyl, Methoxy, Methylthio, Trichlormethyl, Trifluormethyl, Trifluormethoxy, Trifluormethylthio.
- m: steht ganz besonders bevorzugt für 0 oder 1.
- n: steht ganz besonders bevorzugt für 0 oder 1.
- A: steht ganz besonders bevorzugt für einen Rest der Formel 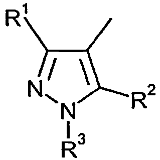 worin
R¹ für Fluor, Chlor, Brom, Iod, Methyl, Isopropyl, Cyclopropyl, Monofluormethyl, Difluormethyl oder Trifluormethyl steht und
R² für Wasserstoff, Fluor, Chlor oder Methyl steht und
R³ für Methyl steht.
- A: steht außerdem ganz besonders bevorzugt für einen Rest der Formel 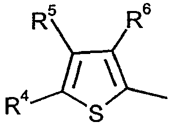 worin
R⁴ und R⁵ unabhängig voneinander für Wasserstoff, Fluor oder Methyl stehen und
R⁶ für Methyl steht.
- A: steht außerdem ganz besonders bevorzugt für einen Rest der Formel 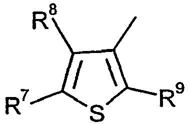 worin
R⁷ und R⁸ unabhängig voneinander für Wasserstoff, Fluor oder Methyl stehen und
R⁹ für Methyl steht.
- A: steht außerdem ganz besonders bevorzugt für einen Rest der Formel 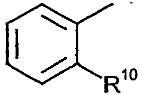 worin
R¹⁰ für Iod, Methyl, Difluormethyl oder Trifluormethyl steht,
R¹⁰ außerdem für Chlor oder Brom steht.
- A: steht außerdem ganz besonders bevorzugt für einen Rest der Formel 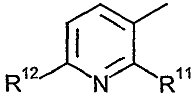 worin
R¹¹ für Fluor, Chlor, Brom, Methyl oder Trifluormethyl steht und
R¹² für Wasserstoff steht.
- A: steht außerdem ganz besonders bevorzugt für einen Rest der Formel 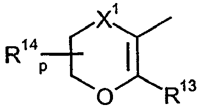 worin
R¹³ für Methyl oder Trifluonnethyl steht und
R¹⁴ für Methyl steht,
X¹ für S (Schwefel) oder CH₂ steht und
p für 0 oder 1 steht.
- A: steht außerdem ganz besonders bevorzugt für einen Rest der Formel 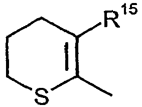 worin
R¹⁵ für Methyl, Trifluormethyl oder Difluormethyl steht.
- A: steht außerdem ganz besonders bevorzugt für einen Rest der Formel 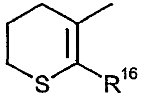 worin
R¹⁶ für Methyl, Trifluormethyl oder Difluormethyl steht.
- A: steht außerdem ganz besonders bevorzugt für einen Rest der Formel 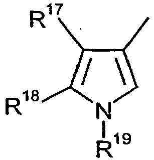 worin
R¹⁷ für Fluor, Methyl oder Trifluormethyl steht,
R¹⁸ für Wasserstoff oder Methyl steht und
R¹⁹ für Methyl oder Methoxymethyl steht.
- A: steht außerdem ganz besonders bevorzugt für einen Rest der Formel 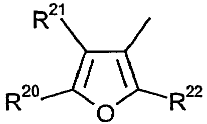 worin
R²⁰ und R²¹ unabhängig voneinander für Wasserstoff, Fluor oder Methyl stehen und
R²² für Methyl oder Trifluormethyl steht.
- A: steht außerdem ganz besonders bevorzugt für einen Rest der Formel 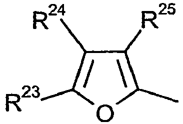 worin
R²³ und R²⁴ unabhängig voneinander für Wasserstoff, Fluor oder Methyl stehen und
R²⁵ für Methyl steht.
- A: steht außerdem ganz besonders bevorzugt für einen Rest der Formel 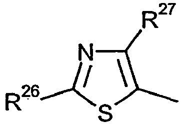 worin
R²⁶ für Fluor, Chlor, Amino oder Methyl steht und
R²⁷ für Chlor, Methyl, Trifluormethyl oder Difluormethyl steht.
- A: steht außerdem ganz besonders bevorzugt für einen Rest der Formel 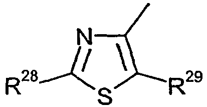 worin
R²⁸ für Fluor, Chlor, Amino oder Methyl steht und
R²⁹ für Chlor, Methyl, Trifluormethyl oder Difluormethyl steht
- A: steht außerdem ganz besonders bevorzugt für einen Rest der Formel 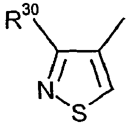 worin
R³⁰ für Chlor, Methyl, Trifluormethyl oder Difluormethyl steht.
- A: steht außerdem ganz besonders bevorzugt für einen Rest der Formel 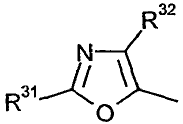 worin
R³¹ für Methyl steht und
R³² für Chlor oder Methyl steht.
- A: steht außerdem ganz besonders bevorzugt für einen Rest der Formel 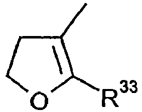 worin
R³³ für Methyl, Trifluormethyl oder Difluormethyl steht.
- A: steht außerdem ganz besonders bevorzugt für einen Rest der Formel 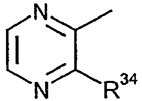 worin
R³⁴ für Wasserstoff, Chlor oder Methyl steht.
- R: steht insbesondere ganz besonders bevorzugt für Wasserstoff oder Methyl.
- Z: steht insbesondere ganz besonders bevorzugt für Allyl, 2-Methyl-allyl, 1-Methyl-allyl, 3-Methyl-2-butenyl, Propargyl, 2-Butinyl, 3,3-Difluorallyl, Cyclopropylmethyl.
- X: steht insbesondere ganz besonders bevorzugt für Fluor.
- m: steht insbesondere ganz besonders bevorzugt für 0 oder 1.
- n: steht insbesondere ganz besonders bevorzugt für 0.
- A: steht insbesondere ganz besonders bevorzugt für einen Rest der Formel 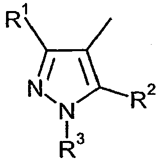 worin
R¹ für Methyl, Monofluormethyl, Difluormethyl oder Trifluormethyl steht und
R² für Wasserstoff, Fluor, Chlor oder Methyl steht und
R³ für Methyl steht.
- A: steht außerdem insbesondere ganz besonders bevorzugt für einen Rest der Formel 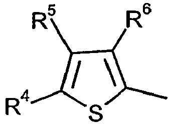 worin
R⁴ und R⁵ jeweils für Wasserstoff stehen und
R⁶ für Methyl steht.
- A: steht außerdem insbesondere ganz besonders bevorzugt für einen Rest der Formel 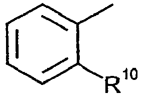 worin
R¹⁰ für Iod, Methyl oder Trifluormethyl.
- A: steht außerdem insbesondere ganz besonders bevorzugt für einen Rest der Formel 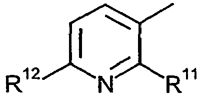 worin
R¹¹ für Chlor oder Trifluormethyl steht und
R¹² für Wasserstoff steht
- A: steht außerdem insbesondere ganz besonders bevorzugt für einen Rest der Formel 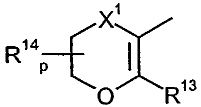 worin
R¹³ für Methyl oder Trifluormethyl steht und
X¹ für S (Schwefel) steht und
p für 0 steht.
- A: steht außerdem insbesondere ganz besonders bevorzugt für einen Rest der Formel 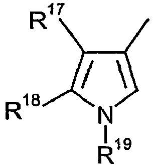 worin
R¹⁷ für Methyl oder Trifluormethyl steht,
R¹⁸ für Wasserstoff oder Methyl steht und
R¹⁹ für Methyl steht.
- A: steht außerdem insbesondere ganz besonders bevorzugt für einen Rest der Formel 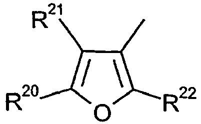 worin
R²⁰ und R²¹ jeweils für Wasserstoff stehen und
R²² für Methyl steht.
- A: steht außerdem insbesondere ganz besonders bevorzugt für einen Rest der Formel 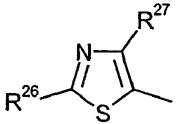 worin
R²⁶ für Amino oder Methyl steht und
R²⁷ für Methyl, Trifluormethyl oder Difluormethyl steht.
- A: steht außerdem insbesondere ganz besonders bevorzugt für einen Rest der Formel 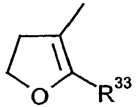 worin
R³³ für Methyl steht.

Bevorzugt sind weiterhin Verbindungen der Formel (I-1) 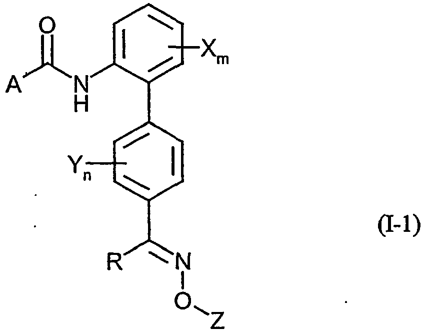 in welcher
R, Z, X, Y, m, n und A die oben angegebenen Bedeutungen haben. Es kommen jeweils alle Kombinationen der oben genannten allgemeinen, bevorzugten, besonders bevorzugten, ganz besonders bevorzugten bzw. insbesondere ganz besonders bevorzugten Bedeutungen infrage.

Besonders bevorzugt sind Verbindungen der Formel (I-1), in welcher X für Fluor steht und m für 0 oder 1 steht.

Besonders bevorzugt sind Verbindungen der Formel (I-1), in welcher n für 0 steht Besonders bevorzugt sind Verbindungen der Formel (I-1), in welcher R für Wasserstoff oder Methyl steht.

Außerdem besonders bevorzugt sind Verbindungen der Formel (I), in welcher X für Fluor steht und m für 0 oder 1 steht.

Außerdem besonders bevorzugt sind Verbindungen der Formel (I), in welcher n für 0 steht. Außerdem besonders bevorzugt sind Verbindungen der Formel (I), in welcher R für Wasserstoff oder Methyl steht.

Bevorzugt, besonders bevorzugt, ganz besonders bevorzugt bzw. insbesondere ganz besonders bevorzugt sind Verbindungen, welche die unter bevorzugt, besonders bevorzugt, ganz besonders bevorzugt bzw. insbesondere ganz besonders bevorzugt genannten Substituenten tragen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können. Mehrere Reste mit denselben Indizes wie beispielsweise m Reste X für m > 1, können gleich oder verschieden sein.

Durch Halogen substituierte Reste, wie z.B. Halogenalkyl, sind einfach oder mehrfach halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die oben aufgeführten allgemeinen oder in Vorzugsbereichen aufgeführten Restedefinitionen bzw. Erläuterungen können jedoch auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können auch einzelne Definitionen entfallen.

Verwendet man 5-Fluor-1,3-dimethyl-1*H*-pyrazol-4-carbonylchlorid und 1-(2'-Aminobiphenyl-4-yl)-ethanon-*O*-allyl-oxim als Ausgangsstoffe, so kann der Verlauf des erfindungsgemäßen Verfahrens (a) durch das folgende Formelschema veranschaulicht werden.

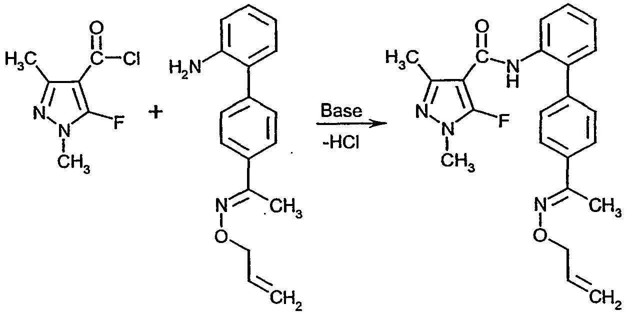

Verwendet man *N*-(2-Iodphenyl)-5-fluor-1,3-dimethyl-1*H*-pyrazol-4-carboxamid und 4-[1-(Allyloxyimino)-ethyl]-phenylboronsäure als Ausgangsstoffe sowie einen Katalysator, so kann der Verlauf des erfindungsgemäßen Verfahrens (b) durch das folgende Formelschema veranschaulicht werden.

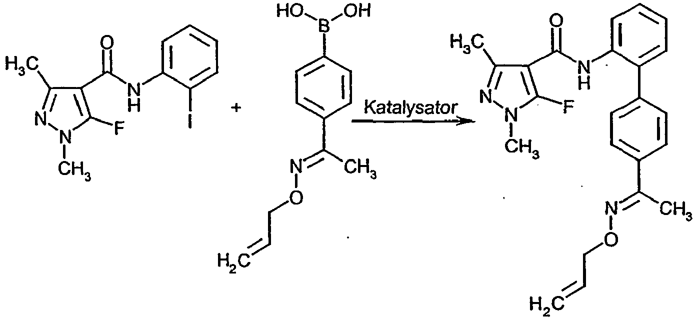

Verwendet man 2-[(5-Fluor-1,3-dimethylpyrazol-4-yl)carbonylamino]phenyl-boronsäure und 1-(4-Brom-phenyl)-ethanon-*O*-allyl-oxim als Ausgangsstoffe sowie einen Katalysator, so kann der Verlauf des erfindungsgemäßen Verfahrens (c) durch das folgende Formelschema veranschaulicht werden.

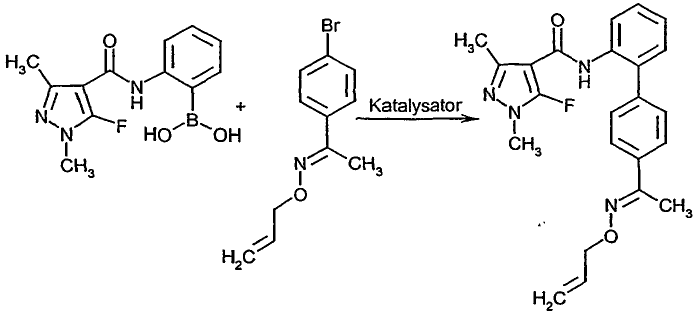

Verwendet man *N*-(4'-Acetyl-biphenyl-2-yl)-2-chlor-nicotinamid und *O*-(3,3-Dichlorallyl)-hydroxylamin Hydrochlorid als Ausgangsstoffe, so kann der Verlauf des erfindungsgemäßen Verfahrens (d) durch das folgende Formelschema veranschaulicht werden.

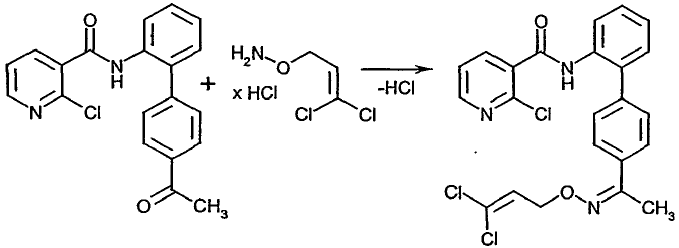

Verwendet man 1,3-Dimethyl-1*H*-pyrazol-4-carbonsäure-(4'-{1-[hydroxyimino]-ethyl}-biphenyl-2-yl)-amid und 1,1,3-Trichlorpropen als Ausgangsstoffe, so kann der Verlauf des erfindungsgemäßen Verfahrens (e) durch das folgende Formelschema veranschaulicht werden.

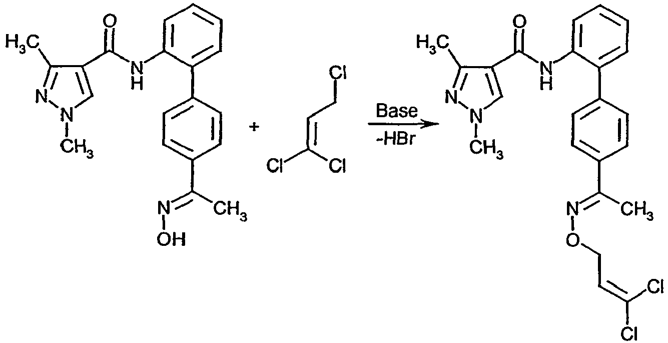

Verwendet man *N*-(2-Bromphenyl)-5-fluor-1,3-dimethyl-1*H*-pyrazol-4-carboxamid und 1-(4-Brom-phenyl)-ethanon-*O*-allyl-oxim als Ausgangsstoffe sowie einen Katalysator und 4,4,4',4',5,5,5',5'-Octamethyl-2,2'-bis-1,3,2-dioxaborolan, so kann der Verlauf des erfindungsgemäßen Verfahrens (f) durch das folgende Formelschema veranschaulicht werden.

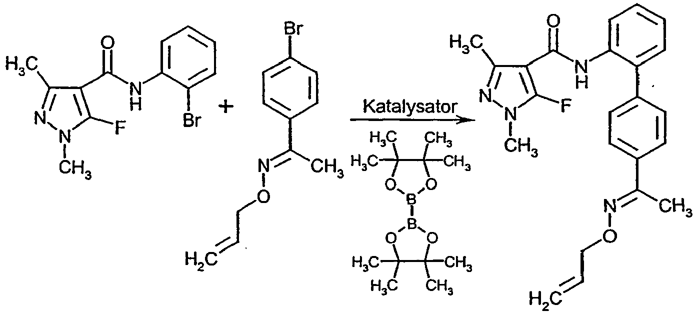

### Erläuterung der Verfahren und Zwischenprodukte

Die bei der Durchführung des erfindungsgemäßen Verfahrens (a) als Ausgangsstoffe benötigten Carbonsäure-Derivate sind durch die Formel (II) allgemein definiert. In dieser Formel steht A vorzugsweise für diejenigen Bedeutungen, die bereits in Zusammenhang mit der Beschreibung der erfindungsgemäßen Stoffe der Formel (I) für diese Reste als bevorzugt, besonders bevorzugt, ganz besonders bevorzugt bzw. insbesondere ganz besonders bevorzugt genannt wurden. G steht bevorzugt für Chlor, Brom, Hydroxy, Methoxy oder Ethoxy, besonders bevorzugt für Chlor, Hydroxy oder Methoxy.

Die Carbonsäure-Derivate der Formel (II) sind bekannt oder lassen sich nach bekannten Verfahren herstellen (vgl. WO 93/11 117, EP-A 0 545 099, EP-A 0 589 301 und EP-A 0 589 313).

Die bei der Durchführung des erfindungsgemäßen Verfahrens (a) als Reaktionskomponenten benötigten Anilin-Derivate sind durch die Formel (III) allgemein definiert. In dieser Formel haben R, Z, X, Y, m und n vorzugsweise diejenigen Bedeutungen, die bereits im Zusammenhang mit der Beschreibung der erfindungsgemäßen Stoffe der Formel (I) bevorzugt, besonders bevorzugt, ganz besonders bevorzugt bzw. insbesondere ganz besonders bevorzugt für diese Reste bzw. diese Indices genannt wurden.

Die Anilin-Derivate der Formel (III) sind neu. Sie lassen sich teilweise nach bekannten Methoden herstellen (vgl. EP-A 0 545 099 und EP-A 0 589 301).

Man erhält Anilin-Derivate der Formel (III) außerdem, indem man
g) 2-Halogenanilin-Derivate der allgemeinen Formel (XI) 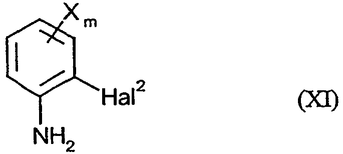 in welcher
   - X und m: die oben angegebenen Bedeutungen haben und
   - Hal²: für Halogen steht,
   mit Boronsäure-Derivaten der Formel (V) 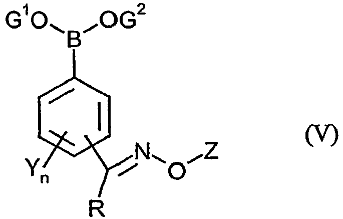 in welcher R, Z, Y, n, G¹ und G² die oben angegebenen Bedeutungen haben,
   gegebenenfalls in Gegenwart eines Säurebindemittels, sowie gegebenenfalls in Gegenwart eines inerten organischen Verdünnungsmittels, sowie gegebenenfalls in Gegenwart eines Katalysators umsetzt oder
h) Anilinboronsäuren der Formel (XII) 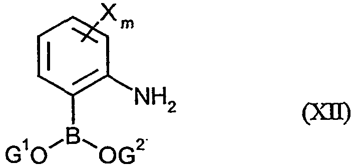 in welcher X, m, G¹ und G² die oben angegebenen Bedeutungen haben
   mit Phenyloxim-Derivaten der Formel (VII) 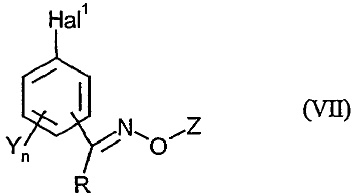 in welcher R, Z, Y, n und Hal¹ die oben angegebenen Bedeutungen haben,
   gegebenenfalls in Gegenwart eines Säurebindemittels, sowie gegebenenfalls in Gegenwart eines inerten organischen Verdünnungsmittels, sowie gegebenenfalls in Gegenwart eines Katalysators umsetzt.

Die bei der Durchführung der erfindungsgemäßen Verfahren (g) und (1) (siehe unten) als Reaktionskomponenten benötigten 2-Halogenanilin-Derivate sind durch die Formel (XI) allgemein definiert. In dieser Formel haben X und m vorzugsweise diejenigen Bedeutungen, die bereits im Zusammenhang mit der Beschreibung der erfindungsgemäßen Stoffe der Formel (I) bevorzugt, besonders bevorzugt, ganz besonders bevorzugt bzw. insbesondere ganz besonders bevorzugt für diese Reste bzw. diese Indices genannt wurden. Hal steht vorzugsweise für Fluor, Chlor oder Brom, besonders bevorzugt für Chlor oder Brom.

2-Halogenanilin-Derivate der Formel (XI) sind kommerziell erhältlich oder lassen sich aus den entsprechenden Nitroverbindungen durch Reduktion herstellen.

Die bei der Durchführung der erfindungsgemäßen Verfahren (h) und (j) (siehe unten) als Reaktionskomponenten benötigten Anilinboronsäuren sind durch die Formel (XII) allgemein definiert. In dieser Formel haben X und m vorzugsweise diejenigen Bedeutungen, die bereits im Zusammenhang mit der Beschreibung der erfindungsgemäßen Stoffe der Formel (I) bevorzugt, besonders bevorzugt, ganz besonders bevorzugt bzw. insbesondere ganz besonders bevorzugt für diese Reste bzw. diese Indices genannt wurden. G¹ und G² stehen bevorzugt jeweils für Wasserstoff.

Anilinboronsäuren der Formel (XII) sind kommerziell erhältlich.

Die bei der Durchführung der erfindungsgemäßen Verfahren (b) und (f) als Ausgangsstoffe benötigten Carboxamid-Derivate sind durch die Formel (IV) allgemein definiert. In dieser Formel stehen A, X und m vorzugsweise für diejenigen Bedeutungen, die bereits in Zusammenhang mit der Beschreibung der erfindungsgemäßen Stoffe der Formel (I) für diese Reste bzw. diese Indices als bevorzugt, besonders bevorzugt, ganz besonders bevorzugt bzw. insbesondere ganz besonders bevorzugt genannt wurden.

Die Carboxamid-Derivate der Formel (IV) sind bekannt oder lassen sich nach bekannten Verfahren herstellen (vgl. WO 91/01311, EP-A 0 371 950).

Die bei der Durchführung des erfindungsgemäßen Verfahrens (b) sowie des Verfahrens (g) zur Herstellung der Reaktionskomponenten benötigten Boronsäure-Derivate sind durch die Formel (V) allgemein definiert. In dieser Formel haben R, Z, Y und n vorzugsweise diejenigen Bedeutungen, die bereits im Zusammenhang mit der Beschreibung der erfindungsgemäßen Stoffe der Formel (I) bevorzugt, besonders bevorzugt, ganz besonders bevorzugt bzw. insbesondere ganz besonders bevorzugt für diese Reste bzw. diese Indices genannt wurden. G¹ und G² stehen bevorzugt jeweils für Wasserstoff.

Die Boronsäure-Derivate der Formel (V) sind neu und lassen sich herstellen, indem man
i) Phenylboronsäuren der Formel (XIII) 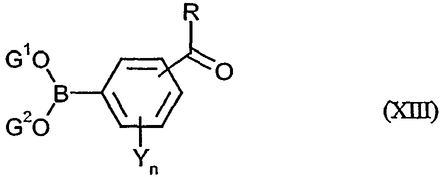 in welcher R, Y, n, G¹ und G² die oben angegebenen Bedeutungen haben,
   mit Hydroxylamin-Derivaten der Formel (IX)

   Z-O-NH₂ x HCl (IX)

   in welcher Z die oben angegebenen Bedeutungen hat,
   gegebenenfalls in Gegenwart eines Säurebindemittels, sowie gegebenenfalls in Gegenwart eines inerten organischen Verdünnungsmittels, sowie gegebenenfalls in Gegenwart eines Katalysators umsetzt.

Die bei der Durchführung der erfindungsgemäßen Verfahren (i) und (1) (siehe unten) als Reaktionskomponenten benötigten Phenylboronsäuren sind durch die Formel (XIII) allgemein definiert. In dieser Formel haben R, Y und n vorzugsweise diejenigen Bedeutungen, die bereits im Zusammenhang mit der Beschreibung der erfindungsgemäßen Stoffe der Formel (I) bevorzugt, besonders bevorzugt, ganz besonders bevorzugt bzw. insbesondere ganz besonders bevorzugt für diese Reste bzw. diese Indices genannt wurden. G¹ und G² stehen bevorzugt jeweils für Wasserstoff.

Die Phenylboronsäuren der Formel (XIII) sind kommerziell erhältlich.

Die bei der Durchführung des erfindungsgemäßen Verfahrens (c) als Reaktionskomponenten benötigten Carboxamid-Boronsäure-Derivate sind durch die Formel (VI) allgemein definiert. In dieser Formel stehen A, X und m, vorzugsweise für diejenigen Bedeutungen, die bereits in Zusammenhang mit der Beschreibung der erfindungsgemäßen Stoffe der Formel (I) für diese Reste bzw. diese Indices als bevorzugt, besonders bevorzugt, ganz besonders bevorzugt bzw. insbesondere ganz besonders bevorzugt genannt wurden. G¹ und G² stehen bevorzugt jeweils für Wasserstoff oder zusammen für Tetramethylethylen.

Die Carboxamid-Boronsäure-Derivate der Formel (VI) lassen sich herstellen, indem man
j) Carbonsäure-Derivate der Formel (II) 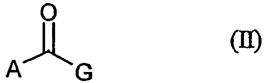 in welcher A und G die oben angegebenen Bedeutungen haben,
   mit Anilinboronsäuren der Formel (XII) 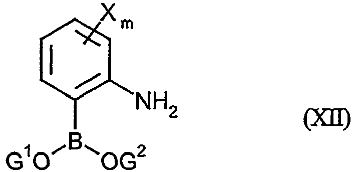 in welcher X, m, G¹ und G² die oben angegebenen Bedeutungen haben,
   gegebenenfalls in Gegenwart eines Säurebindemittels, sowie gegebenenfalls in Gegenwart eines inerten organischen Verdünnungsmittels, sowie gegebenenfalls in Gegenwart eines Katalysators umsetzt.

Die bei der Durchführung der erfindungsgemäßen Verfahren (c) und (f) sowie des Verfahrens (h) als Reaktionskomponenten benötigten Phenyloxim-Derivate sind durch die Formel (VII) allgemein definiert. In dieser Formel stehen R, Z, Y und n vorzugsweise für diejenigen Bedeutungen, die bereits in Zusammenhang mit der Beschreibung der erfindungsgemäßen Stoffe der Formel (I) für diese Reste bzw. diese Indices als bevorzugt, besonders bevorzugt, ganz besonders bevorzugt bzw. insbesondere ganz besonders bevorzugt genannt wurden.

Die Phenyloxim-Derivate der Formel (VII) sind bekannt und/oder lassen sich nach bekannten Verfahren herstellen (vgl. Synth. Commun. 2000, 30, 665-669, Synth. Commun. 1999, 29, 697-1701).

Die bei der Durchführung des erfindungsgemäßen Verfahrens (d) als Ausgangsstoffe benötigten Biphenylacyl-Derivate sind durch die Formel (VIII) allgemein definiert. In dieser Formel stehen A, R, X, Y, m und n für diejenigen Bedeutungen, die bereits in Zusammenhang mit der Beschreibung der erfindungsgemäßen Stoffe der Formel (I) für diese Reste bzw. diese Indices als bevorzugt, besonders bevorzugt, ganz besonders bevorzugt bzw. insbesondere ganz besonders bevorzugt genannt wurden.

Die Biphenylacyl-Derivate der Formel (VIII) lassen sich herstellen, indem man
k) Carbonsäure-Derivate der Formel (II) 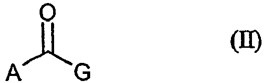 in welcher A und G die oben angegebenen Bedeutungen haben,
   mit 2-Benzaldehyd-anilin-Derivaten der Formel (XIV) 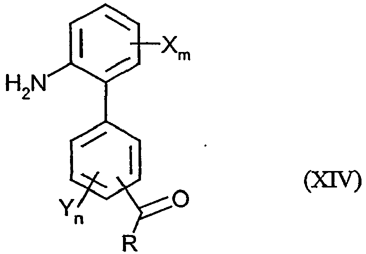 in welcher R, X, Y, m und n die oben angegebenen Bedeutungen haben,
   gegebenenfalls in Gegenwart eines Säurebindemittels, sowie gegebenenfalls in Gegenwart eines inerten organischen Verdünnungsmittels umsetzt.

Die bei der Durchführung des erfindungsgemäßen Verfahrens (k) als Reaktionskomponenten benötigten 2-Benzaldehyd-anilin-Derivate sind durch die Formel (XIV) allgemein definiert. In dieser Formel stehen R, X, Y, m und n vorzugsweise für diejenigen Bedeutungen, die bereits in Zusammenhang mit der Beschreibung der erfindungsgemäßen Stoffe der Formel (I) für diese Reste bzw. diese Indices als bevorzugt, besonders bevorzugt, ganz besonders bevorzugt bzw. insbesondere ganz besonders bevorzugt genannt wurden.

Die 2-Benzaldehyd-anilin-Derivate der Formel (XIV) lassen sich herstellen, indem man
1) Anilin-Derivate der Formel (XI) 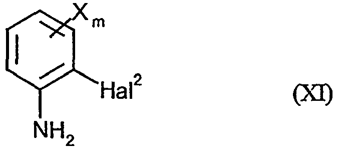 in welcher X, m und Hal² die oben angegebenen Bedeutungen haben und
   mit Phenylboronsäure-Derivaten der Formel (XIII) 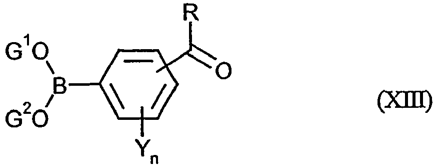 in welcher R, Y, n, G¹ und G² die oben angegebenen Bedeutungen haben,
   gegebenenfalls in Gegenwart eines Säurebindemittels, sowie gegebenenfalls in Gegenwart eines inerten organischen Verdünnungsmittels umsetzt.

Die bei der Durchführung des erfindungsgemäßen Verfahrens (d) sowie des Verfahrens (i) als Reaktionskomponenten benötigten Hydroxylamin-Derivate sind durch die Formel (IX) allgemein definiert. In dieser Formel hat Z vorzugsweise diejenigen Bedeutungen, die bereits im Zusammenhang mit der Beschreibung der erfindungsgemäßen Stoffe der Formel (I) bevorzugt, besonders bevorzugt, ganz besonders bevorzugt bzw. insbesondere ganz besonders bevorzugt für diesen Rest genannt wurden. Bevorzugt werden die in der Beschreibung angegebenen Hydrochloride eingesetzt. Es können aber auch die freien Hydroxylamin-Derivate in dem erfindungsgemäßen Verfahren verwendet werden.

Hydroxylamin-Derivate der Formel (IX) sind kommerziell erhältlich.

Die bei der Durchführung des erfindungsgemäßen Verfahrens (e) als Ausgangsstoffe benötigten Hydroxyimino-Derivate sind durch die Formel (I-a) allgemein definiert. In dieser Formel steht A, R, X, Y, m und n vorzugsweise für diejenigen Bedeutungen, die bereits in Zusammenhang mit der Beschreibung der erfindungsgemäßen Stoffe der Formel (I) für diese Reste bzw. diese Indices als bevorzugt, besonders bevorzugt, ganz besonders bevorzugt bzw. insbesondere ganz besonders bevorzugt genannt wurden.

Die erfindungsgemäßen Hydroxyimino-Derivate der Formel (I-a) lassen sich nach einem der oben beschriebenen erfindungsgemäßen Verfahren (a), (b), (c), (d) oder (f) herstellen.

Die bei der Durchführung des erfindungsgemäßen Verfahrens (e) als Reaktionskomponenten benötigten Verbindungen sind durch die Formel (X) allgemein definiert. In dieser Formel steht Z vorzugsweise für diejenigen Bedeutungen, die bereits in Zusammenhang mit der Beschreibung der erfindungsgemäßen Stoffe der Formel (I) für diesen Rest als bevorzugt, besonders bevorzugt, ganz besonders bevorzugt bzw. insbesondere ganz besonders bevorzugt genannt wurden. E steht bevorzugt für Chlor, Brom, Iod, Methansulfonyl oder p-Toluolsulfonyl. E steht besonders bevorzugt für Chlor oder Brom.

Verbindungen der Formel (X) sind kommerziell erhältlich.

Als Säurebindemittel kommen bei der Durchführung der erfindungsgemäßen Verfahren (a), (b), (c), (d), (e) und (f) jeweils alle für derartige Reaktionen üblichen anorganischen und organischen Basen in Betracht Vorzugsweise verwendbar sind Erdalkali- oder Alkalimetallhydroxide, wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU). Es ist jedoch auch möglich, ohne zusätzliches Säurebindemittel zu arbeiten, oder die Aminkomponente in einem Überschuss einzusetzen, so dass sie gleichzeitig als Säurebindemittel fungiert.

Als Verdünnungsmittel kommen bei der Durchführung der erfindungsgemäßen Verfahren (a), (b), (c), (d), (e) und (f) jeweils alle üblichen inerten, organischen Solventien in Frage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid oder Sulfone, wie Sulfolan.

Die Reaktionstemperaturen können bei der Durchführung der erfindungsgemäßen Verfahren (a), (b), (c), (d), (e) und (f) jeweils in einem größeren Bereich variiert werden. Im allgemeinen arbeitet man bei Temperaturen zwischen 0°C und 140°C, vorzugsweise zwischen 10°C und 120°C.

Bei der Durchführung der erfindungsgemäßen Verfahren (a), (b), (c), (d), (e) und (f) arbeitet man im allgemeinen jeweils unter Atmosphärendruck. Es ist aber auch möglich, jeweils unter erhöhtem oder vermindertem Druck zu arbeiten.

Bei der Durchführung des erfindungsgemäßen Verfahrens (a) setzt man auf 1 Mol an Säurehalogenid der Formel (II) im allgemeinen 1 Mol oder auch einen Überschuss an Anilin-Derivat der Formel (III) sowie 1 bis 3 Mol an Säurebindemittel ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, die organische Phase abtrennt und nach dem Trocknen unter vermindertem Druck einengt. Der verbleibende Rückstand kann gegebenenfalls nach üblichen Methoden, wie Chromatographie oder Umkristallisation, von eventuell noch vorhandenen Verunreinigungen befreit werden.

Bei der Durchführung des erfindungsgemäßen Verfahrens (b) setzt man auf 1 Mol an Carboxamid der Formel (IV) im allgemeinen 1 Mol oder auch einen Überschuss an Boronsäure-Derivat der Formel (V) sowie 1 bis 5 Mol an Säurebindemittel ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, den Niederschlag abtrennt und trocknet. Der verbleibende Rückstand kann gegebenenfalls nach üblichen Methoden, wie Chromatographie oder Umkristallisation, von eventuell noch vorhandenen Verunreinigungen befreit werden.

Bei der Durchführung des erfindungsgemäßen Verfahrens (c) setzt man auf 1 Mol an Carboxamid-Boronsäure-Derivat der Formel (VI) im allgemeinen 1 Mol oder auch einen Überschuss an Phenyloxim-Derivat der Formel (VII) sowie 1 bis 10 Mol an Säurebindemittel und 0.5 bis 5 Molprozent eines Katalysators ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, den Niederschlag abtrennt und trocknet. Der verbleibende Rückstand kann gegebenenfalls nach üblichen Methoden, wie Chromatographie oder Umkristallisation, von eventuell noch vorhandenen Verunreinigungen befreit werden.

Bei der Durchführung des erfindungsgemäßen Verfahrens (d) setzt man auf 1 Mol an Biphenylacyl-Derivat der Formel (VIII) im allgemeinen 1 Mol oder auch einen Überschuss an Hydroxylamin-Derivat der Formel (IX) sowie 1 bis 5 Mol an Säurebindemittel ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, den Niederschlag abtrennt, mit Wasser und Diisopropylether wäscht und anschließend trocknet. Der verbleibende Rückstand kann gegebenenfalls nach üblichen Methoden, wie Chromatographie oder Umkristallisation, von eventuell noch vorhandenen Verunreinigungen befreit werden.

Bei der Durchführung des erfindungsgemäßen Verfahrens (e) setzt man auf 1 Mol an Hydroxyimino-Derivat der Formel (I-a) im allgemeinen 1 Mol oder auch einen Überschuss an Reagenz der Formel (X) sowie 1 bis 5 Mol an Säurebindemittel ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, den Niederschlag abtrennt und trocknet. Der verbleibende Rückstand kann gegebenenfalls nach üblichen Methoden, wie Chromatographie oder Umkristallisation, von eventuell noch vorhandenen Verunreinigungen befreit werden.

Bei der Durchführung des erfindungsgemäßen Verfahrens (f) setzt man auf 1 Mol an Carboxamid-Derivat der Formel (IV) im allgemeinen 1 Mol oder auch einen Überschuss an Phenyloxim-Derivat der Formel (VII) sowie 1 bis 5 Mol an Säurebindemittel ein, sowie 1 bis 5 Mol eines Katalysators. Es ist jedoch auch möglich, die Reaktionskomponenten in anderen Verhältnissen einzusetzen. Die Aufarbeitung erfolgt nach üblichen Methoden. Im allgemeinen verfährt man in der Weise, dass man das Reaktionsgemisch mit Wasser versetzt, den Niederschlag abtrennt und trocknet. Der verbleibende Rückstand kann gegebenenfalls nach üblichen Methoden, wie Chromatographie oder Umkristallisation, von eventuell noch vorhandenen Verunreinigungen befreit werden.

Die Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren und Nematoden, die in der Landwirtschaft, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spp..

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Acheta domesticus, Gryllotalpa spp., Locusta migratoria migratorioides, Melanoplus spp., Schistocerca gregaria.

Aus der Ordnung der Blattaria z.B. Blatta orientalis, Periplaneta americana, Leucophaea maderae, Blattella germanica.

Aus der Ordnung der Dermaptera z.B. Forficula auricularia.

Aus der Ordnung der Isoptera z.B. Reticulitermes spp..

Aus der Ordnung der Phthiraptera z.B. Pediculus humanus corporis, Haematopinus spp., Linognathus spp., Trichodectes spp., Damalinia spp..

Aus der Ordnung der Thysanoptera z.B. Hercinothrips femoralis, Thrips tabaci, Thrips palmi, Frankliniella accidentalis.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..

Aus der Ordnung der Siphonaptera z.B. Xenopsylla cheopis, Ceratophyllus spp..

Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst- und Gemüseanbau einsetzen, wie beispielsweise gegen Venturia-, Botrytis-, Sclerotinia-, Rhizoctonia-, Uncinula-, Sphaerotheca-, Podosphaera-, Alternaria- und Colletotrichum-Arten. Mit gutem Erfolg werden auch Reiskrankheiten, wie Pyricularia- und Pellicularia-Arten, bekämpft.

Die erfindungsgemäßen Wirkstoffe eignen sich auch zur Steigerung des Ernteertrags. Sie sind außerdem mindertoxisch und weisen eine gute Pflanzenverträglichkeit auf

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Emtegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Altemaria, wie Altemaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:
**Fungizide:**
   2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesil; Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tollofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-Chlorphenyl)-2-propinyl]oxy]-3-methoxyphenyl]ethyl]-3-methyl-2-[(methylsulfonyl)amino]-butanamid; 1-(1-Naphthalenyl)-1H-pyrrol-2,5-dion; 2,3,5,6-Tetrachlor-4-(methylsulfonyl)-pyridin; 2-Amino-4-methyl-N-phenyl-5-thiazolcarboxamid; 2-Chlor-N-(2,3-dihydro-1,1,3-trimethyl-1H-inden-4-yl)-3-pyridincarboxamid; 3,4,5-Trichlor-2,6-pyridindicarbonitril; Actinovate; cis-1-(4-Chlorphenyl)-2-(1H-1,2,4-triazol-1-yl)-cycloheptanol; Methyl-1-(2,3-dihydro-2,2-dimethyl-1H-inden-1-yl)-1H-imidazol-5-carboxylat; Monokaliumcarbonat; N-(6-Methoxy-3-pyridinyl)-cyclopropancarboxamid; N-Butyl-8-(1,1-dimethylethyl)-1-oxaspiro[4.5]decan-3-amin; Natriumtetrathiocarbonat; sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxine-copper.
**Bakterizide:**
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin, Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentylisomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben, Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine, DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimethoate, Dimethylvinphos, Dinobuton, Dinocap, Dinotefuran, Diofenolan, Disulfoton, Docusat-sodium, Dofenapyn, DOWCO-439, Eflusilanate, Emamectin, Emamectin-benzoate, Empenthrin (IR-isomer), Endosulfan, Entomopthora spp., EPN, Esfenvalerate, Ethiofencarb, Ethiprole, Ethion, Ethoprophos, Etofenprox, Etoxazole, Etrimfos, Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb, Gamma-HCH, Gossyplure, Grandlure, Granuloseviren, Halfenprox, Halofenozide, HCH, HCN-801, Heptenophos, Hexaflumuron, Hexythiazox, Hydramethylnone, Hydroprene, IKA-2002, Imidacloprid, Imiprothrin, Indoxacarb, Iodofenphos, Iprobenfos, Isazofos, Isofenphos, Isoprocarb, Isoxathion, Ivermectin, Japonilure, Kadethrin, Kernpolyederviren, Kinoprene, Lambda-Cyhalothrin, Lindane, Lufenuron, Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metolcarb, Metoxadiazone, Mevinphos, Milbemectin, Milbemycin, MKI-245, MON-45700, Monocrotophos, Moxidectin, MTI-800, Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron, Ob-5101, OK-5201, OK-9601, OK-9602, OK-9701, OK-9802, Omethoate, Oxamyl, Oxydemetonmethyl, Paecilomyces fumosoroseus, Parathion-methyl, Parathion (-ethyl), Permethrin (cis-, trans-), Petroleum, PH-6045, Phenothrin (IR-trans isomer), Phenthoate, Phorate, Phosalone, Phosmet, Phosphamidon, Phosphocarb, Phoxim, Piperonyl butoxide, Pirimicarb, Pitimiphos-methyl, Pirimiphos-ethyl, Prallethrin, Profenofos, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen, Quinalphos, Resmethrin, RH-5849, Ribavirin, RU-12457, RU-15525, S-421, S-1833, Salithion, Sebufos, SI-0009, Silafluofen, Spinosad, Spirodiclofen, Spiromesifen, Sulfluramid, Sulfotep, Sulprofos, SZI-121, Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (IR-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Triflumuron, Trimethacarb, Vamidothion, Vaniliprole, Verbutin, Verticillium lecanii, WL-108477, WL-40027, YI-5201, YI-5301, YI-5302, XMC, Xylylcarb, ZA-3274, Zeta-Cypermethrin, Zolaprofos, ZXI-8901, die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO 96/37494, WO 98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semicochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Die zum Schutz technischer Materialien verwendeten Mittel enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD^{®} (z.B. Mais, Baumwolle, Soja), KnockOut^{®} (z.B. Mais), StarLink^{®} (z.B. Mais), Bollgard^{®} (Baumwolle), Nucotn^{®} (Baumwolle) und NewLeaf^{®} (Kartoffel) vertrieben werden. Als Beispiele für Herbizid tolerante Pflanzen seien Maissorten, Baumwollsorten und Sojasorten genannt, die unter den Handelsbezeichnungen Roundup Ready^{®} (Toleranz gegen Glyphosate z.B. Mais, Baumwolle, Soja), Liberty Link^{®} (Toleranz gegen Phosphinotricin, z.B. Raps), IMI^{®} (Toleranz gegen Imidazolinone) und STS^{®} (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield^{®} vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Herstellungsbeispiele

### Beispiel 1

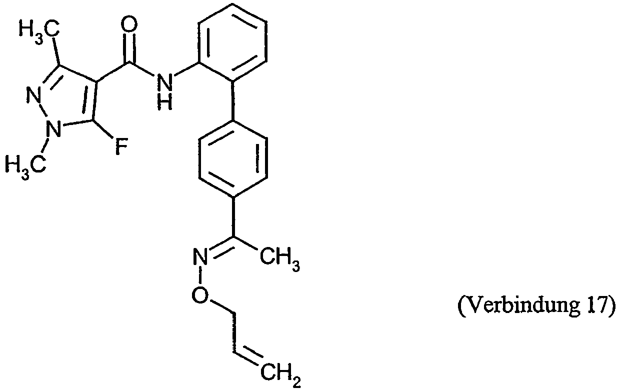

### Verfahren (a)

0,300 g (1,126 mmol) 1-(2'-Amino-biphenyl-4-yl)-ethanon-*O*-allyl-oxim und 0,114 g (1,126 mmol) Triethylamin werden in 20 ml Toluol vorgelegt. Bei Raumtemperatur gibt man 0,199 g (1,126 mmol) 5-Fluor-1,3-dimethyl-1*H*-pyrazol-4-carbonylchlorid in das Reaktionsgemisch und rührt 2 h bei 50°C nach. Zur Aufarbeitung wird das Reaktionsgemisch auf Raumtemperatur abgekühlt, zweimal mit je 100 ml Wasser gewaschen, über Natriumsulfat getrocknet und unter vermindertem Druck eingeengt. Der Rückstand wird aus n-Hexan umkristallisiert.

Man erhält 0,44 g (94,4 % der Theorie) an 5-Fluor-1,3-dimethyl-1*H*-pyrazol-4-carbonsäure-(4'-{1-(allyloxyimino)-ethyl}-biphenyl-2-yl)-amid (Verbindung 17, Tabelle 1) mit dem Schmelzpunkt 118°C.

### Beispiel 2

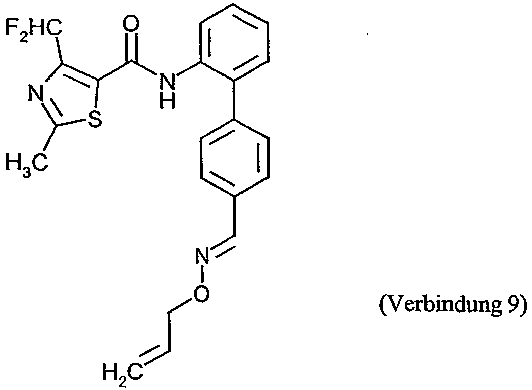

### Verfahren (a)

0,300 g (1,000 mmol) 2'-Amino-biphenyl-4-carbaldehyd-*O*-allyl-oxim (III-1) und 0,120 g (1,000 mmol) Triethylamin werden in 15 ml Toluol vorgelegt. Bei Raumtemperatur gibt man 0,251 g (1,000 mmol) 4-Difluoromethyl-2-methyl-thiazol-5-carbonylchlorid, gelöst in 5 ml Toluol, in das Reaktionsgemisch, heizt auf 50°C auf und lässt 3 h nachrühren. Zur Aufarbeitung wird das Reaktionsgemisch auf Raumtemperatur abgekühlt, zweimal mit je 80 ml Wasser gewaschen, die organische Phase über Magnesiumsulfat getrocknet und sodann unter vermindertem Druck eingeengt. Der Rückstand wird säulenchromatographisch an Kieselgel (Hexan/Methyl-tert.-butylether 3:1) gereinigt.

Man erhält 0,30 g (49,6 % der Theorie) an 4-Difluormethyl-2-methyl-thiazol-5-carbonsäure-[4'-(allyloxyimino-methyl)-biphenyl-2-yl]-amid (Verbindung 9, Tabelle 1). ¹H-NMR (DMSO-D₆): δ = 2,71 ppm (s, 3H)

### Beispiel 3

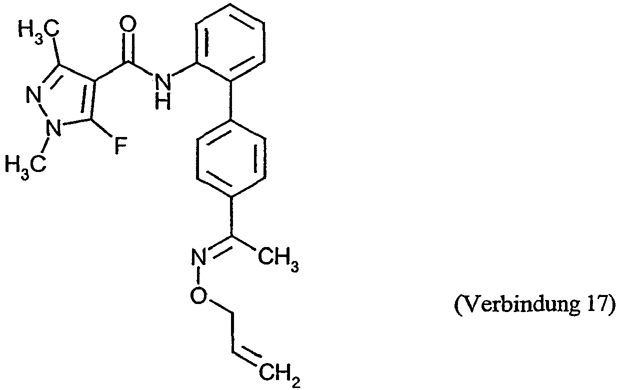

### Verfahren (b)

1,00 g (2,75 mmol) *N*-(2-Iodphenyl)-5-fluor-1,3-dimethyl-1*H*-pyrazol-4-carboxamid, 0,61 g (2,78 mmol) 4-[1-(Allyloxyimino)-ethyl]-phenylboronsäure und 0,20 g Tetrakis(triphenylphosphin)palladium (0) werden bei Raumtemperatur in 15 ml Dimethoxyethan vorgelegt. Bei Raumtemperatur wird unter Rühren eine Lösung von 1,18 g (11,14 mmol) Natriumcarbonat in 15 ml Wasser zugegeben. Man erhitzt auf Rückflusstemperatur und lässt 15 h nachrühren. Zur Aufarbeitung wird auf Raumtemperatur abgekühlt und zweimal mit je 50 ml Diethylether extrahiert. Die organischen Phasen werden mit Wasser gewaschen, über Natriumsulfat getrocknet und unter vermindertem Druck eingeengt. Der Rückstand wird säulenchromatographisch an Kieselgel (Hexan/Methyl-tert.-butylether 3:1) gereinigt.

Man erhält 0,13 g (10,5 % der Theorie) 5-Fluor-1,3-dimethyl-1*H*-pyrazol-4-carbonsäure-(4'-{1-(allyloxyimino)-ethyl}-biphenyl-2-yl)-amid (Verbindung 17, Tabelle 1) mit dem LogP (pH 2,3) = 3,80.

### Beispiel 4

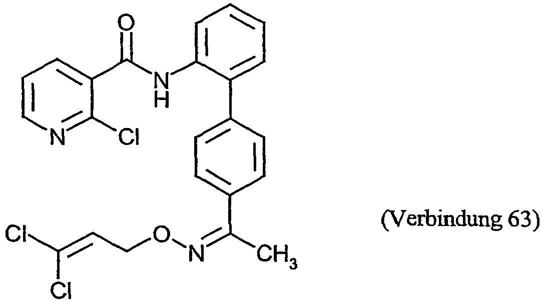

### Verfahren (d)

0,79 g (2,24 mmol) N-(4'-Acetyl-biphenyl-2-yl)-2-chlor-nicotinamid (VIII-1), 0,40 g (2,24 mmol) O-(3,3-Dichlor-allyl)-hydroxylamin-Hydrochlorid und 0,22 g Natriumacetat werden in einem Gemisch aus 5 ml Methanol und 2 ml Wasser vorgelegt und 15 h bei Raumtemperatur nachgerührt. Zur Aufarbeitung wird in 30 ml Wasser verrührt und anschließend mit 30 ml Dichlormethan extrahiert. Die organische Phase wird mit 15 ml Wasser gewaschen und die Phasen getrennt. Die organische Phase wird über Natriumsulfat getrocknet und sodann unter vermindertem Druck eingeengt. Der Rückstand wird in wenig heißem Hexan verrührt, abgekühlt und der Rückstand abgesaugt.

Man erhält 0,68 g (63,9 % der Theorie) an 2-Chlor-N-{4'-[1-(3,3-dichloro-allyloxyimino)-ethyl]-biphenyl-2-yl}-nicotinamid (Verbindung 63, Tabelle 1) mit dem LogP (pH 2,3) = 4,43.

### Beispiel 5

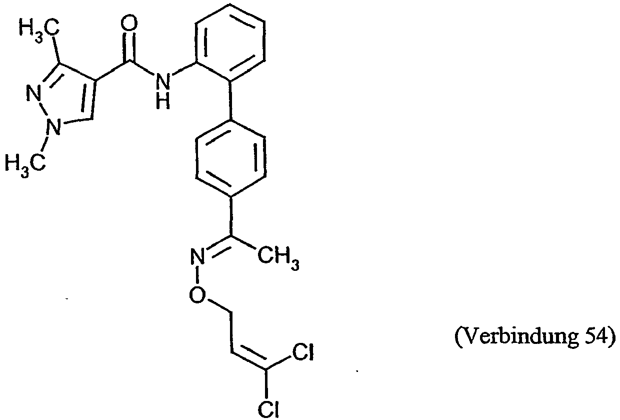

### Verfahren (e)

1,00 g (2,87 mmol) 1,3-Dimethyl-1H-pyrazol-4-carbonsäure-{4'-[1-(hydroximino)-ethyl]-biphenyl-2-yl}-amid und 0,60 g Kaliumcarbonat (4,31 mmol) werden in 50 ml Acetonitril bei Raumtemperatur vorgelegt und sodann 0,417 g 1,1,3-Trichlorpropen (2,870 mmol), gelöst in 5 ml Acetonitril, zugegeben. Man bringt auf Rückflusstemperatur und rührt 15 h nach. Zur Aufarbeitung wird abgekühlt, über eine Nutsche abgesaugt und die Mutterlauge unter vermindertem Druck eingeengt. Der Rückstand wird über Kieselgel (Hexan/Aceton 9:1) chromatographiert.

Man erhält 0,66 g (47,1 % der Theorie) an 1,3-Dimethyl-1H-pyrazol-4-carbonsäure-(4'-{1-[3,3-dichlor-allyloxyimino]-ethyl}-biphenyl-2-yl)-amid (Verbindung 54, Tabelle 1) als Feststoff mit dem LogP (pH 2,3) = 3,97.

Entsprechend den Beispielen 1 bis 5 sowie entsprechend den allgemeinen Verfahrensbeschreibungen und Methoden werden auch die in der folgenden Tabelle aufgeführten Biphenylcarboxamide der Formel (I-1) hergestellt.
Tabelle 1

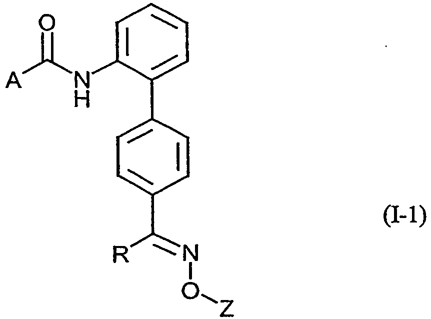

| **Nr.** | **A** | **R** | **Z** | **LogP (pH 23)** | **Fp. (°C)** |
|---|---|---|---|---|---|
| 1 | 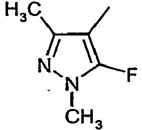 | H | 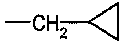 | 3,77 | |
| 2 | 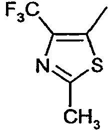 | H | 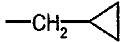 | 4,16 | |
| 3 | 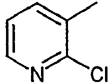 | H | 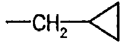 | 3,57 | |
| 4 | 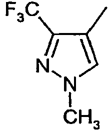 | H | 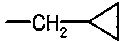 | 3,77 | |
| 5 | 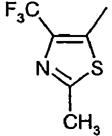 | H | 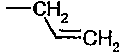 | 3,91 | 107-109 |
| 6 | 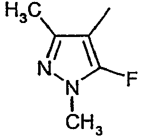 | H | 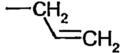 | 3,47 | 88-90 |
| 7 | 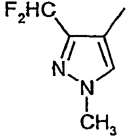 | H | 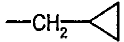 | 3,53 | |

| **Nr.** | **A** | **R** | **Z** | **LogP (pH 2.3)** | **Fp. (°C)** |
|---|---|---|---|---|---|
| 8 | 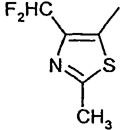 | H | 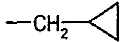 | 3,87 | |
| 9 | 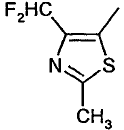 | H | 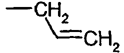 | 3,65 | |
| 10 | 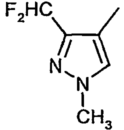 | H | 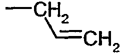 | 3,32 | |
| 11 | 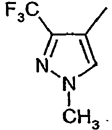 | H | 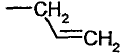 | 3,53 | 126 |
| 12 | 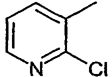 | H | 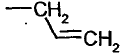 | 3,30 | 88-90 |
| 13 | 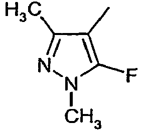 | H | 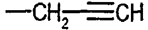 | 3,04 | |
| 14 | 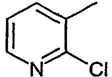 | CH₃ | 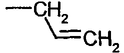 | 3,61 | 78-80 |
| 15 | 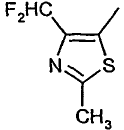 | CH₃ | 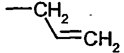 | 3,92 | 104-106 |
| 16 | 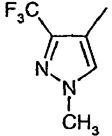 | CH₃ | 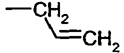 | 3,81 | 155 |
| 17 | 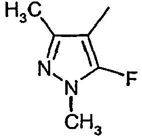 | CH₃ | 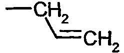 | 3,80 | 118 |
| 18 | 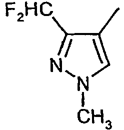 | CH₃ | 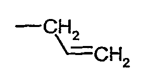 | 3,57 | 135-137 |
| 19 | 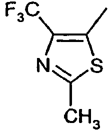 | CH₃ | 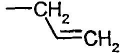 | 4,19 | 126-128 |
| 20 | 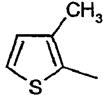 | CH₃ | 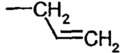 | 4,54 | 76 |
| 21 | 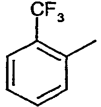 | CH₃ | 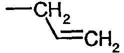 | 4,31 | 118-120 |
| 22 | 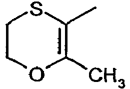 | CH₃ | 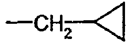 | 4,69 | 78-79 |
| 23 | 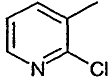 | CH₃ | 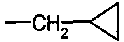 | 3,86 | 96-97 |
| 24 | 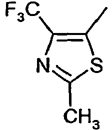 | CH₃ | 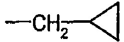 | 4,45 | 122-124 |
| 25 | 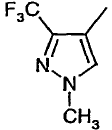 | CH₃ | 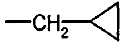 | 4,05 | 149-150 |
| 26 | 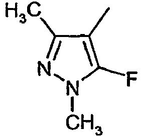 | CH₃ | 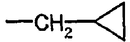 | 4,09 | 141-142 |
| 27 | 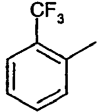 | CH₃ | 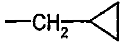 | 4,56 | 115-116 |
| 28 | 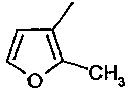 | CH₃ | 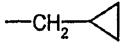 | 4,43 | 116-117 |
| 29 | 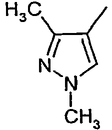 | CH₃ | 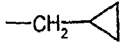 | 3,42 | 142-144 |
| 30 | 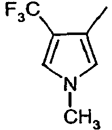 | CH₃ | 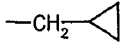 | 3,82 | 154-155 |
| 31 | 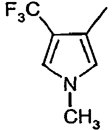 | CH₃ | 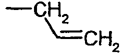 | 4,06 | 152 |
| 32 | 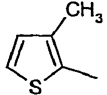 | CH₃ | 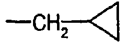 | 4,87 | 137-138 |
| 33 | 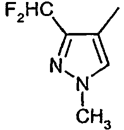 | CH₃ | 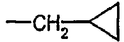 | 3,82 | 170-171 |
| 34 | 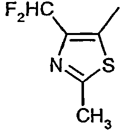 | CH₃ | 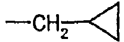 | 4,19 | 132-133 |
| 35 | 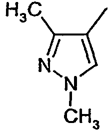 | CH₃ | 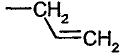 | 3,16 | 108-110 |
| 36 | 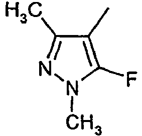 | CH₃ | 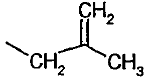 | 4,18 | |
| 37 | 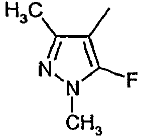 | CH₃ | 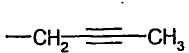 | 3,63 | |
| 38 | 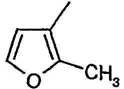 | CH₃ | 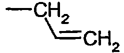 | 4,14 | |
| 39 | 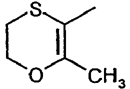 | CH₃ | 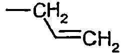 | 4,38 | |
| 40 | 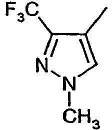 | CH₃ | 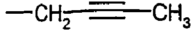 | 3,67 | |
| 41 | 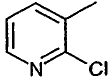 | CH₃ | 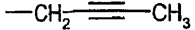 | 3,48 | |
| 42 | 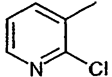 | CH₃ | 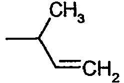 | 2,89 | |
| 43 | 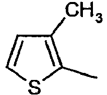 | CH₃ | 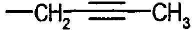 | 4,33 | |
| 44 | 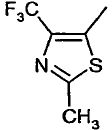 | CH₃ | 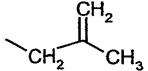 | 3,49 | |
| 45 | 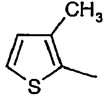 | CH₃ | 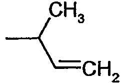 | 4,90 | |
| 46 | 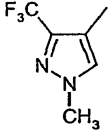 | CH₃ | 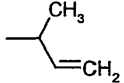 | 4,15 | |
| 47 | 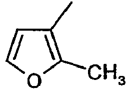 | CH₃ | 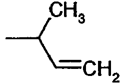 | 4,52 | |
| 48 | 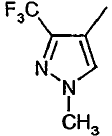 | CH₃ | 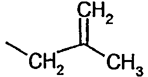 | 4,14 | |
| 49 | 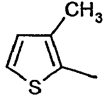 | CH₃ | 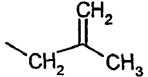 | 4,92 | |
| 50 | 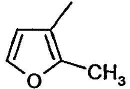 | CH₃ | 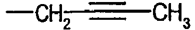 | 3,95 | |
| 51 | 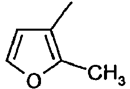 | CH₃ | 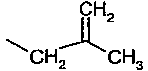 | 4,51 | |
| 52 | 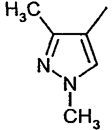 | CH₃ | 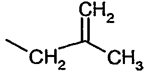 | 3,52 | |
| 53 | 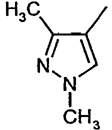 | CH₃ | 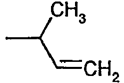 | 3,53 | |
| 54 | 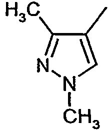 | CH₃ | 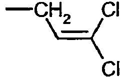 | 3,97 | |
| 55 | 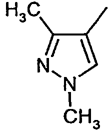 | CH₃ | 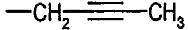 | 3,06 | |
| 56 | 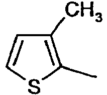 | CH₃ | 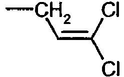 | 5,39 | |
| 57 | 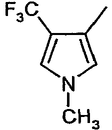 | CH₃ | 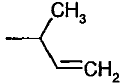 | 4,41 | |
| 58 | 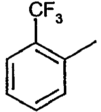 | CH₃ | 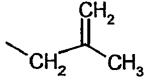 | 4,64 | |
| 59 | 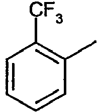 | CH₃ | 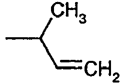 | 4,65 | |
| 60 | 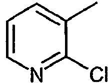 | CH₃ | 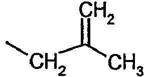 | 3,95 | |
| 61 | 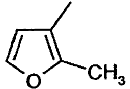 | CH₃ | 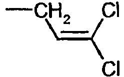 | 4,98 | |
| 62 | 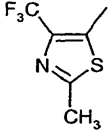 | CH₃ | 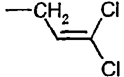 | 3,81 | |
| 63 | 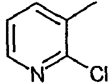 | CH₃ | 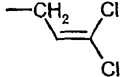 | 4,43 | |
| 64 | 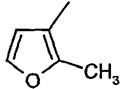 | CH₃ | 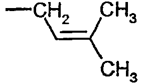 | 4,84 | |
| 65 | 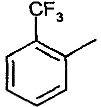 | CH₃ | 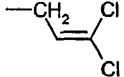 | 5,05 | |
| 66 | 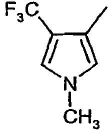 | CH₃ | 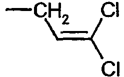 | 4,83 | |
| 67 | 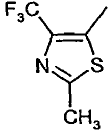 | CH₃ | 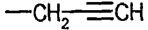 | 3,72 | 108 |
| 68 | 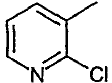 | CH₃ | 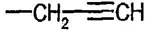 | 3,16 | 130-132 |
| 69 | 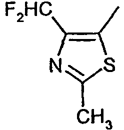 | CH₃ | 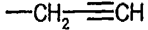 | 3,45 | 113-115 |
| 70 | 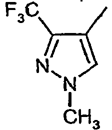 | CH₃ | 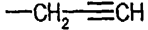 | 3,36 | 137-138 |
| 71 | 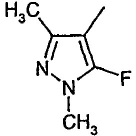 | CH₃ | 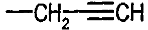 | 3,27 | 109 |
| 72 | 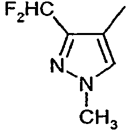 | CH₃ | 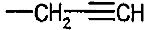 | 3,14 | 107 |
| 73 | 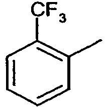 | CH₃ | 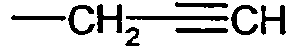 | 3,83 | 140-142 |
| 74 | 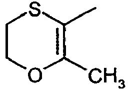 | CH₃ | 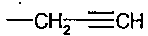 | 3,81 | 108-110 |
| 75 | 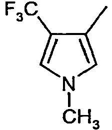 | CH₃ | 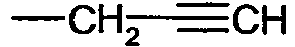 | 3,6 | 131-133 |
| 76 | 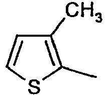 | CH₃ | 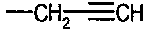 | 3,94 | 109-110 |
| 77 | 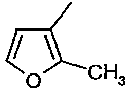 | CH₃ | 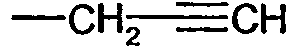 | 3,59 | |
| 78 | 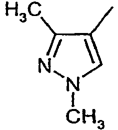 | CH₃ | 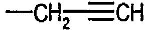 | 2,75 | 98-101 |
| 79 | 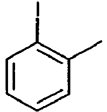 | CH₃ | 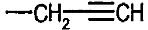 | 3,81 | 122 |
| 80 | 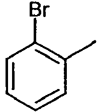 | CH₃ | 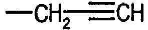 | 3,74 | 116-118 |
| 81 | 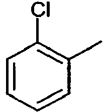 | CH₃ | 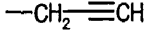 | 3,73 | 120 |
| 82 | 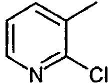 | H | 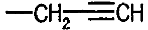 | 2,92 | 126-128 |
| 83 | 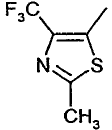 | H | 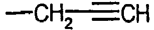 | 3,48 | |

### Herstellung von Ausgangsstoffen der Formel (III)

### Beispiel (III-1)

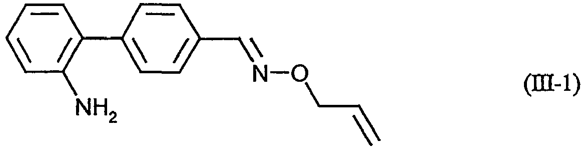

16,50 g (80,48 mmol) 4-Allyloxyiminomethyl-phenyl-boronsäure (V-1) und 13,84 g (80,48 mmol) 2-Bromanilin werden mit 0,50 g (0,43 mmol) Tetrakis(triphenylphosphin)-palladium in einem Gemisch aus 100 ml 1,2-Dimethoxyethan und 100 ml Wasser vorgelegt. Nach Zugabe von 34,12 g (321,92 mmol) Natriumcarbonat wird das Gemisch 12 h auf Rückfluss erhitzt. Zur Aufarbeitung wird das Reaktionsgemisch auf Raumtemperatur abgekühlt und zweimal mit je 200 ml Diethylether extrahiert. Die vereinigten Etherphasen werden mit 400 ml Wasser gewaschen, anschließend über Magnesiumsulfat getrocknet und unter vermindertem Druck eingeengt. Der Rückstand wird an Kieselgel chromatographiert (Hexan/Methyl-tert.-butyl-ether 3:1).
Man erhält 4,3 g (15 % der Theorie) an 2'-Amino-biphenyl-4-carbaldehyd-*O*-allyl-oxim.
¹H-NMR (DMSO-D₆): δ = 8,33 ppm (s, 1H)

### Herstellung von Ausgangsstoffen der Formel (V)

### Beispiel (V-1)

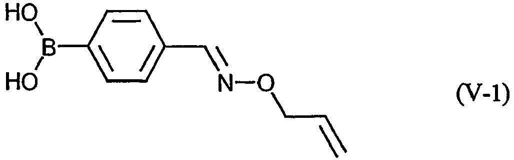

13,69 g (91,28 mmol) Formylphenylboronsäure, 10,0 g *O*-Allylhydroxylamin-Hydrochlorid (91,28 mmol) und 9,36 g (114,10 mmol) Natriumacetat werden 12 h bei Raumtemperatur in einem Gemisch aus 100 ml Methanol und 40 ml Wasser gerührt. Zur Aufarbeitung wird unter vermindertem Druck eingeengt, der Rückstand in 150 ml Wasser venührt, über einer Glasfritte abgesaugt, mit wenig Wasser gewaschen und auf einem Tonteller getrocknet.

Man erhält 16,5 g (84,6 % der Theorie) an 4-Allyloxyiminomethyl-phenyl-boronsäure.
¹H-NMR (DMSO-d₆): δ = 8,27 ppm (s, 1H)

### Herstellung von Ausgangsstoffen der Formel (VIII)

### Beispiel (VIII-1)

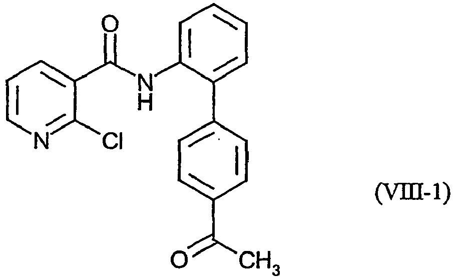

10,00 g (47,33 mmol) 1-(2'-Amino-biphenyl-4-yl)-ethanon (XIV-1) und 4,79 g (47,33 mmol) Triethylamin, gelöst in 150 ml Toluol, werden bei Raumtemperatur innerhalb von 5 Minuten mit 8,33 g (47,33 mmol) 2-Chlor-nicotinoylchlorid, gelöst in 20 ml Toluol, versetzt. Man erwärmt auf 50°C und lässt 10 h nachreagieren. Zur Aufarbeitung wird abgekühlt, mit 100 ml Wasser versetzt und die Phasen getrennt. Die organische Phase wird über Natriumsulfat getrocknet und unter vermindertem Druck eingeengt. Der Rückstand wird aus n-Hexan umkristallisiert.

Man erhält 13,7 g (82,3 % der Theorie) an *N*-(4'-Acetyl-biphenyl-2-yl)-2-chlor-nicotinamid mit dem LogP (pH 2,3) = 2,12.

### Herstellung von Ausgangsstoffen der Formel (XIV)

### Beispiel (XIV-1)

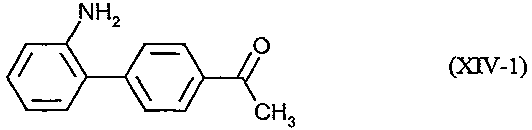

15,74 g (91,48 mmol) 2-Bromanilin, 15,00 g 4-Acetylphenylboronsäure (91,48 mmol) und 0,529 g Tertrakis(triphenylphosphin)palladium (0) (0,457 mmol) werden in 150 ml Dimethoxyethan vorgelegt 38,78 g (365,93 mmol) Natriumcarbonat, gelöst in 150 ml Wasser, werden innerhalb von 5 Minuten zugegeben. Man erwärmt auf Rückflusstemperatur und lässt 15 h nachrühren. Zur Aufarbeitung wird abgekühlt und zweimal mit je 150 ml Ether extrahiert. Die gesammelten organischen Phasen werden mit Wasser gewaschen, über Natriumsulfat getrocknet und unter vermindertem Druck eingeengt. Der Rückstand wird säulenchromatographisch an Kieselgel gereinigt (Cyclohexan/Essigsäureethylester 3:1).

Man erhält 15,0 g (72,6 % der Theorie) an 1-(2'-Amino-biphenyl-4-yl)-ethanon mit dem LogP (pH 2,3) = 1,80.

Die Bestimmung der in Tabelle 1 angegebenen LogP-Werte erfolgte gemäß EEC-Directive 79/831 Annex V.A8 durch HPLC (High Performance Liquid Chromatography) an einer Phasenumkehrsäule (C 18). Temperatur: 43°C.

Eluenten für die Bestimmung im sauren Bereich (pH 2,3): 0,1 % wässrige Phosphorsäure, Acetonitril; linearer Gradient von 10 % Acetonitril bis 90 % Acetonitril.

Die Eichung erfolgte mit unverzweigten Alkan-2-onen (mit 3 bis 16 Kohlenstoffatomen), deren LogP-Werte bekannt sind (Bestimmung der LogP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A

### Venturia - Test (Apfel) / protektiv

| | | |
|---|---|---|
| Lösungsmittel: | 24,5 | Gewichtsteile Aceton |
| | 24,5 | Gewichtsteile Dimethylacetamid |
| Emulgator: | 1,0 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers *Venturia inaequalis* inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen z.B. die folgenden erfindungsgemäßen Verbindungen der Herstellungsbeispiele gute Wirksamkeit:

**Tabelle A:**

| **Venturia -Test (Apfel) / protektiv** | | | |
|---|---|---|---|
| Wirkstoff | | Aufwandmenge an Wirkstoff in g/ha | Wirkungsgrad in % |
| 2 | 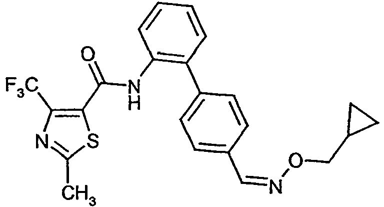 | 100 | 98 |
| 4 | 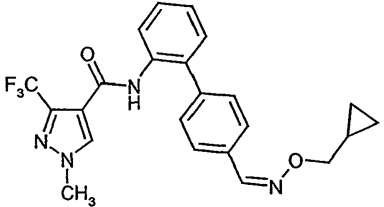 | 100 | 91 |
| 6 | 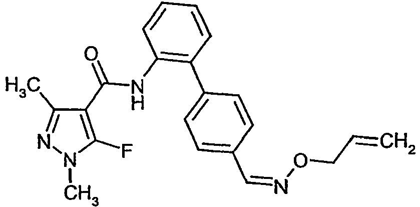 | 100 | 97 |
| 5 | 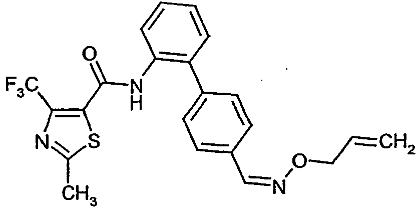 | 100 | 100 |
| 13 | 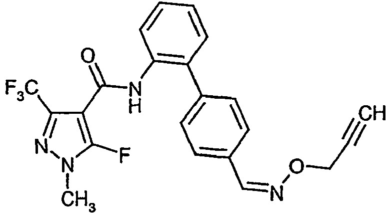 | 100 | 100 |
| 7 | 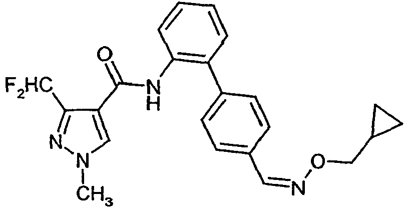 | 100 | 100 |
| 8 | 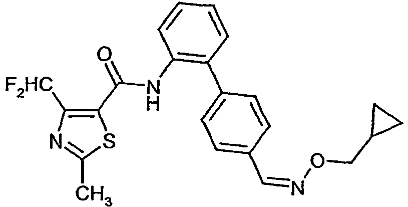 | 100 | 100 |
| 9 | 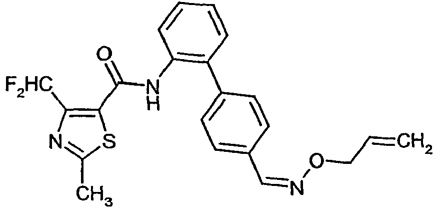 | 100 | 100 |
| 10 | 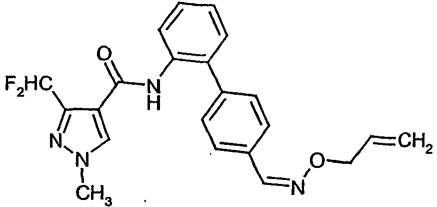 | 100 | 100 |

### Beispiel B

### Botrytis - Test (Bohne) / protektiv

| | | |
|---|---|---|
| Lösungsmittel: | 24,5 | Gewichtsteile Aceton |
| | 24,5 | Gewichtsteile Dimethylacetamid |
| Emulgator: | 1,0 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit *Botrytis cinerea* bewachsene Agarstückchen aufgelegt. Die inokulierten Pflanzen werden in einer abgedunkelten Kammer ca. 20°C und 100 % relativer Luftfeuchtigkeit aufgestellt.

2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen z.B. die folgenden erfindungsgemäßen Verbindungen der Herstellungsbeispiele gute Wirksamkeit:

**Tabelle B:**

| **Botrytis - Test (Bohne) / protektiv** | | | |
|---|---|---|---|
| Wirkstoff | | Aufwandmenge an Wirkstoff in g/ha | Wirkungsgrad in % |
| 2 | 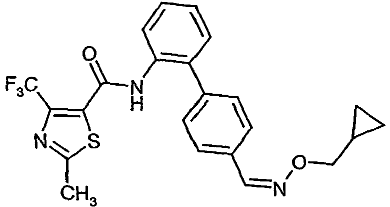 | 500 | 98 |
| 3 | 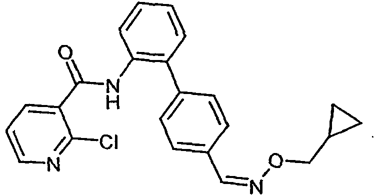 | 500 | 95 |
| 6 | 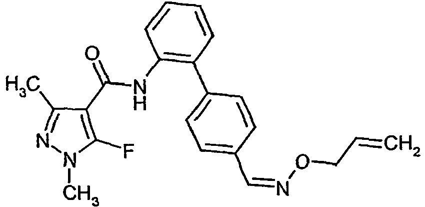 | 500 | 98 |
| 5 | 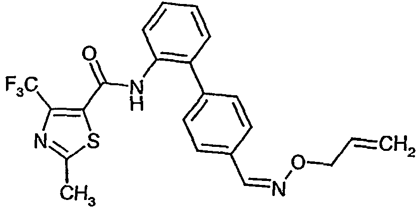 | 500 | 83 |
| 13 | 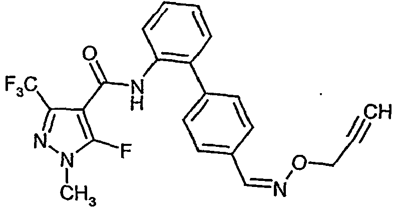 | 500 | 100 |
| 7 | 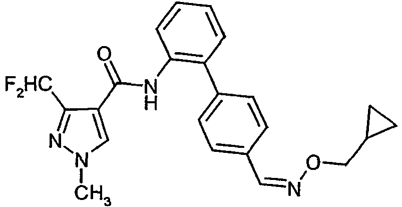 | 500 | 96 |
| 8 | 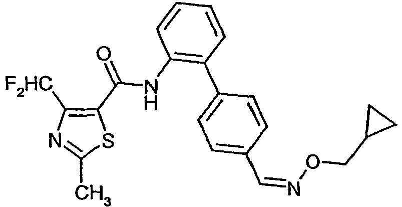 | 500 | 85 |
| 9 | 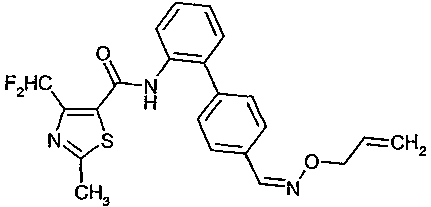 | 500 | 95 |
| 10 | 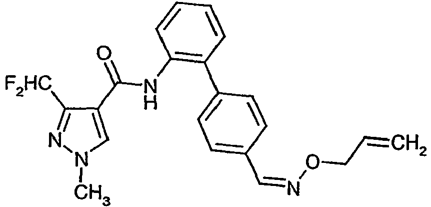 | 500 | 95 |

### Beispiel C

### Alternaria - Test (Tomate) / protektiv

| | | |
|---|---|---|
| Lösungsmittel: | 49 | Gewichtsteile N,N-Dimethylfomamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von *Alternaria solani* inokuliert und stehen dann 24 Stunden bei 100 % relativer Luftfeuchtigkeit und 20°C. Anschließend stehen die Pflanzen bei 96 % relativer Luftfeuchtigkeit und einer Temperatur von 20°C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen z.B. die folgenden erfindungsgemäßen Verbindungen der Herstellungsbeispiele gute Wirksamkeit:

**Tabelle C:**

| **Alternaria - Test (Tomate) / protektiv** | | | |
|---|---|---|---|
| Wirkstoff | | Aufwandmenge an Wirkstoff in g/ha | Wirkungsgrad in % |
| 1 | 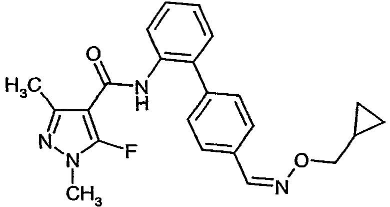 | 750 | 90 |
| 2 | 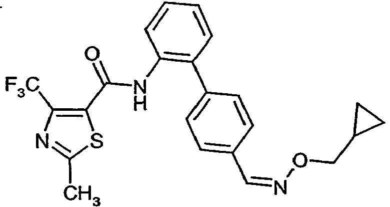 | 750 | 90 |
| 6 | 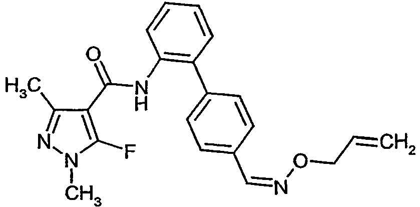 | 750 | 100 |
| 5 | 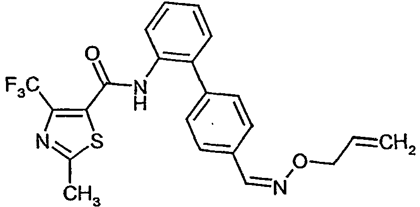 | 750 | 100 |
| 11 | 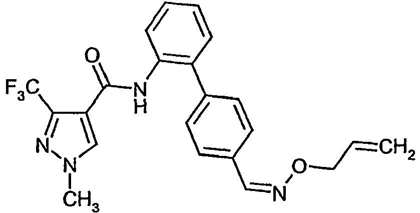 | 750 | 100 |
| 12 | 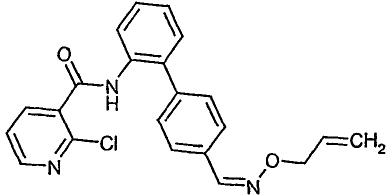 | 750 | 100 |

### Beispiel D

### Puccinia-Test (Weizen) / protektiv

| | | |
|---|---|---|
| Lösungsmittel: | 25 | Gewichtsteile N,N-Dimethylacetamid |
| Emulgator: | 0,6 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von *Puccinia recondita* in einer 0,1%igen wässrigen Agarlösung inokuliert. Nach Antrocknen des Spritzbelages werden die Pflanzen mit der Wirkstoffzubereitung in der angegebenen Aufwandmenge besprüht. Die Pflanzen verbleiben 24 Stunden bei 20°C und 100 % relativer Luftfeuchtigkeit in einer Inkubationskabine.

Die Pflanzen werden dann in einem Gewächshaus bei einer Temperatur von ca. 20°C und eine relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Bei diesem Test zeigen z.B. die folgenden erfindungsgemäßen Verbindungen der Herstellungsbeispiele gute Wirksamkeit:

**Tabelle D:**

| **Puccinia-Test (Weizen) / protektiv** | | | |
|---|---|---|---|
| Wirkstoff | | Aufwandmenge an Wirkstoff in g/ha | Wirkungsgrad in % |
| 1 | 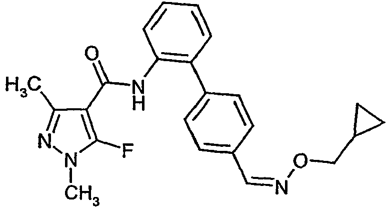 | 500 | 100 |
| 6 | 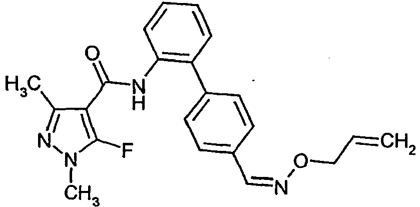 | 500 | 100 |
| 7 | 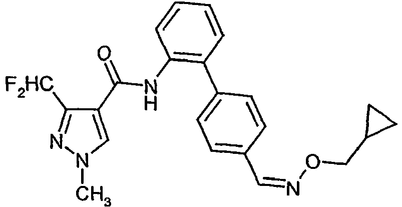 | 500 | 100 |
| 10 | 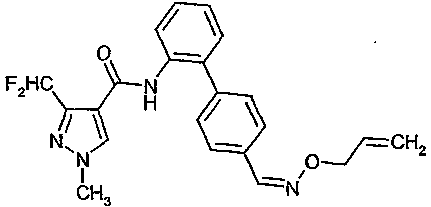 | 500 | 100 |
| 15 | 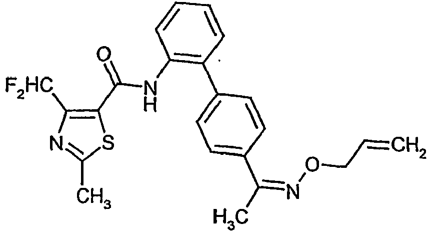 | 500 | 100 |
| 40 | 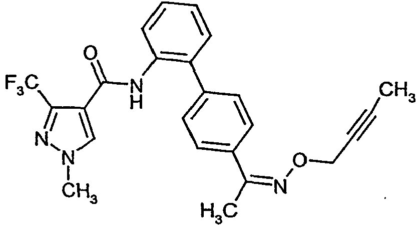 | 500 | 100 |
| 55 | 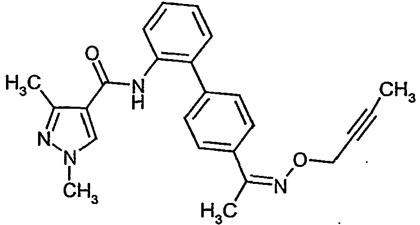 | 500 | 100 |
| 69 | 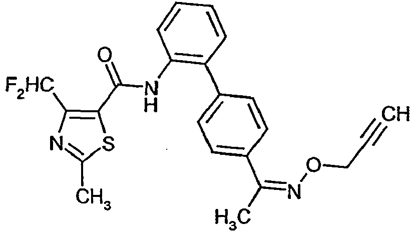 | 500 | 100 |
| 72 | 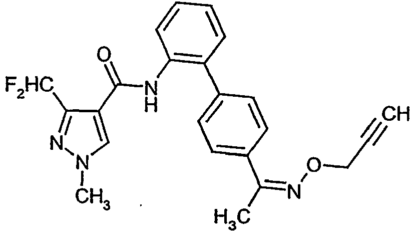 | 500 | 100 |

### Beispiel E

### Plutella-Test

| | | |
|---|---|---|
| Lösungsmittel: | 100 | Gewichtsteile Aceton |
| | 1900 | Gewichtsteile Methanol |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Methanol auf die gewünschten Konzentrationen.

Auf eine genormte Menge Kunstfutter wird eine angegebene Menge Wirkstoffzubereitung der gewünschten Konzentration pipettiert. Nachdem das Methanol verdunstet ist, werden ca. 200-300 Eier der Kohlschabe (*Plutella xylostella*) auf das Futter gegeben.

Nach der gewünschten Zeit wird die Abtötung der Eier in % bestimmt. Dabei bedeutet 100 %, dass alle Tiere abgetötet wurden; 0 % bedeutet, dass keine Tiere abgetötet wurden.

Bei diesem Test zeigen z. B. die folgenden Verbindungen der Herstellungsbeispiele gute Wirksamkeit:

**Tabelle E:**

| **Plutella-Test** | | | |
|---|---|---|---|
| Wirkstoff | | Wirkstoffkonzentration in ppm | Abtötungsgrad in % nach 7^{d} |
| 15 | 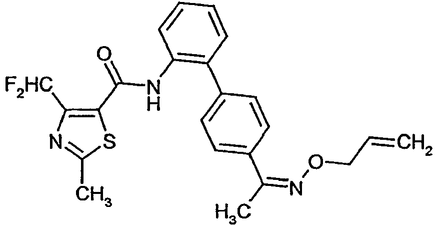 | 1000 | 95 |
| 22 | 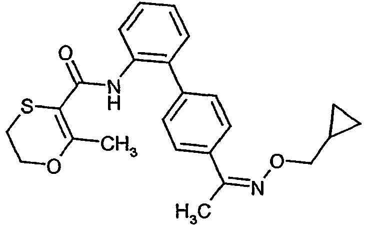 | 1000 | 95 |

## Patentansprüche

1. Biphenylcarboxamide der Formel (I) 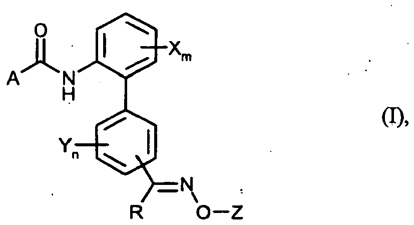 in welcher
R für Wasserstoff, C₁-C₆-Alkyl oder C₁-C₃-Halogenalkyl mit jeweils 1 bis 7 Fluor-, Chlor- und/oder Bromatomen steht,
Z für C₃-C₈-Alkenyl, C₃-C₈-Alkinyl, C₃-C₈-Halogenalkenyl, C₃-C₈-Halogenalkinyl mit jeweils 1 bis 5 Fluor-, Chlor- und/oder Bromatomen, oder (C₃-C₈-Cycloalkyl)-(C₁-C₄-alkyl) steht,
X und Y unabhängig voneinander für Halogen, Cyano, Nitro, C₁-C₈-Alkyl, C₁-C₈-Alkoxy, C₁-C₈-Alkylthio, C₁-C₆-Halogenalkyl, C₁-C₆-Halogenalkoxy oder C₁-C₆-Halogen-alkylthio mit jeweils 1 bis 13 Fluor-, Chlor- und/oder Bromatomen stehen,
m für 0, 1, 2, 3 oder 4 steht, wobei X für gleiche oder verschiedene Reste steht, wenn m für 2, 3 oder 4 steht.
n für 0, 1, 2, 3 oder 4 steht, wobei Y für gleiche oder verschiedene Reste steht, wenn n für 2, 3 oder 4 steht,
und
A für einen Rest der Formel 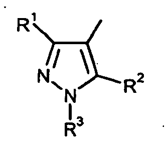 steht, worin
R¹ für Wasserstoff, Cyano, Halogen, Nitro, C₁-C₄-Alkyl, C₃-C₆-Cycloalkyl, C₁-C₄-Alkoxy, C₁-C₄-Alkylthio, Aminocarbonyl, Aminocarbonyl-C₁-C₄-alkyl oder für C₁-C₄-Halogenalkyl, C₁-C₄-Halogenakoxy, C₁-C₄-Halogenalkylthio mit jeweils 1 bis 5 Halogenatomen, steht und
R² für Wasserstoff, Halogen, Cyano, C₁-C₄-Alkyl, C₁-C₄-Alkoxy oder C₁-C₄-alkylthio steht und
R³ für Wasserstoff, C₁-C₄-Alkyl, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-C₁-C₄-alkyl, oder für C₁-C₄-Halogenalkyl, Halogen(C₁-C₄-alkylthio-C₁-C₄-alkyl), Halogen(C₁-C₄-alkoxy-C₁-C₄-alkyl) mit jeweils 1 bis 5 Halogenatomen oder für Phenyl steht,
oder
A für einen Rest der Formel 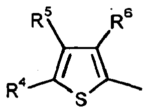 steht, worin
R⁴ und R⁵ unabhängig voneinander für Wasserstoff, Halogen, C₁-C₄-Alkyl oder C₁-C₄-Halogenalkyl mit 1 bis 5 Halogenatomen stehen und
R⁶ für Halogen, Cyano oder C₁-C₄-Alkyl, C₁-C₄-Halogenalkyl, C₁-C₄-Halogen-alkoxy mit jeweils 1 bis 5 Halogenatomen steht,
oder
A für einen Rest der Formel 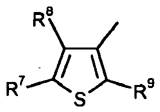 steht, worin
R⁷ und R⁸ unabhängig voneinander für Wasserstoff, Halogen, C₁-C₄-Alkyl oder C₁-C₄-Halogenalkyl mit 1 bis 5 Halogenatomen stehen und
R⁹ für Wasserstoff, Halogen oder C₁-C₄-Alkyl steht,
oder
A für einen Rest der Formel 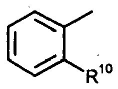 steht, worin
R¹⁰ für Wasserstoff, Halogen, Hydroxy, Cyano, C₁-C₆-Alkyl, C₁-C₄-Halogenalkyl, C₁-C₄-Halogenalkoxy, C₁-C₄-Halogenalkylthio mit jeweils 1 bis 5 Halogenatomen steht,
oder
A für einen Rest der Formel 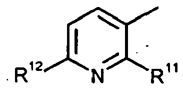 steht, worin
R¹¹ für Halogen, Hydroxy, Cyano, C₁-C₄-Alkyl, C₁-C₄-Alkoxy, C₁-C₄-Alkylthio, C₁-C₄-Halogenalkyl, C₁-C₄-Halogenakoxy, C₁-C₄-Halogenalkylthio mit jeweils 1 bis 5 Halogenatomen steht und
R¹² für Wasserstoff, Halogen, Cyano, C₁-C₄-Alkyl, C₁-C₄-Alkoxy, C₁-C₄-Al-kylthio, C₁-C₄-Alkylsulfinyl, C₁-C₄-Alkylsulfonyl oder für C₁-C₄-Halogenalkyl, C₁-C₄-Halogenalkoxy mit jeweils 1 bis 5 Halogenatomen steht,
oder
A für einen Rest der Formel 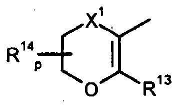 steht, worin
R¹³, für C₁-C₄-Alkyl oder für C₁-C₄-Halogenalkyl mit 1 bis 5 Halogenatomen steht,
R¹⁴ für C₁-C₄-Alkyl steht,
X¹ für S (Schwefel), für SO, SO₂ oder CH₂ steht und
p für 0, 1 oder 2 steht,
oder
A für einen Rest der Formel 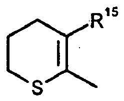 steht, worin
R¹⁵ für C₁-C₄-Alkyl oder für C₁-C₄-Halogenalkyl mit 1 bis 5 Halogenatomen steht,
oder
A für einen Rest der Formel 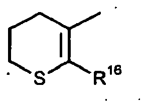 steht, worin
R¹⁶ für C₁-C₄-Alkyl oder für C₁-C₄-Halogenakyl mit 1 bis 5 Halogenatomen steht,
oder
A für einen Rest der Formel 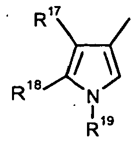 steht, worin
R" für Halogen, Cyano, C₁-C₄-Alkyl oder für C₁-C₄-Halogenalkyl mit 1 bis 5 Halogenatomen steht,
R¹⁸ für Wasserstoff, Halogen, C₁-C₄-Alkyl oder für C₁-C₄-Halogenalkyl mit 1 bis 5 Halogenatomen steht,
R¹⁹ für Wasserstoff, Cyano, C₁-C₄-Alkyl, C₁-C₄-Halogenalkyl mit 1 bis 5 Halogenatomen, C₁-C₄-Alkoxy-C₁-C₄-alkyl, Hydroxy-C₁-C₄-alkyl, C₁-C₄-Alkylsulfonyl, Di(C₁-C₄-alkyl)aminosulfonyl, C₁-C₆-Alkylcarbonyl oder für gegebenenfalls substituiertes Phenylsulfonyl oder Benzoyl steht,
oder
A für einen Rest der Formel 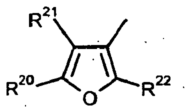 steht, worin
R²⁰ und R²¹ unabhängig voneinander für Wasserstoff, Halogen, Amino, C₁-C₄-Alkyl oder für C₁-C₄-Halogenalkyl mit 1 bis 5 Halogenatomen stehen und
R²² für Wasserstoff, Halogen, C₁-C₄-Alkyl oder für C₁-C₄-Halogenalkyl mit 1 bis 5 Halogenatomen steht,
oder
A für einen Rest der Formel 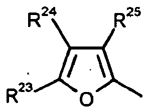 steht, worin.
R²³ und R²⁴ unabhängig voneinander für Wasserstoff, Halogen, Amino, Nitro, C₁-C₄-Alkyl oder für C₁-C₄-Halogenalkyl mit 1 bis 5 Halogenatomen stehen und
R²⁵ für Wasserstoff, Halogen, C₁-C₄-Alkyl oder für C₁-C₄-Halogenalkyl mit 1 bis 5 Halogenatomen steht,
oder
A für einen Rest der Formel 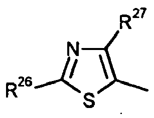 steht, worin
R²⁶ für Wasserstoff, Halogen, Amino, C₁-C₄-Alkylamino, Di-(C₁-C₄-alkyl)amino, Cyano, C₁-C₄-Alkyl oder für C₁-C₄-Halogenalkyl mit 1 bis 5 Halogenatomen steht und
R²⁷ für Halogen, C₁-C₄-Alkyl oder C₁-C₄-Halogenalkyl mit 1 bis 5 Halogenatomen steht,
oder
A für einen Rest der Formel 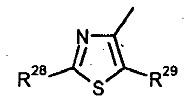 steht, worin
R²⁸ für Wasserstoff, Halogen, Amino, C₁-C₄-Alkylamino, Di-(C₁-C₄-alkyl)amino, Cyano, C₁-C₄-Alkyl oder für C₁-C₄-Halogenalkyl mit 1 bis 5 Halogenatomen steht und
R²⁹ für Halogen, C₁-C₄-Alkyl oder C₁-C₄-Halogenalkyl mit 1 bis 5 Halogenatomen steht,
oder
A für einen Rest der Formel 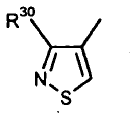 steht, worin
R³⁰ für Halogen, C₁-C₄-Alkyl oder C₁-C₄-Halogenalkyl mit 1 bis 5 Halogenatomen steht,
oder
A für einen Rest der Formel 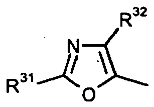 steht, worin
R³¹ für Wasserstoff oder C₁-C₄-Alkyl steht und
R³² für Halogen oder C₁-C₄-Alkyl steht,
oder
A für einen Rest der Formel 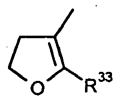 steht, worin
R³³ für C₁-C₄-Alkyl oder C₁-C₄-Halogenalkyl mit 1 bis 5 Halogenatomen steht,
oder
A für einen Rest der Formel 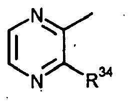 steht, worin
R³⁴ für Wasserstoff, Halogen, C₁-C₄-Alkyl oder C₁-C₂-Halogenalkyl mit 1 bis 5 Halogenatomen steht.

2. Biphenylcarboxamide der Formel (I) gemäß Anspruch 1, in welcher
R für Wasserstoff, C₁-C₄-Alkyl oder C₁-C₃-Halogenalkyl mit jeweils 1 bis 7 Fluor-, Chlor- und/oder Bromatomen steht,
Z für C₃-C₆-Alkenyl, C₃-C₆-Alkinyl, C₃-C₆-Halogenalkenyl, C₃-C₆-Halogenalkinyl mit jeweils 1 bis 5 Fluor-, Chlor- und/oder Bromatomen, oder (C₃-C₆-Cycloalkyl)-(C₁-C₄-alkyl) steht,
X und Y unabhängig voneinander für Fluor, Chlor, Brom, Cyano, Nitro, C₁-C₆-Alkyl, C₁-C₆-Alkoxy, C₁-C₆-Alkylthio, C₁-C₂-Halogenalkyl, C₁-C₂-Halogenalkoxy oder C₁-C₂-Halogenalkylthio mit jeweils 1 bis 5 Fluor-, Chlor und/oder Bromatomen stehen,
m für 0, 1, 2 oder 3 steht, wobei X für gleiche oder verschiedene Reste steht, wenn m für 2 oder 3 steht,
n für 0, 1, 2 oder 3 steht, wobei Y für gleiche oder verschiedene Reste steht, wenn n für 2 oder 3 steht,
und
A für einen Rest der Formel 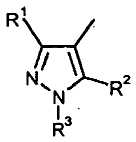 steht, worin
R¹ für Wasserstoff, Cyano, Fluor, Chlor, Brom, Iod, Methyl, Ethyl, Isopropyl, Cyclopropyl, Methoxy, Ethoxy, Methylthio, Ethylthio, Aminocarbonyl, Aminocarbonylmethyl, Aminocarbonylethyl, C₁-C₂-Halogenakyl C₁-C₂-Halogenalkoxy mit jeweils 1 bis 5 Fluor-, Chlor- und/oder Bromatomen, Trifluormethylthio oder Difluormethylthio steht,
R² für Wasserstoff, Fluor, Chlor, Brom, Iod, Methyl, Ethyl, Methoxy, Ethoxy, Methylthio oder Ethylthio steht und
R³ für Wasserstoff, Methyl, Ethyl, n-Propyl, Isopropyl, Hydroxymethyl, Hydroxyethyl, Cyclopropyl, cyclopentyl, Cyclohexyl, C₁-C₂-Halogenalkyl mit 1 bis 5 Fluor-, Chlor und/oder Bromatomen oder für Phenyl steht,
oder
A für einen Rest der Formel 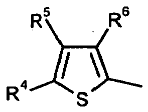 steht, worin
R⁴ und R⁵ unabhängig voneinander für Wasserstoff, Fluor, Chlor, Brom, Methyl, Ethyl oder C₁-C₂-Halogenalkyl mit 1 bis 5 Fluor-, Chlor- und/oder Bromatomen stehen und
R⁶ für Fluor, Chlor, Brom, Iod, Cyano, Methyl, Ethyl, Trifluormethyl oder C₁-C₂-Halogenalkoxy mit 1 bis 5 Fluor-, Chlor- und/oder Bromatomen steht,
oder
A für einen Rest der Formel 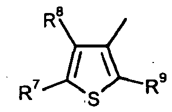 steht, worin
R⁷ und R⁸ unabhängig voneinander für Wasserstoff, Fluor, Chlor, Brom, Methyl, Ethyl oder C₁-C₂-Halogenalkyl mit 1 bis 5 Fluor-, Chlor- und/oder Bromatomen stehen und .
R⁹ für Wasserstoff, Fluor, Chlor, Brom, Methyl oder Ethyl steht,
oder
A für einen Rest der Formel 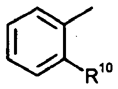 steht, worin
R¹⁰ für Wasserstoff, Fluor, Chlor, Brom, Iod, Hydroxy, Cyano, C₁-C₄-Alkyl, C₁-C₂-Halogenalkyl, C₁-C₂-Halogenalkoxy, C₁-C₂-Halogenalkylthio mit jeweils 1 bis 5 Fluor-, Chlor- und/oder Bromatomen steht,
oder
A für einen Rest der Formel 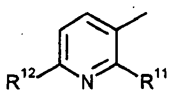 steht, worin
R¹¹ für Fluor, Chlor, Brom, Iod, Hydroxy, Cyano, C₁-C₄-Alkyl, Methoxy, Ethoxy, Methylthio, Ethylthio, C₁-C₂-Halogenalkyl, C₁-C₂-Halogenalkoxy mit jeweils 1 bis 5 Fluor-, Chlor- und/oder Bromatomen, Trifluormethylthio, Difluormethylthio steht und
R¹² für Wasserstoff, Fluor, Chlor, Brom, Iod, Cyano, C₁-C₄-Alkyl, Methoxy, Ethoxy, Methylthio, Ethylthio, C₁-C₂-Alkylsulfinyl, C₁-C₂-Akylsulfonyl, C₁-C₂-Halogenalkyl, C₁-C₂-Halogenalkoxy mit jeweils 1 bis 5 Fluor-, Chlor-und/oder Bromatomen steht,
oder
A für einen Rest der Formel 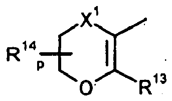 steht, worin
R¹³ für Methyl, Ethyl oder für C₁-C₂-Halogenalkyl mit 1 bis 5 Fluor-, Chlor- und/oder Bromatomen steht und
R¹⁴ für Methyl oder Ethyl steht,
X¹ für S (Schwefel), für SO, SO₂ oder CH₂ steht und
P für 0, 1 oder 2 steht,
oder
A für einen Rest der Formel 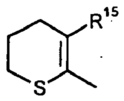 steht, worin
R¹⁵ für Methyl, Ethyl oder für C₁-C₂-Halogenalkyl mit 1 bis 5 Fluor-, Chlor-und/oder Bromatomen steht,
oder
A für einen Rest der Formel 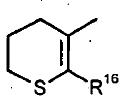 steht, worin
R¹⁶ für Methyl, Ethyl oder für C₁-C₂-Halogenalkyl mit 1 bis 5 Fluor-, Chlor-und/oder Bromatomen steht,
oder
A für einen Rest der Formel 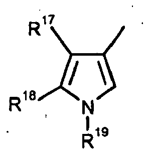 steht, worin
R¹⁷ für Fluor, Chlor, Brom, Cyano, Methyl, Ethyl, Isopropyl oder für C₁-C₂-Halogenalkyl mit 1 bis 5 Fluor-, Chlor- und/oder Bromatomen steht,
R¹⁸ für Wasserstoff, Fluor, Chlor, Brom, Methyl, Ethyl oder für C₁-C₂-Halogenalkyl mit 1 bis 5 Fluor-, Chlor- und/oder Bromatomen steht und
R¹⁹ für Wasserstoff, Methyl, Ethyl, C₁-C₂-Halogenalkyl mit 1 bis 5 Fluor-, Chlor- und/oder Bromatomen, C₁-C₂-Alkoxy-C₁-C₂-alkyl, Hydroxymethyl, Hydroxyethyl, Methylsulfonyl oder Dimethylaminosulfonyl steht,
oder
A für einen Rest der Formel 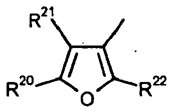 steht, worin
R²⁰ und R²¹ unabhängig voneinander für Wasserstoff, Fluor, Chlor, Brom, Amino, Methyl, Ethyl oder für C₁-C₂-Halogenalkyl mit 1 bis 5 Fluor-, Chlor-und/oder Bromatomen stehen und
R²² für Wasserstoff, Fluor, Chlor, Brom, Methyl, Ethyl oder für C₁-C₂-Halogenalkyl mit 1 bis 5 Fluor-, Chlor- und/oder Bromatomen steht,
oder
A für einen Rest der Formel 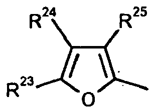 steht, worin
R²³ und R²⁴ unabhängig voneinander for Wasserstoff, Fluor, Chlor, Brom, Amino, Nitro, Methyl, Ethyl oder für C₁-C₂-Halogenalkyl mit 1 bis 5 Fluor-, Chlor-und/oder Bromatomen stehen und
R²⁵ für Wasserstoff, Fluor, Chlor, Brom, Methyl, Ethyl oder für C₁-C₂-Halogenalkyl mit 1 bis 5 Fluor-, Chlor- und/oder Bromatomen steht,
oder
A für einen Rest der Formel 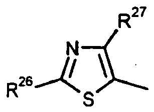 steht, worin
R²⁶ für Wasserstoff, Fluor, Chlor, Brom, Amino, C₁-C₄-Alkylamino, Di-(C₁-C₄-alkyl)amino, Cyano, Methyl, Ethyl oder für C₁-C₂-Halogenalkyl mit 1 bis 5 Fluor-, Chlor- und/oder Bromatomen steht und
R²⁷ für Fluor, Chlor, Brom, Methyl, Ethyl oder C₁-C₂-Halogenalkyl mit 1 bis 5 Fluor-, Chlor- und/oder Bromatomen steht,
oder
A für einen Rest der Formel 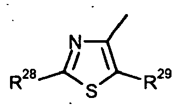 steht, worin
R²⁸ für Wasserstoff, Fluor, Chlor, Brom, Amino, C₁-C₄-Alkylamino, Di-(C₁-C₄-alkyl)amino, Cyano, Methyl, Ethyl oder für C₁-C₂-Halogenalkyl mit 1 bis 5 Fluor-, Chlor- und/oder Bromatomen steht und
R²⁹ für Fluor, Chlor, Brom, Methyl, Ethyl oder C₁-C₂-Halogenalkyl mit 1 bis 5 Fluor-, Chlor- und/oder Bromatomen steht,
oder
A für einen Rest der Formel 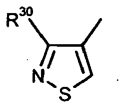 steht, worin
R³⁰ für Fluor, Chlor, Brom, Methyl, Ethyl oder C₁-C₂-Halogenalkyl mit 1 bis 5 Fluor-, Chlor- und/oder Bromatomen steht,
oder
A für einen Rest der Formel 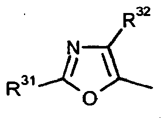 steht, worin
R³¹ für Wasserstoff, Methyl oder Ethyl steht und
R³² für Fluor, Chlor, Brom, Methyl oder Ethyl steht,
oder
A für einen Rest der Formel 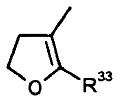 steht, worin
R³³ für Methyl, Ethyl oder C₁-C₂-Halogenalkyl mit 1 bis 5 Fluor-, Chlor-und/oder Bromatomen steht,
oder
A für einen Rest der Formel 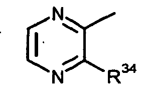 steht, worin
R³⁴ für Wasserstoff, Fluor, Chlor, Brom, Methyl, Ethyl oder Trifluormethyl steht.

3. Biphenylcarboxamide der Formel (I) gemäß Anspruch 1, in welcher
R für Wasserstoff, Methyl, Ethyl, Isopropyl, tert.-Butyl steht,
Z für Allyl, 2-Butenyl, 2-Methyl-allyl, 1-Methyl-allyl, 3-Methyl-2-butenyl, Propargyl, 2-Butinyl, 3-Butinyl, 2-Methyl-3-butinyl, 3,3-Difluorallyl, 3,3-Dichlorallyl, Cyclopropylmethyl, Cyclopentylmethyl, Cyclohexylmethyl steht,
X und Y unabhängig voneinander für Fluor, Chlor, Brom, Cyano, Nitro, Methyl, Ethyl, n-Propyl, Isopropyl, n-Butyl, sek.-Butyl, iso-Butyl, tert.-Butyl, Methoxy, Ethoxy, Methylthio, Trichlormethyl, Trifluormethyl, Difluormethyl, Difluorchlormethyl, Difluormethoxy, Trifluormethoxy, Trifluormethylthio, Difluorchlormethylthio stehen,
m für 0 oder 1 steht,
n für 0, 1 oder 2 steht, wobei Y für gleiche oder verschiedene Reste steht, wenn n für 2 steht,
und
A für einen Rest der Formel 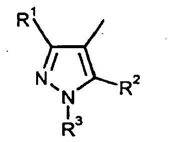 steht, worin
R¹ für Wasserstoff, Fluor, Chlor, Brom, Iod, Methyl, Ethyl, Isopropyl, Cyclopropyl, Methoxy, Ethoxy, Methylthio, Ethylthio, Monofluormethyl, Difluormethyl, Trifluormethyl, Difluorchlormethyl, Trichlormethyl, Trifluormethoxy, Trichlomethoxy, Trifluormethylthio oder Difluormethylthio steht und
R² für Wasserstoff, Fluor, Chlor, Brom, Iod, Methyl, Ethyl, Methoxy, Ethoxy, Methylthio oder Ethylthio steht und
R³ für Wasserstoff, Methyl, Ethyl, Hydroxymethyl, Hydroxyethyl, Trifluormethyl, Difluormethyl oder Phenyl steht,
oder
A für einen Rest der Formel 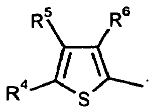 steht, worin
R⁴ und R⁵ unabhängig voneinander für Wasserstoff, Fluor, Chlor, Brom, Methyl, Ethyl, Difluormethyl, Trifluormethyl, Difluorchlormethyl oder Trichlormethyl stehen und
R⁶ für Fluor, Chlor, Brom, Cyano, Methyl, Trifluormethyl, Trifluormethoxy, Difluormethoxy, Difluorchlormethoxy oder Trichlormethoxy steht,
oder
A für einen Rest der Formel 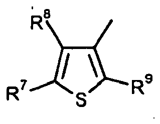 steht, worin
R⁷ und R⁸ unabhängig voneinander für Wasserstoff, Fluor, Chlor, Brom, Methyl, Ethyl, Difluormethyl, Trifluormethyl, Difluorchlormethyl oder Trichlormethyl stehen und
R⁹ für Wasserstoff, Fluor, Chlor, Brom, Methyl oder Ethyl steht,
oder
A für einen Rest der Formel 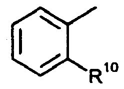 steht, worin
R¹⁰ für Wasserstoff, Fluor, Chlor, Brom, Iod, Hydroxy, Cyano, Methyl, Ethyl, n-Propyl, Isopropyl, n-Butyt, iso-Butyl, sek,-Butyl, tert,-Butyl, Difluormethyl, Trifluormethyl, Difluorchlormethyl, Trichlormethyl, Trifluormethoxy, Difluormethoxy, Difluorchlormethoxy, Trichlormethoxy, Trifluormethylthio, Difluormethylthio, Difluorchlormethylthio oder Trichlormethylthio steht,
oder
A für einen Rest der Formel 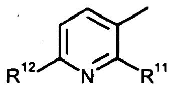 steht, worin
R¹¹ für Fluor, Chlor, Brom, Iod, Hydroxy, Cyano, Methyl, Ethyl, n-Propyl, Isopropyl, n-Butyl, iso-Butyl, sek,-Butyl, tert,-Butyl, Methoxy, Ethoxy, Methylthio, Ethylthio, Trifluormethyl, Difluormethyl, Difluorchlormethyl, Trichlormethyl, Trifluormethoxy, Difluormethoxy, Difluorchlormethoxy, Trichlormethoxy, Difluormethylthio, Trifluormethylthio steht und
R¹² für Wasserstoff, Fluor, Chlor, Brom, Iod, Cyano, Methyl, Ethyl, n-Propyl, Isopropyl, n-Butyl, iso-Butyl, sek,-Butyl, tert,-Butyl, Methoxy, Ethoxy, Methylthio, Ethylthio, Methylsulfinyl, Methylsulfonyl, Trifluormethyl, Difluormethyl, Difluorchlormethyl, Trichlormethyl, Trifluormethoxy, Difluormethoxy, Difluorchlormethoxy oder Trichlormethoxy steht,
oder
A für einen Rest der Formel 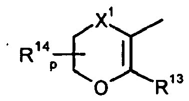 steht, worin
R¹³ für Methyl, Ethyl, Trifluormethyl, Difluormethyl, Difluorchlormethyl oder Trichlormethyl steht und
R¹⁴ für Methyl oder Ethyl steht,
X¹ für S (Schwefel), für SO, SO₂ oder CH₂ steht und
p für 0, 1 oder 2 steht,
oder
A für einen Rest der Formel 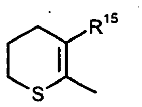 steht, worin
R¹⁵ für Methyl, Ethyl, Trifluormethyl, Difluormethyl, Difluorchlormethyl oder Trichlormethyl steht,
oder
A für einen Rest der Formel 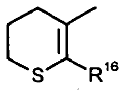 steht, worin
R¹⁶ für Methyl, Ethyl, Trifluormethyl, Difluomethyl, Difluorchlormethyl oder Trichlormethyl steht,
oder
A für einen Rest der Formel 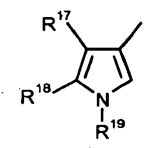 steht, worin
R¹⁷ für Fluor, Chlor, Brom, Cyano, Methyl, Ethyl, Isopropyl, Trifluormethyl, Difluormethyl, Difluorchlormethyl oder Trichlormethyl steht,
R¹⁸ für Wasserstoff, Fluor, Chlor, Brom, Methyl, Ethyl, Trifluormethyl, Difluormethyl oder Trichlormethyl steht und
R¹⁹ für Wasserstoff, Methyl, Ethyl, Trifluormethyl, Methoxymethyl, Ethoxymethyl, Hydroxymethyl oder Hydroxyethyl steht,
oder
A für einen Rest der Formel 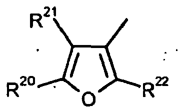 steht, worin
R²⁰ und R²¹ unabhängig voneinander für Wasserstoff, Fluor, Chlor, Brom, Methyl, Ethyl, Trifluormethyl, Difluormethyl, Difluorchlormethyl oder Trichlormethyl stehen und
R²² für Wasserstoff, Fluor, Chlor, Brom, Methyl, Ethyl, Trifluormethyl, Difluormethyl, Difluorchlormethyl oder Trichlormethyl steht,
oder
A für einen Rest der Formel 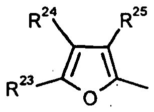 steht, worin
R²³ und R²⁴ unabhängig voneinander für Wasserstoff, Fluor; Chlor, Brom, Nitro, Methyl, Ethyl, Trifluormethyl, Difluormethyl, Difluorchlormethyl oder Trichlormethyl stehen und
R²⁵ für Wasserstoff, Fluor, Chlor, Brom, Methyl, Ethyl, Trifluormethyl, Difluormethyl, Difluorchlormethyl oder Trichlormethyl steht,
oder
A für einen Rest der Formel 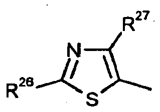 steht, worin
R²⁶ für Wasserstoff, Fluor, Chlor, Brom, Amino, Methylamino, Dimethylamino, Cyano, Methyl, Ethyl, Trifluormethyl, Difluormethyl, Difluorchlormethyl oder Trichlormethyl steht und
R²⁷ für Fluor, Chlor, Brom, Methyl, Ethyl, Trifluormethyl, Difluormethyl, Difluorchlormethyl oder Trichlormethyl steht,
oder
A für einen Rest der Formel 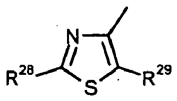 steht, worin
R²⁸ für Wasserstoff, Fluor, Chlor, Brom, Amino, Methylamino, Dimethylamino, Cyano, Methyl, Ethyl, Trifluormethyl, Difluormethyl, Difluorchlormethyl oder Trichlormethyl steht und
R²⁹ für Fluor, Chlor, Brom, Methyl, Ethyl, Trifluormethyl, Difluormethyl, Difluorchlormethyl oder Trichlormethyl steht,
oder
A für einen Rest der Formel 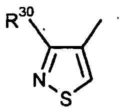 steht, worin
R³⁰ für Fluor, Chlor, Brom, Methyl, Ethyl, Trifluormethyl, Difluormethyl, Difluorchlormethyl oder Trichlormethyl steht,
oder
A für einen Rest der Formel 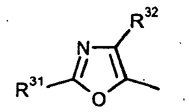 steht, worin
R³¹ für Wasserstoff, Methyl oder Ethyl steht und
R³² für Fluor, Chlor, Brom, Methyl oder Ethyl steht,
oder
A für einen Rest der Formel 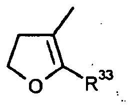 steht, worin
R³³ für Methyl, Ethyl, Trifluormethyl, Difluormethyl, Difluorchlormethyl oder Trichlormethyl steht,
oder
A für einen Rest der Formel 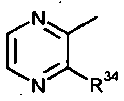 steht, worin
R³⁴ fär Wasserstoff, Fluor, Chlor, Brom, Methyl, Ethyl oder Trifluormethyl setht.

4. Biphenylcarboxamide der Formel (I-1) 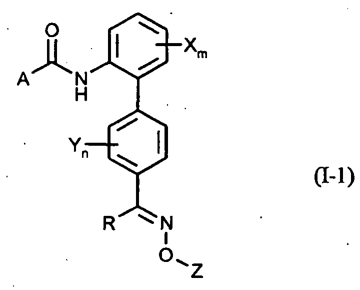 in welcher
R, Z, X, Y, m, n und A die in einem der Ansprüche 1 bis 3 angegebenen Bedeutungen haben.

5. Verfahren zur Herstellung von Biphenylcarboxamiden der Formel (I) gemäß Anspruch 1, **dadurch gekennzeichnet, dass** man
a) Carbonsäure-Derivate der Formel (II) 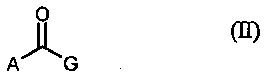 in welcher
A die in Anspruch 1 angegebenen Bedeutungen hat und
G für Halogen, Hydroxy oder C₁-C₆-Alkoxy steht,
mit Anilin-Derivaten der Formel (III) 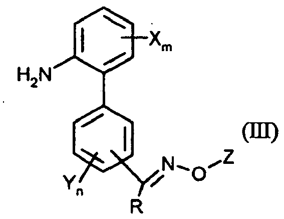 in welcher
R, Z, X, Y, m und n die in Anspruch 1 angegebenen Bedeutungen haben,
gegebenenfalls in Gegenwart eines Katalysators, gegebenenfalls in Gegenwart eines Säurebindemittels und gegebenenfalls in Gegenwart eines Verdünnungsmittels umsetzt,
oder
b) Carboxamid-Derivate der Formel (IV) 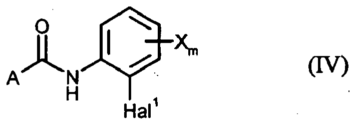 in welcher
A, X und m die in Anspruch 1 angegebenen Bedeutungen haben,
Hal¹ für Brom oder Iod steht,
mit Boronsäure-Derivaten der Formel (V) 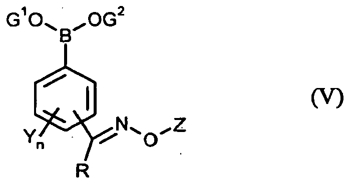 in welcher
R, Z, Y und n die in Anspruch 1 angegebenen Bedeutungen haben und
G¹ und G² jeweils für Wasserstoff oder zusammen für Tetramethylethylen stehen,
in Gegenwart eines Katalysators, gegebenenfalls in Gegenwart eines Säurebindemittels und gegebenenfalls in Gegenwart eines Verdünnungsmittels umsetzt,
oder
c) Carboxamid-Boronsäure-Derivate der Formel (VI) 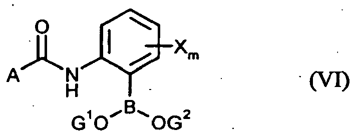 in welcher
A, X und m die in Anspruch 1 angegebenen Bedeutungen haben und
G¹ und G² jeweils für Wasserstoff oder zusammen für Tetramethylethylen stehen,
mit Phenyloxim-Derivaten der Formel (VII) 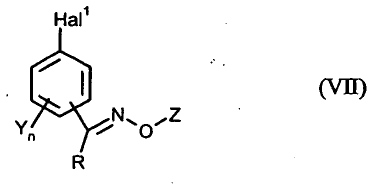 in welcher
R, Z, Y und n die in Anspruch 1 angegebenen Bedeutungen haben,
Hall¹ für Brom oder Iod steht,
in Gegenwart eines Katalysators, gegebenenfalls in Gegenwart eines Säurebindemittels und gegebenenfalls in Gegenwart eines Verdünnungsmittels umsetzt,
oder
d) Biphenylacyl-Derivate der Formel (VIII) 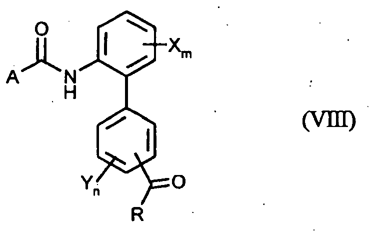 in welcher
A, R, X, Y, m und n die in Anspruch 1 angegebenen Bedeutungen haben,
mit Hydroxylamin-Derivaten der Formel (IX)
Z-O-NH₂ × HCl (IX)
in welcher
Z die in Anspruch 1 angegebenen Bedeutungen hat,
gegebenenfalls in Gegenwart eines Säurebindemittels und gegebenenfalls in Gegenwart eines Verdünnungsmittels umsetzt,
oder
e) Hydroxyimino-Derivate der Formel (I-a) 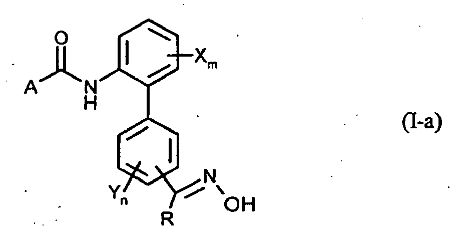 in welcher
A, R, X, Y, m und n die in Anspruch 1 angegebenen Bedeutungen haben,
mit Verbindungen der Formel (X)
Z-E (X)
in welcher
Z die in Anspruch 1 angegebenen Bedeutungen hat,
E für Chlor, Brom, Iod, Methansulfonyl oder p-Toluolsulfonyl steht,
gegebenenfalls in Gegenwart eines Säurebindemittels und gegebenenfalls in Gegenwart eines Verdünnungsmittels umsetzt,
oder
f) Carboxamid-Derivate der Formel (IV) 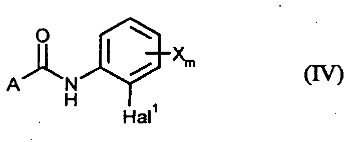 in welcher
A, X und m die in Anspruch 1 angegebenen Bedeutungen haben,
Hal¹ für Brom oder Iod steht,
mit Phenyloxim-Derivaten der Formel (VII) 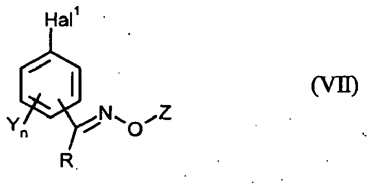 in welcher
R, Z, Y und n die in Anspruch 1 angegebenen Bedeutungen haben,
Hal¹ für Brom oder Iod steht,
in Gegenwart eines Palladium- oder Platin-Katalysators und in Gegenwart von 4,4,4',4',5,5,5',5'-Octamethyl-2,2'-bis-1,3,2-dioxaborolan, gegebenenfalls in Gegenwart eines Säurebindemittels und gegebenenfalls in Gegenwart eines Verdünnungsmittels umsetzt.

6. Mittel zur Bekämpfung unerwünschter Mikroorganismen, **gekennzeichnet durch** einen Gehalt an mindestens einem Biphenylcarboxamid der Formel (I) gemäß Anspruch 1 neben Streckmitteln und/oder oberflächenaktiven Stoffen.

7. Verwendung von Biphenylcarboxamiden der Formel (I) gemäß Anspruch 1 zur Bekämpfung unerwünschter Mikroorganismen im Pflanzenschutz und Materialschutz.

8. Verfahren zur Bekämpfung unerwünschter Mikroorganismen im Pflanzenschutz und Materialschutz, **dadurch gekennzeichnet, dass** man Biphenylcarboxamide der Formel (I) gemäß Anspruch 1 auf die Mikroorganismen und/oder deren Lebensraum ausbringt.

9. Verfahren zur Herstellung von Mitteln zur Bekämpfung unerwünschter Mikroorganismen, **dadurch gekennzeichnet, dass** man Biphenylcarboxamide der Formel (I) gemäß Anspruch 1 mit Streckmitteln und/oder oberflächenaktiven Stoffen vermischt.

10. Anilin-Derivaten der Formel (III) 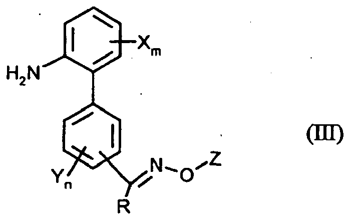 in welcher
R, Z, X, Y, m und n die in Anspruch 1 angegebenen Bedeutungen haben.

11. Boronsäure-Derivaten der Formel (V) 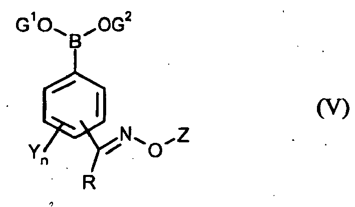 in welcher
R, Z, Y und n die in Anspruch 1 angegebenen Bedeutungen haben und
G¹ und G² jeweils für Wasserstoff oder zusammen für Tetramethylethylen stehen.

## Claims

1. Biphenylcarboxamides of the formula (I) 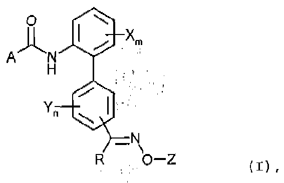 in which
R represents hydrogen, C₁-C₆-alkyl or C₁-C₃-haloalkyl having in each case 1 to 7 fluorine, chlorine and/or bromine atoms,
Z represents C₂-C₆-alkenyl, C₃-C₈-alkynyl, C₃-C₈-haloalkenyl, C₃-C₈-haloalkynyl having in each case 1 to 5 fluorine, chlorine and/or bromine atoms, or (C₃-C₈-cycloalkyl) (C₁-C₄-alkyl),
X and Y independently of one another represent halogen, cyano, nitro, C₁-C₆-alkyl, C₁-C₈-alkoxy, C₁-C₈-alkylthio, C₁-C₆-haloalkyl, C₁-C₆-haloalkoxy or Ci-Cs-haloalkylthio having in each case 1 to 13 fluorine, chlorine and/or bromine atoms,
m represents 0, 1, 2, 3 or 4, where x represents identical or different radicals if m represents 2, 3 or 4,
n represents 0, 1, 2, 3 or 4, where y represents identical or different radicals if n represents 2, 3 or 4,
and
A represents a radical of the formula 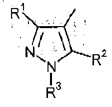 in which
R¹ represents hydrogen, cyano, halogen, nitro, C₁-C₄-alkyl, C₃-C₆-cycloalkyl, C₁-C₄-alkoxy, C₁-C₄-alkylthio, aminocarbonyl, aminocarbonyl-C₁-C₄-alkyl or represents C₁-C₄-haloalkyl, C₁-C₄-haloalkoxy, C₁-C₄-haloalkylthio having in each case 1 to 5 halogen atoms, and
R² represents hydrogen, halogen, cyano, C₁-C₄-alkyl, C₁-C₄-alkoxy or C₁-C₄-alkylthio and
R³ represents hydrogen, C₁-C₄-alkyl, hydroxy-C₁-C₄-alkyl, C₂-C₆-alkenyl, C₃-C₆-cycloalkyl, C₁-C₄-alkylthio-C₁-C₄-alkyl, C₁-C₄-alkoxy-C₁-C₄-alkyl, or represents C₁-C₄-haloalkyl, halo(C₁-C₄-alkylthio-C₁-C₄-alkyl), halo(C₁-C₄-alkoxy-C₁-C₄-alkyl) having in each case 1 to 5 halogen atoms or represents phenyl,
or
A represents a radical of the formula 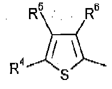 in which
R⁴ and R⁵ independently of one another represent hydrogen, halogen, C₁-C₄-alkyl or C₁-C₄-haloalkyl having 1 to 5 halogen atoms and
R⁶ represents halogen, cyano or C₁-C₄-alkyl, C₁-C₄-haloalkyl, C₁-C₄-haloalkoxy having in each case 1 to 5 halogen atoms,
or
A represents a radical of the formula 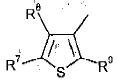 in which
R⁷ and R⁸ independently of one another represent hydrogen, halogen, C₁-C₄-alkyl or C₁-C₄-haloalkyl having 1 to 5 halogen atoms and
R⁹ represents hydrogen, halogen or C₁-C₄-alkyl,
or
A represents a radical of the formula 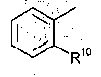 in which
R¹⁰ represents hydrogen, halogen, hydroxyl, cyano, C₁-C₆-alkyl, C₁-C₄-haloalkyl, C₁-C₄-haloalkoxy, C₁-C₄-haloalkylthio having in each case 1 to 5 halogen atoms,
or
A represents a radical of the formula 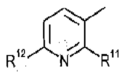 in which
R¹¹ represents halogen, hydroxyl, cyano, C₁-C₄-alkyl, C₁-C₄-alkoxy, C₁-C₄-alkylthio, C₁-C₄-haloalkyl, C₁-C₄-haloalkoxy, C₁-C₄,-haloalkylthio having in each case 1 to 5 halogen atoms and
R¹² represents hydrogen, halogen, cyano, C₁-C₄-alkyl, C₁-C₄-alkoxy, C₁-C₄-alkylthio, C₁-C₄-alkylsulfinyl, C₁-C₄-alkylsulfonyl or represents C₁-C₄-haloalkyl, C₁-C₄-haloalkoxy having in each case 1 to 5 halogen atoms,
or
A represents a radical of the formula 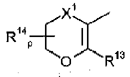 in which
R¹³ represents C₁-C₄-alkyl or represents C₁-C₄-haloalkyl having 1 to 5 halogen atoms,
R¹⁴ represents C₁-C₄-alkyl X¹ represents S (sulfur) represents SO, SO₂ or CH₂ and
p represents 0, 1 or 2,
or
A represents a radical of the formula 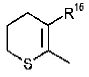 in which
R¹⁵ represents C₁-C₄-alkyl or represents C₁-C₄-haloalkyl having 1 to 5 halogen atoms,
or
A represents a radical of the formula 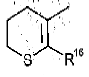 in which
R¹⁶ represents C₁-C₄-alkyl or represents C₁-C₄-haloalkyl having 1 to 5 halogen atoms,
or
A represents a radical of the formula 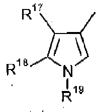 in which
R¹⁷ represents halogen, cyano, C₁-C₄-alkyl or represents C₁-C₄-haloalkyl having 1 to 5 halogen atoms, R¹⁸ represents hydrogen, halogen, C₁-C₄-alkyl or represents C₁-C₄-haloalkyl having 1 to 5 halogen atoms, R¹⁹ represents hydrogen, cyano, C₁-C₄-alkyl, C₁-C₄-haloalkyl having 1 to 5 halogen atoms, C₁-C₄-alkoxy-C₁-C₄-alkyl, hydroxy-C₁-C₄-alkyl, C₁-C₄-alkysulfonyl, di(C₁-C₄-alkyl)aminosulfonyl, C₁-C₆-alkylcarbonyl or represents optionally substituted phenylsulfonyl or benzoyl,
or
A represents a radical of the formula 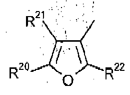 in which
R²⁰ and R²¹ independently of one another represent hydrogen, halogen, amino, C₁-C₄-alkyl or represent C₁-C₄-haloalkyl having 1 to 5 halogen atoms and
R²² represents hydrogen, halogen, C₁-C₄-alkyl or represents C₁-C₄-haloalkyl having 1 to 5 halogen atoms, or
A represents a radical of the formula 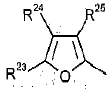 in which
R²³ and R²⁴ independently of one another represent hydrogen, halogen, amino, nitro, C₁-C₄-alkyl or represent C₁-C₄-halcalkyl having 1 to 5 halogen atoms and
R²⁵ represents hydrogen, halogen, C₁-C₄-alkyl or represents C₁-C₄-haloalkyl having 1 to 5 halogen atoms, or
A represents a radical of the formula 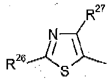 in which
R²⁶ represents hydrogen, halogen, amino, C₃-C₄-.alkylamino, di-(C₁-C₄-alkyl)amino, cyano, C₁-C₄-alkyl or represents C₁-C₄-haloalkyl having 1 to 5 halogen atoms and
R²⁷ represents halogen, C₁-C₄-alkyl or C₁-C₄-haloalkyl having 1 to 5 halogen atoms,
or
A represents a radical of the formula 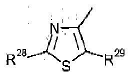 in which
R²⁸ represents hydrogen, halogen, amino, C₁-C₄-alkylamino, di-(C₁-C₄-alkyl)amino, cyano, C₁-C₄-alkyl or represents C₁-C₄-haloalkyl having 1 to 5 halogen atoms and
R²⁹ represents halogen, C₁-C₄-alkyl or C₁-C₄-haloalkyl having 1 to 5 halogen atoms,
or
A represents a radical of the formula 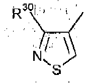 in which
R³⁰ represents halogen, C₁-C₄-alkyl, C₁-C₄-haloalkyl having 1 to 5 halogen atoms,
or
A represents a radical of the formula 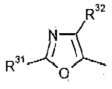 in which
R³¹ represents hydrogen or C₁-C₄-alkyl and
R³² represents halogen or C₁-C₄-alkyl,
or
A represents a radical of the formula 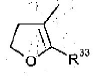 in which
R³³ represents C₁-C₄-alkyl or C₁-C₄-haloalkyl having 1 to 5 halogen atoms,
or
A represents a radical of the formula 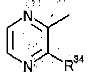 in which
R³⁴ represents hydrogen, halogen, C₁-C₄-alkyl or C₁-C₂-haloalkyl having 1 to 5 halogen atoms.

2. Biphenylcarboxamides of the formula (I) according to Claim 1 in which
R represents hydrogen, C₁-C₄-alkyl or C₁-C₃-haloalkyl having in each case 1 to 7 fluorine, chlorine and/or bromine atoms,
Z represents C₃-C₆-alkenyl, C₃-C₆-alkynyl, C₃-C₆-haloalkenyl, C₁-C₆-haloalkynyl having in each case 1 to 5 fluorine, chlorine and/or bromine atoms, or (C₃-C₆-cycloalkyl) - (C₁-C₄-alkyl),
X and Y independently of one another represent fluorine, chlorine, bromine, cyano, nitro, C₁-C₆-alkyl, C₁-C₆-alkoxy, C₁-C₆-alkylthio, C₁-C₂-haloalkyl, C₁-C₂-haloalkoxy or C₁-C₂-haloalkylthio having in each case 1 to 5 fluorine, chlorine and/or bromine atoms,
m represents 0, 1, 2 or 3, where x represents identical or different radicals if m represents 2 or 3,
n represents 0, 1, 2 or 3, where y represents identical or different radicals if m represents 2 or 3, and
A represents a radical of the formula 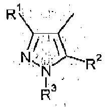 in which
R¹ represents hydrogen, cyano, fluorine, chlorine, bromine, iodine, methyl, ethyl, isopropyl, cyclopropyl, methoxy, ethoxy, methylthio, ethylthio, aminocarbonyl, aminocarbonylmethyl, aminocarbonylethyl, C₁-C₂-haloalkyl C₁-C₂-haloalkoxy having in each case 1 to 5 fluorine, chlorine and/or bromine atoms, trifluoromethylthio or difluoromethylthio,
R² represents hydrogen, fluorine, chlorine, bromine, iodine, methyl, ethyl, methoxy, ethoxy, methylthio or ethylthio and
R³ represents hydrogen, methyl, ethyl, n-propyl, isopropyl, hydroxymethyl, hydroxyethyl, cyclopropyl, cyclopentyl, cyclohexyl, C₁-C₂-haloalkyl having 1 to 5 fluorine, chlorine and/or bromine atoms or represents phenyl,
or
A represents a radical of the formula 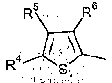 in which
R⁴ and R⁵ independently of one another represent hydrogen, fluorine, chlorine, bromine, methyl, ethyl or C₁-C₂-haloalkyl having 1 to 5 fluorine, chlorine and/or bromine atoms and
R⁶ represents fluorine, chlorine, bromine, iodine, cyano, methyl, ethyl, trifluoromethyl or C₁-C₂-haloalkoxy having 1 to 5 fluorine, chlorine and/or bromine atoms,
or
A represents a radical of the formula 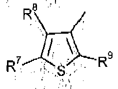 in which
R⁷ and R⁸ independently of one another represent hydrogen, fluorine, chlorine, bromine, methyl, ethyl or C₁-C₂-haloalkyl having 1 to 5 fluorine, chlorine and/or bromine atoms and
R⁹ represents hydrogen, fluorine, chlorine, bromine, methyl or ethyl,
or
A represents a radical of the formula 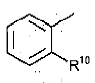 in which
R¹⁰ represents hydrogen, fluorine, chlorine, bromine, iodine, hydroxyl, cyano, C₁-C₄-alkyl, C₁-C₂-haloalkyl, C₁-C₂-haloalkoxy, C₁-C₂-haloalkylthio having in each case 1 to 5 fluorine, chlorine and/or bromine atoms,
or
A represents a radical of the formula 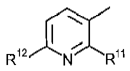 in which
R¹¹ represents fluorine, chlorine, bromine, iodine, hydroxyl, cyano, C₁-C₄-alkyl, methoxy, ethoxy, methylthio, ethylthio, C₁-C₂-haloalkyl, C₁-C₂-haloalkoxy having in each case 1 to 5 fluorine, chlorine and/or bromine atoms, trifluoromethylthio, difluoromethylthio and
R¹² represents hydrogen, fluorine, chlorine, bromine, iodine, cyano, C₁-C₄-alkyl, methoxy, ethoxy, Methylthio, ethylthio, C₁-C₂-alkylsulfinyl, C₁-C₂-alkylsulfonyl, C₁-C₂-haloalkyl, C₁-C₂-haloalkoxy having in each case 1 to 5 fluorine, chlorine and/or bromine atoms,
or
A represents a radical of the formula 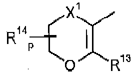 in which
R¹³ represents methyl, ethyl or represents C₁-C₂-haloalkyl having 1 to 5 fluorine, chlorine and/or bromine atoms and
R¹⁴ represents methyl or ethyl,
X¹ represents S (sulfur), represents SO, SO₂ or CH₂ and
p represents 0, 1 or 2,
or
A represents a radical of the formula 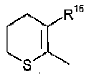 in which
R¹⁵ represents methyl, ethyl or represents C₁-C₂-haloalkyl having 1 to 5 fluorine, chlorine and/or bromine atoms,
or
A represents a radical of the formula 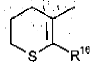 in which
R¹⁶ represents methyl, ethyl or represents C₁-C₂-haloalkyl having 1 to 5 fluorine, chlorine and/or bromine atoms,
or
A represents a radical of the formula 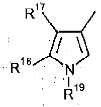 in which
R¹⁷ represents fluorine, Chlorine, bromine, cyano, methyl, ethyl, Isopropyl or represents C₁-C₂-haloalkyl having 1 to 5 fluorine, chlorine and/or bromine atoms, R¹⁸ represents hydrogen, fluorine, chlorine, bromine, methyl, ethyl or represents C₁-C₂-haloalkyl having 1 to 5 fluorine, chlorine and/or bromine atoms and
R¹⁹ represents hydrogen, methyl, ethyl, C₁-C₂-haloalkyl having 1 to 5 fluorine, chlorine and/or bromine atoms, C₁-C₂-alkoxy-C₁-C₂-alkyl, hydroxymethyl, hydroxyethyl, methylsulfonyl or dimethylaminosulfonyl,
or
A represents a radical of the formula 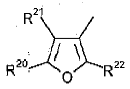 in which
R²⁰ and R²¹ independently of one another represent hydrogen, fluorine, chlorine, bromine, amino, methyl, ethyl or represent C₁-C₂-haloalkyl having 1 to 5 fluorine, chlorine and/or bromine atoms and
R²² represents hydrogen, fluorine, chlorine, bromine, methyl, ethyl or represents C₁-C₂-haloalkyl having 1 to 5 fluorine, chlorine and/or bromine atoms,
or
A represents a radical of the formula 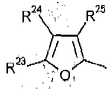 in which
R²³ and R²⁴ independently of one another represent hydrogen, fluorine, chlorine, bromine, amino, nitro, methyl, ethyl or represent C₁-C₂-haloalkyl having 1 to 5 fluorine, chlorine and/or bromine atoms and
R²⁵ represents hydrogen, fluorine, chlorine, bromine, methyl, ethyl or represents C₁-C₂-haloalkyl having 1 to 5 fluorine, chlorine and/or bromine atoms,
or
A represents a radical of the formula 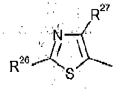 in which
R²⁶ represents hydrogen, fluorine, chlorine, bromine, amino, C₁-C₄-alkylamino, di-(C₁-C₄-alkyl)amino, cyano, methyl, ethyl or represents C₁-C₂-haloalkyl having 1 to 5 fluorine, chlorine and/or bromine atoms and
R²⁷ represents fluorine, chlorine, bromine, methyl, ethyl, C₁-C₂-haloalkyl having 1 to 5 fluorine, chlorine and/or bromine atoms,
or
A represents a radical of the formula 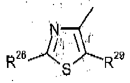 in which
R²⁸ represents hydrogen, fluorine, chlorine, bromine, amino, C₁-C₄-alkylamino, di-(C₁-C₄-alkyl)amino, cyano, methyl, ethyl or represents C₁-C₂-haloalkyl having 1 to 5 fluorine, chlorine and/or bromine atoms and
R²⁹ represents fluorine, chlorine, bromine, methyl, ethyl or C₁-C₂-haloalkyl having 1 to 5 fluorine, chlorine and/or bromine atoms,
or
A represents a radical of the formula 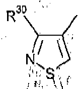 in which
R³⁰ represents fluorine, chlorine, bromine, methyl, ethyl or C₁-C₂-haloalkyl having 1 to 5 fluorine, chlorine and/or bromine atoms,
or
A represents a radical of the formula 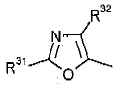 in which
R³¹ represents hydrogen, methyl or ethyl and
R³² represents fluorine, chlorine, bromine, methyl or ethyl,
or
A represents a radical of the formula 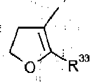 in which
R³³ represents methyl, ethyl or C₁-C₂-haloalkyl having 1 to 5 fluorine, chlorine and/or bromine atoms,
or
A represents a radical of the formula 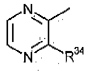 in which
R³⁴ represents hydrogen, fluorine, chlorine, bromine, methyl, ethyl or trifluoromethyl.

3. Biphenylcarboxamides of the formula (I) according to Claim 1 in which
R represents hydrogen, methyl, ethyl, isopropyl, tert-butyl,
Z represents allyl, 2-butenyl, 2-methylallyl, 1-methylallyl, 3-methyl-2-butenyl, propargyl, 2-butynyl, 3-butynyl, 2-methyl-3-butynyl, 3,3-difluoroallyl, 3,3-dichloroallyl, cyclopropylmethyl, cyclopentylmethyl, cyclohexylmethyl,
X and Y independently of one another represent fluorine, chlorine, bromine, cyano, nitro, methyl, ethyl, n-propyl, isopropyl, n-butyl, sec-butyl, isobutyl, tert-butyl, methoxy, ethoxy, methylthio, trichloromethyl, trifluormethyl, difluoromethyl, difluorochloromethyl, difluoromethoxy, trifluoromethoxy, trifluoromethylthio, difluorochloromethylthio,
m represents 0 or 1,
n represents 0, 1 or 2, where y represents identical or different radicals if n represents 2,
and
A represents a radical of the formula 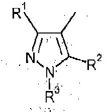 in which
R¹ represents hydrogen, fluorine, chlorine, bromine, iodine, methyl, ethyl, isopropyl, cyclopropyl, methoxy,
ethoxy, methylthio, ethylthio, monofluoromethyl, difluoromethyl, trifluoromethyl, difluorochloromethyl, trichloromethyl, trifluoromethoxy, trichloromethoxy, trifluoromethylthio or difluoromethylthio and
R² represents hydrogen, fluorine, chlorine, bromine, iodine, methyl, ethyl, methoxy, ethoxy, methylthio or ethylthio and
R³ represents hydrogen, methyl, ethyl, hydroxymethyl, hydroxyethyl, trifluoromethyl, difluoromethyl or phenyl,
or
A represents a radical of the formula 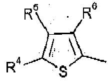 in which
R⁴ and R⁵ independently of one another represent hydrogen, fluorine, chlorine, bromine, methyl, ethyl, difluoromethyl, trifluoromethyl, difluorochloromethyl or trichloromethyl and
R⁶ represents fluorine, chlorine, bromine, cyano, methyl, trifluoromethyl, trifluoromethoxy, difluoromethoxy, difluorochloromethoxy or trichloromethoxy,
or
A represents a radical of the formula 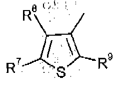 in which
R⁷ and R⁸ independently of one another represent hydrogen, fluorine, chlorine, bromine, methyl, ethyl, difluoromethyl, trifluoromethyl, difluorochloromethyl or trichloromethyl and
R⁹ represents hydrogen, fluorine, chlorine, bromine, methyl or ethyl,
or
A represents a radical of the formula 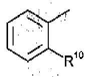 in which
R¹⁰ represents hydrogen, fluorine, chlorine, bromine, iodine, hydroxyl, cyano, methyl, ethyl, n-propyl, isopropyl, n-butyl, isobutyl, sec-butyl, tert-butyl, difluoromethyl, trifluoromethyl, difluorochloromethyl, trichloromethyl, trifluoromethoxy, difluoromethoxy, difluorochloromethoxy, trichloromethoxy, trifluoromethylthio, difluoromethylthio, difluorochloromethylthio or trichloromethylthio,
or
A represents a radical of the formula 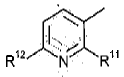 in which
R¹¹ represents fluorine, chlorine, bromine, iodine, hydroxyl, cyano, methyl, ethyl, n-propyl, isopropyl, n-butyl, isobutyl, sec-butyl, tert-butyl, methoxy, ethoxy, methylthio, ethylthio, trifluoromethyl, difluoromethyl, difluorochloromethyl, trichloromethyl, trifluoromethoxy, difluoromethoxy, difluorochloromethoxy, trichloromethoxy, difluoromethylthio, trifluoromethylthio and
R¹² represents hydrogen, fluorine, chlorine, bromine, iodine, cyano, methyl, ethyl, n-propyl, isopropyl, n-butyl, isobutyl, sec-butyl, tert-butyl, methoxy, ethoxy, methylthio, Methylthio, methylsulfinyl, methylsulfonyl, trifluoromethyl, difluoromethyl, difluorochloromethyl, trichloromethyl, trifluoromethoxy, difluoromethoxy, difluorochloromethoxy or trichloromethoxy,
or
A represents a radical of the formula 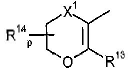 in which
R¹³ represents methyl, ethyl, trifluoromethyl, difluoromethyl, difluorochloromethyl or trichloromethyl and
R¹⁴ represents methyl or ethyl,
X¹ represents S (sulfur), represents SO, SO₂ or CH₂ and
p represents 0, 1 or 2,
or
A represents a radical of the formula 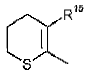 in which
R¹⁵ represents methyl, ethyl, trifluoromethyl, difluoromethyl, difluorochloromethyl or trichloromethyl,
or
A represents a radical of the formula 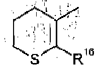 in which
R¹⁶ represents methyl, ethyl, trifluoromethyl, difluoromethyl, difluorochloromethyl or trichloromethyl,
or
A represents a radical of the formula 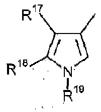 in which
R¹⁷ represents fluorine, chlorine, bromine, cyano, methyl, ethyl, isopropyl, trifluoromethyl, difluoromethyl, difluorochloromethyl or trichloromethyl,
R¹⁸ represents hydrogen, fluorine, chlorine, bromine, methyl, ethyl, trifluoromethyl, difluoromethyl or trichloromethyl and
R¹⁹ represents hydrogen, methyl, ethyl, trifluoromethyl, methoxymethyl, ethoxymethyl, hydroxymethyl or hydroxyethyl,
or
A represents a radical of the formula 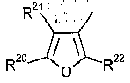 in which
R²⁰ and R²¹ independently of one another represent hydrogen, fluorine, chlorine, bromine, methyl, ethyl, trifluoromethyl, difluoromethyl, difluorochloromethyl or trichloromethyl and
R¹² represents hydrogen, fluorine, chlorine, bromine, methyl, ethyl, trifluoromethyl, difluoromethyl, difluorochloromethyl or trichloromethyl,
or
A represents a radical of the formula 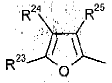 in which
R²³ and R²⁴ independently of one another represent hydrogen, fluorine, chlorine, bromine, nitro, methyl, ethyl, trifluoromethyl, difluoromethyl, difluorochloromethyl or trichloromethyl and
R²⁵ represents hydrogen, fluorine, chlorine, bromine, methyl, ethyl, trifluoromethyl, difluoromethyl, difluorochloromethyl or trichloromethyl,
or
A represents a radical of the formula 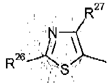 in which
R²⁶ represents hydrogen, fluorine, chlorine, bromine, amino, methylamino, dimethylamino, cyano, methyl, ethyl, trifluoromethyl, difluoromethyl, difluorochloromethyl or trichloromethyl and
R²¹ represents fluorine, chlorine, bromine, methyl, ethyl, trifluoromethyl, difluoromethyl, difluorochloromethyl or trichloromethyl,
or
A represents a radical of the formula 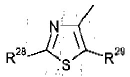 in which
R²⁸ represents hydrogen, fluorine, chlorine, bromine, amino, methylamino, dimethylamino, cyano, methyl, ethyl, trifluoromethyl, difluoromethyl, difluorochloromethyl or trichloromethyl and
R²⁹ represents fluorine, chlorine, bromine, methyl, ethyl, trifluoromethyl, difluoromethyl, difluorochloromethyl or trichloromethyl,
or
A represents a radical of the formula 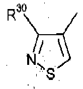 in which
R³⁰ represents fluorine, chlorine, bromine, methyl, ethyl, trifluoromethyl, difluoromethyl, difluorochloromethyl or trichloromethyl,
or
A or represents a radical of the formula 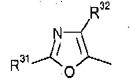 in which
R³¹ represents hydrogen, methyl or ethyl and
R³² represents fluorine, chlorine, bromine, methyl or ethyl,
or
A represents a radical of the formula 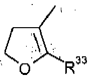 in which
R³³ represents methyl, ethyl, trifluoromethyl, difluoromethyl, difluorochloromethyl or trichloromethyl,
or
A represents a radical of the formula 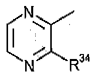 in which
R³⁴ represents hydrogen, fluorine, chlorine, bromine, methyl, ethyl or trifluoromethyl.

4. Biphenylcarboxamides of the formula (I-1) 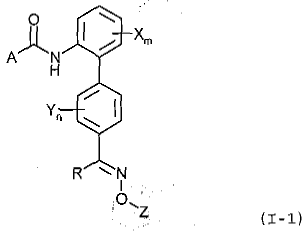 in which
R, Z, X, Y, m, n and A are as defined in any of Claims 1 to 3.

5. Process for preparing biphenylcarboxamides of the formula (I) according to Claim 1, **characterized in that**
a) carboxylic acid derivatives of the formula (II) 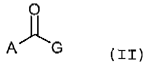 in which
A is as defined in Claim 1 and
G represents halogen, hydroxyl or C₁-C₆-alkoxy
are reacted with aniline derivatives of the formula (III) 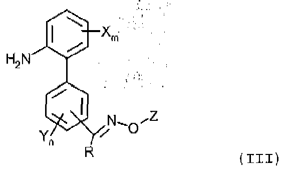 in which
R, Z, X, Y, m and n are as defined in Claim 1,
if appropriate in the presence of a catalyst, if appropriate in the presence of an acid binder and if appropriate in the presence of a diluent,
or
b) carboxamide derivatives of the formula (IV) 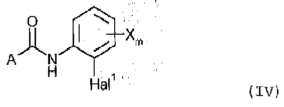 in which
A, X and m are as defined in Claim 1,
Hall represents bromine or iodine,
are reacted with boronic acid derivatives of the formula (V) 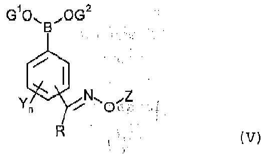 in which
R, Z, Y and n are as defined in Claim 1 and
G¹ and G² each represent hydrogen or together represent tetramethylethylene
in the presence of a catalyst, if appropriate in the presence of an acid binder and if appropriate in the presence of a diluent,
or
c) carboxamide boronic acid derivatives of the formula (VI) 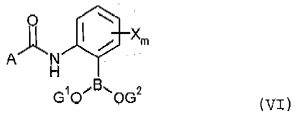 in which
A, X and m are as defined in Claim 1 and
G¹ and G² each represent hydrogen or together represent tetramethylethylene
are reacted with phenyl oxime derivatives of the formula (VII) 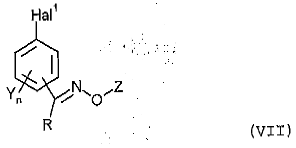 in which
R, Z, Y and n are as defined in Claim 1,
Hal¹ represents bromine or iodine,
in the presence of a catalyst, if appropriate in the presence of an acid binder and if appropriate in the presence of a diluent,
or
d) biphenylacyl derivatives of the formula (VIII) 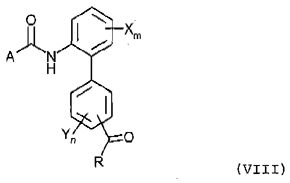 in which
A, R, X, Y, m and n are as defined in Claim 1
are reacted with hydroxylamine derivatives of the formula (IX)
Z-O-NH₂xHCl (IX)
in which
Z is as defined in Claim 1,
if appropriate in the presence of an acid binder and if appropriate in the presence of a diluent,
or
e) hydroxyimino derivatives of the formula (I-a) 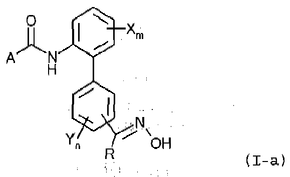 in which
A, R, X, Y, m and n are as defined in Claim 1
are reacted with compounds of the formula (X)
Z-F (X)
in which
Z is as defined in Claim 1,
E represents chlorine, bromine, iodine, methanesulfonyl or p-toluenesulfonyl,
if appropriate in the presence of an acid binder and if appropriate in the presence of a diluent,
or
f) carboxamide derivatives of the formula (IV) 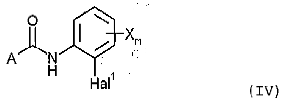
in which
A, X and m are as defined in Claim 1,
Hal¹ represents bromine or iodine,
are reacted with phenyl oxime derivatives of the formula (VII) 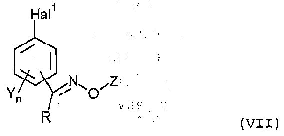 in which
R, Z, Y and n are as defined in Claim 1,
Hal¹ represents bromine or iodine
in the presence of a palladium or platinum catalyst and in the presence of 4,4,4',4',5,5,5',5'-octamethyl-2,2'-bis-1,3,2-dioxaborolane, if appropriate in the presence of an acid binder and if appropriate in the presence of a diluent.

6. Composition for controlling unwanted microorganisms, **characterized in that** it comprises at least one biphenylcarboxamide of the formula (I) according to Claim 1, in addition to extenders and/or surfactants.

7. Use of biphenylcarboxamides of the formula (I) according to Claim 1 for controlling unwanted microorganisms in crop protection and in the protection of materials.

8. Method for controlling unwanted microorganisms in crop protection and in the protection of materials, **characterized in that** biphenylcarboxamides of the formula (I) according to Claim 1 are applied to the microorganisms and/or their habitat.

9. Process for preparing compositions for controlling unwanted microorganisms, **characterized in that** biphenylcarboxamides of the formula (I) according to Claim 1 are mixed with extenders and/or surfactants.

10. Aniline derivatives of the formula (III) 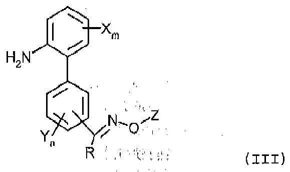 in which
R, Z, X, Y, m and n are as defined in Claim 1.

11. Boronic acid derivatives of the formula (V) 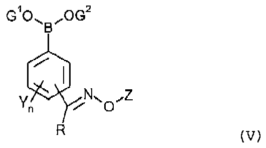 in which
R, Z, Y and n are as defined in Claim 1 and
G¹ and G² each represent hydrogen or together represent tetramethylethylene.

## Revendications

1. Biphénylcarboxamides de formule (I) 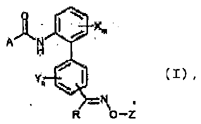 dans laquelle
R représente un atome d'hydrogène, un groupe alkyle en C₁-C₆ ou un groupe halogénoalkyle en C₁-C₃ comportant chaque fois 1 à 7 atomes de fluor, chlore et/ou brome,
Z représente un groupe alcényle en C₂-C₈, alcynyle en C₃-C₈, halogénoalcényle en C₃-C₈, halogénoalcynyle en C₃-C₈ comportant chacun de 1 à 5 atomes de fluor, chlore et/ou brome, ou un groupe [cycloalkyl (C₃-C₈)]-[alkyle(C₁-C₄)],
X et Y représentent indépendamment l'un de l'autre un atome d'halogène, un groupe cyano, nitro, alkyle en C₁-C₈, alcoxy en C₁-C₈, alkyl(C₁-C₈)thio, halogénoalkyle en C₁-C₆, halogénoalcoxy en C₁-C₆ ou halogénoalkyl(C₁-C₆)thio comportant chacun de 1 à 13 atomes de fluor, chlore et/ou brome,
m représente 0, 1, 2, 3 ou 4, X représentant des radicaux identiques ou différents lorsque m représente 2, 3 ou 4,
n représente 0, 1, 2, 3 ou 4, Y représentant des radicaux identiques ou différents lorsque n représente 2, 3 ou 4,
et
A représente un radical de formule 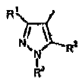 dans laquelle
R¹ représente un atome d'hydrogène ou d'halogène, un groupe cyano, nitro, alkyle en C₁-C₄, cycloalkyle en C₃-C₆, alcoxy en C₁-C₄, alkyl(C₁-C₄)thio, aminocarbonyle, aminocarbonyl-alkyle(C₁-C₄) ou un groupe halogénoalkyle en C₁-C₄, halogénoalcoxy en C₁-C₄, halogénoalkyl(C₁-C₄)thio comportant chacun de 1 à 5 atomes d'halogène, et
R² représente un atome d'hydrogène ou d'halogène, un groupe cyano, alkyle en C₁-C₄, alcoxy en C₁-C₄ ou alkyl(C₁-C₄)thio et
R³ représente un atome d'hydrogène, un groupe alkyle en C₁-C₄, hydroxyalkyle en C₁-C₄, alcényle en C₂-C₆, cycloalkyle en C₂-C₆, alkyl(C₁-C₄)thio-alkyle(C₁-C₄), alcoxy(C₁-C₄)-alkyle(C₁-C₄) ou représente un groupe halogénoalkyle en C₁-C₄, halogèno[alkyl(C₁-C₄)-thio-alkyle(C₁-C₄)], halogéno[alcoxy(C₁-C₄)-alkyle(C₁-C₄)] comportant chacun de 1 à 5 atomes d'halogène, ou un groupe phényle,
ou
A représente un radical de formule 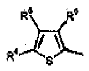 dans laquelle
R⁴ et R⁵ représentent, indépendamment l'un de l'autre, un atome d'hydrogène ou d'halogène, un groupe alkyle en C₁-C₄ ou halogénoalkyle en C₁-C₄ comportant de 1 à 5 atomes d'halogène et
R⁶ représente un atome d'halogène, un groupe cyano ou alkyle en C₁-C₄, halogénoalkyle en C₁-C₄, halogénoalcoxy en C₁-C₄ comportant chacun de 1 à 5 atomes d'halogène,
ou
A représente un radical de formule 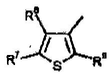 dans laquelle
R⁷ et R⁸ représentent, indépendamment l'un de l'autre, un atome d'hydrogène ou d'halogéne, un groupe alkyle en C₁-C₄ ou halogénoalkyle en C₁-C₄ comportant de 1 à 5 atomes d'halogène, et
R⁹ représente un atome d'hydrogène ou d'halogène, ou un groupe alkyl en C₁-C₄,
ou
A représente un radical de formule 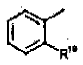 dans laquelle
R¹⁰ représente un atome d'hydrogène ou d'halogène, un groupe hydroxy, cyano, alkyle en C₁-C₆, halogénoalkyle en C₁-C₄, halogénoalcoxy en C₁-C₄, halogénoalkyl(C₁-C₄)thio comportant chacun de 1 à 5 atomes d'halogène,
ou
A représente un radical de formule 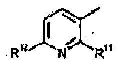 dans laquelle
R¹¹ représente un atome d'halogène, un groupe hydroxy, cyano, alkyle en C₁-C₄, alcoxy en C₁-C₄, alkyl(C₁-C₄)thio, halogénoalkyle en C₁-C₄, halogénoalcoxy en C₁-C₄, halogénoalkyl(C₁-C₄)thio comportant chacun de 1 à 5 atomes d'halogène et
R¹² représente un atome d'hydrogène ou d'halogène, un groupe cyano, alkyle en C₁-C₄, alcoxy en C₁-C₄, alkyl(C₁-C₄)thio, alkyl(C₁-C₄)sulfinyle, alkyl(C₁-C₄)sulfonyle ou halogénoalkyle en C₁-C₄, halogénoalcoxy en C₁-C₄ comportant chacun de 1 à 5 atomes d'halogène,
ou
A représente un radical de formule 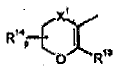 dans laquelle
R¹³ représente un groupe alkyle en C₁-C₄ ou halogénoalkyle en C₁-C₄ ayant de 1 à 5 atomes d'halogène,
R¹⁴ représente un groupe alkyle en C₁-C₄,
X¹ représente un atome de S (soufre), SO, SO₂ ou CH₂ et
p représente 0, 1 ou 2,
ou
A représente un radical de formule 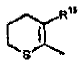 dans laquelle
R¹⁵ représente un groupe alkyle en C₁-C₄ ou halogénoalkyle en C₁-C₄ comportant de 1 à 5 atomes d'halogène,
ou
A représente un radical de formule 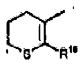 dans laquelle
R¹⁶ représente un groupe alkyle en C₁-C₄ ou halogénoalkyle en C₁-C₄ comportant de 1 à 5 atomes d'halogène,
ou
A représente un radical de formule 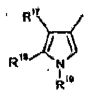 dans laquelle
R¹⁷ représente un atome d'halogène ou un groupe cyano, alkyle en C₁-C₄ ou halogénoalkyle en C₁-C₄ comportant de 1 à 5 atomes d'halogène,
R¹⁸ représente un atome d'hydrogène ou d'halogène ou un groupe alkyle en C₁-C₄ ou halogénoalkyle en C₁-C₄ comportant de 1 à 5 atomes d'halogène,
R¹⁹ représente un atome d'hydrogène, un groupe cyano, alkyle en C₁-C₄, halogénoalkyle en C₁-C₄ comportant de 1 à 5 atomes d'halogène, alcoxy(C₁-C₄)-alkyle(C₁-C₄), hydroxyalkyle(C₁-C₄), alkyl(C₁-C₄)sulfonyle, di[alkyl(C₁-C₄)]aminosulfonyle, alkyl(C₁-C₆)-carbonyle ou un groupe phénylsulfonyle ou benzoyle éventuellement substitué,
ou
A représente un radical de formule 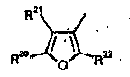 dans laquelle
R²⁰ et R²¹ représentent, indépendamment l'un de l'autre, un atome d'hydrogène ou d'halogène, un groupe amino, alkyle en C₁-C₄ ou halogénoalkyle en C₁-C₄ comportant de 1 à 5 atomes d'halogène et
R²² représente un atome d'hydrogène ou d'halogène, un groupe alkyle en C₁-C₄ ou halogénoalkyle en C₁-C₄ comportant de 1 à 5 atomes d'halogène,
ou
A représente un radical de formula 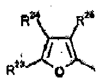 dans laquelle
R²³ et R²⁴ représentent, indépendamment l'un de l'autre, un atome d'hydrogène ou d'halogéne, un groupe amino, nitro, alkyle en C₁-C₄ ou halogénoalkyle en C₁-C₄ comportant de 1 à 5 atomes d'halogène et
R²⁶ représente un atome d'hydrogène ou d'halogène, un groupe alkyle en C₁-C₄ ou halogénoalkyle en C₁-C₄ comportant dé 1 à 5 atomes d'halogène,
ou
A représente un radical de formule 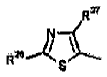 dans laquelle
R²⁶ représente un atome d'hydrogène ou d'halogène, un groupe amino, alkyl C₁-C₄)amino, di[alkyl(C₁-C₄)]amino, cyano, alkyle en C₁-C₄ ou halogénoalkyle en C₁-C₄ comportant de 1 à 5 atomes d'halogène, et
R²⁷ représente un atome, d'halogène, un groupe alkyle en C₁-C₄ ou halogénoalkyle en C₁-C₄ comportant de 1 à 5 atomes d'halogène,
ou
A représente un radical de formule 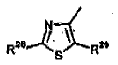 dans laquelle
R²⁸ représente un atome d'hydrogène ou d'halogène, un groupe amino, alkyl(C₁-C₄]amino, di[alkyl(C₁-C₄)]amino, cyano, alkyle en C₁-C₄ ou halogénoalkyle en C₁-C₄ comportant de 1 à 5 atomes d'halogène et
R²⁹ représente un atome d'halogène, un groupe alkyle en C₁-C₄ ou halogénoalkyle en C₁-C₄ comportant de 1 à atomes d'halogène,
ou
A représente un radical de formule 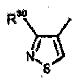 dans laquelle
R³⁰ représente un atome d'halogène, un groupe alkyle en C₁-C₄ ou halogénoalkyle en C₁-C₄ comportant de 1 à 5 atomes d'halogène,
ou
A représente un radical de formule 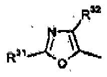 dans laquelle
R³¹ représente un atome d'hydrogène ou un groupe alkyle en C₁-C₄ et
R³² représente un atome d'halogène ou un groupe alkyle en C₁-C₄,
ou
A représente un radical de formule 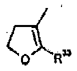 dans laquelle
R³³ représente un groupe alkyle en C₁-C₄ ou halogénoalkyle en C₁-C₄ comportant de 1 à 5 atomes d'halogène,
ou
A représente un radical de formule 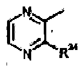 dans laquelle
R³⁴ représente un atome d'hydrogéne ou d'halogène, un groupe alkyle en C₁-C₄ ou halogénoalkyle en C₁-C₂ comportant de 1 à 5 atomes d'halogène.

2. Biphénylcarboxamides de formule (I) selon la revendication 1, dans lesquels
R représente un atome d'hydrogène, un groupe alkyle en C₁-C₄ ou un groupe halogénoalkyle en C₁-C₃ comportant chaque fois de 1 à 7 atomes de fluor, chlore et/ou brome,
Z représente un groupe alcényle en C₃-C₆, alcynyle en C₃-C₆, halogénoalcényle en C₃-C₆, halogénoalcynyle en C₃-C₆ comportant chacun de 1 à 5 atomes de fluor, chlore et/ou brome, ou un groupe [cycloalkyl(C₃-C₆)]-[alkyle(C₁-C₄)],
X et Y représentent indépendamment l'un de l'autre un atome de fluor, chlore, brome, un groupe cyano, nitro, alkyle en C₁-C₆, alcoxy en C₁-C₆, alkyl(C₁-C₆)thio, halogénoalkyle en C₁-C₂, halogénoalcoxy en C₁-C₂ ou halogénoalkyl(C₁-C₂)thio comportant chacun de 1 à 5 atomes de fluor, chlore et/ou brome,
m représente 0, 1, 2 ou 3, X représentant des radicaux identiques ou différents lorsque m représente 2 ou 3,
n représente 0, 1, 2 ou 3, Y représentant des radicaux identiques ou différents lorsque n représente 2 ou 3,
et
A représente un radical de formule 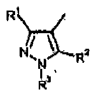 dans laquelle
R¹ représente un atome d'hydrogène, un groupe cyano, un atome de fluor, chlore, brome, iode, le groupe méthyle, éthyle, isopropyle, cyclopropyle, méthoxy, éthoxy, méthylthio, éthylthio, aminocarbonyle, aminocarbonyl-méthyle, aminocarbonyléthyle, halogénoalkyle en C₁-C₂, halogénoalcoxy en C₁-C₂ comportant chacun de 1 à 5 atomes de fluor, chlore et/ou brome, le groupe trifluorométhylthio ou difluorométhylthio,
R² représente un atome d'hydrogène ou de fluor, chlore, brome, iode, le groupe méthyle, éthyle, méthoxy, éthoxy, méthylthio ou éthylthio et
R³ représente un atome d'hydrogène, le groupe méthyle, méthyle, n-propyle, isopropyle, hydroxyméthyle, hydroxyéthyle, cyclopropyle, cyclopentyle, cyclohexyle, halogénoalkyle en C₁-C₂ comportant de 1 à 5 atomes de fluor, chlore et/ou brome ou représente le groupe phényle,
ou
A représente un radical de formule 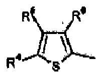 dans laquelle
R⁴ et R⁵ représentent, indépendamment l'un de l'autre, un atome d'hydrogène, de fluor, chlore, brome, le groupe méthyle, éthyle ou un groupe halogénoalkyle en C₁-C₂ comportant de 1 à 5 atomes de fluor, chlore et/ou brome et
R⁶ représente un atome de fluor, chlore, brome, iode, le groupe cyano, méthyle, éthyle, trifluorométhyle ou un groupe halogénoalcoxy en C₁-C₂ comportant de 1 à 5 atomes de fluor, chlore et/ou brome,
ou
A représente un radical de formule 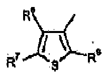 dans laquelle
R⁷ et R⁸ représentent, indépendamment l'un de l'autre, un atome d'hydrogène, de fluor, chlore, brome, le groupe méthyle, éthyle ou un groupe halogénoalkyle en C₁-C₂ comportant de 1 à 5 atomes de fluor, chlore et/ou brome et
R⁹ représente un atome d'hydrogène ou de fluor, chlore, brome, le groupe méthyle ou éthyle,
ou
A représente un radical de formule 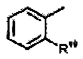 dans laquelle
R¹⁰ représente un atome d'hydrogène, de fluor, chlore, brome, iode, un groupe hydroxy, cyano, alkyle en C₁-C₄, halogénoalkyle en C₁-C₂, halogénoalcoxy en C₁-C₂, halogénoalkyl(C₁-C₂)-thio comportant chacun de 1 à 5 atomes de fluor, chlore et/ou brome,
ou
A représente un radical de formule 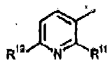 dans laquelle
R¹¹ représente un atome de fluor, chlore, brome, iode, un groupe hydroxy, cyano, alkyle en C₁-C₄, méthoxy, éthoxy, méthylthio, éthylthio, halogénoalkyle en C₁-C₂, halogénoalcoxy en C₁-C₂ comportant chacun de 1 à 5 atomes de fluor, chlore et/ou brome, trifluorométhylthio, difluorométhylthio et
R¹² représente un atome d'hydrogène, de fluor, chlore, brome, iode, un groupe cyano, alkyle en C₁-C₄, méthoxy, éthoxy, méthylthio, éthylthio, alkyl(C₁-C₂)sulfinyle, alkyl(C₁-C₂)-sulfonyle, halogénoalkyle en C₁-C₂, halogénoalcoxy en C₁-C₂ comportant chacun de 1 à 5 atomes de fluor, chlore et/ou brome,
ou
A représente un radical de formule 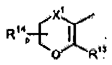 dans laquelle
R¹³ représente le groupe méthyle, éthyle ou un groupe halogénoalkyle en C₁-C₂ ayant de 1 à 5 atomes de fluor, chlore et/ou brome,
R¹⁴ représente le groupe méthyle ou éthyle,
X¹ représente un atome de S (soufre), SO, SO₂ ou CH₂ et
p représente 0, 1 ou 2,
ou
A représente un radical de formule 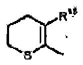 dans laquelle
R¹⁵ représente le groupe méthyle, éthyle ou un groupe halogénoalkyle en C₁-C₂ ayant de 1 à 5 atomes de fluor, chlore et/ou brome,
ou
A représente un radical de formule 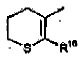 dans laquelle
R¹⁶ représente le groupe méthyle, éthyle ou un groupe halogénoalkyle en C₁-C₂ comportant de 1 à 5 atomes de fluor, chlore et/ou brome,
ou
A représente un radical de formule 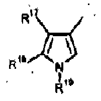 dans laquelle
R¹⁷ représente un atome de fluor, chlore, brome ou le groupe cyano, méthyle, éthyle, isopropyle ou représente un groupe halogénoalkyle en C₁-C₂ comportant de 1 à 5 atomes de fluor, chlore et/ou brome,
R¹⁸ représente un atome d'hydrogène, de fluor, chlore, brome, le groupe méthyle, éthyle ou un groupe halogénoalkyle en C₁-C₂ comportant de 1 à 5 atomes de fluor, chlore et/ou brome,
R¹⁹ représente un atome d'hydrogène, le groupe méthyle, éthyle, un groupe halogénoalkyle en C₁-C₂ comportant de 1 à 5 atomes de fluor, chlore et/ou brome, alcoxy(C₁-C₂)-alkyle(C₁-C₂), hydroxyméthyle, hydroxyéthyle, méthylsulfonyle ou diméthylaminosulfonyle,
ou
A représente un radical de formule 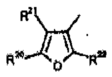 dans laquelle
R²⁰ et R²¹ représentent, indépendamment l'un de l'autre, un atome d'hydrogène, de fluor, chlore, brome, le groupe amino, méthyle, éthyle ou un groupe halogénoalkyle en C₁-C₂ comportant de 1 à 5 atomes de fluor, chlore et/ou brome et
R²² représente un atome d'hydrogène ou de fluor, chlore, brome, le groupe méthyle, éthyle ou un groupe halogénoalkyle en C₁-C₂ comportant de 1 à 5 atomes de fluor, chlore et/ou brome,
ou
A représente un radical de formule 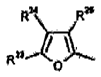 dans laquelle
R²³ et R²⁴ représentent, indépendamment l'un de l'autre, un atome d'hydrogène, de fluor, chlore, brome, le groupe amino, nitro, méthyle, éthyle ou un groupe halogénoalkyle en C₁-C₂ comportant de 1 à 5 atomes de fluor, chlore et/ou brome et
R²⁵ représente un atome d'hydrogène, de fluor, chlore, brome, le groupe méthyle, éthyle ou un groupe halogénoalkyle en C₁ -C₂ comportant de 1 à 5 atomes de fluor, chlore et/ou brome,
ou
A représente un radical de formule 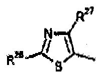 dans laquelle
R²⁶ représente un atome d'hydrogène, de fluor, chlore, brome, un groupe amino, alkyl(C₁-C₄)amino, di [alkyl (C₁-C₄)] amino, cyano, méthyle, éthyle ou un groupe halogénoalkyle en C₁-C₂ comportant de 1 à 5 atomes de fluor, chlore et/ou brome et
R²⁷ représente un atome de fluor, chlore, brome, le groupe méthyle, éthyle, ou un groupe halogenoalkyle en C₁ - C₂ comportant de 1 à 5 atomes de fluor, chlore et/ou brome,
ou
A représente un radical de formule 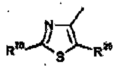 dans laquelle
R²⁸ représente un atome d'hydrogène, de fluor, chlore, brome, un groupe amino, alkyl(C₁-C₄)amino, di[alkylC₁-C₄)]amino, cyano, méthyle, éthyle ou un groupe halogénoalkyle en C₁-C₂ comportant de 1 à 5 atomes de fluor, chlore et/ou brome et
R²⁹ représente un atome de fluor, chlore, brome, le groupe méthyle, éthyle, ou un groupe halogénoalkyle en C₁-C₂ comportant de 1 à 5 atomes de fluor, chlore et/ou brome,
ou
A représente un radical de formule 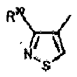 dans laquelle
R³⁰ représente un atome de fluor, chlore, brome, le groupe méthyle, éthyle ou un groupe halogénoalkyle en C₁-C₂ comportant de 1 à 5 atomes de fluor, chlore et/ou brome,
ou
A représente un radical de formule 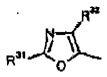 dans laquelle
R³¹ représente un atome d'hydrogène, le groupe méthyle ou éthyle et
R³² représente un atome de fluor, chlore, brome, le groupe méthyle ou éthyle,
ou
A représente un radical de formule 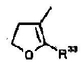 dans laquelle
R³³ représente le groupe méthyle, éthyle ou un groupe halogénoalkyle en C₁-C₂ comportant de 1 à 5 atomes de fluor, chlore et/ou brome,
ou
A représente un radical de formule 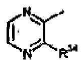 dans laquelle
R³⁴ représente un atome d'hydrogène, de fluor, chlore, brome, le groupe méthyle, éthyle ou trifluorométhyle.

3. Biphénylcarboxamides de formule (I) selon la revendication 1, dans lesquels
R représente un atome d'hydrogène, le groupe méthyle, éthyle, isopropyle, tert-butyle,
Z représente le groupe allyle, 2-butényle, 2-méthyl-allyle, 1-méthyl-allyle, 3-méthyl-2-butényle, propargyle, 2-butynyle, 3-butynyle, 2-méthyl-3-butynyle, 3,3-difluoroallyle, 3,3-dichloroallyle, cyclopropylméthyle, cyclopentylméthyle, cyclohexylméthyle,
X et Y représentent indépendamment l'un de l'autre un atome de fluor, chlore, brome, le groupe cyano, nitro, méthyle, éthyle, n-propyle, isopropyle, n-butyle, sec-butyle, isobutyle, tert-butyle, méthoxy, éthoxy, méthylthio, trichlorométhyle, trifluorométhyle, difluorométhyle, difluorochloro-méthyle, difluorométhoxy, trifluorométhoxy, trifluorométhylthio, difluorochlorométhylthio,
m représente 0 ou 1,
n représente 0, 1 ou 2, Y représentant des radicaux identiques ou différents lorsque n représente 2,
et
A représente un radical de formule 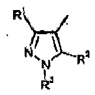 dans laquelle
R¹ représente un atome d'hydrogène ou de fluor, chlore, brome, iode, le groupe méthyle, éthyle, isopropyle, cyclopropyle, méthoxy, éthoxy, méthylthio, éthylthio, monofluoro-méthyle, difluorométhyle, trifluorométhyle, difluorochlorométhyle, trichlorométhyle, triflurométhoxy, trichlorométhoxy, trifluorométhylthio ou difluorométhylthio et
R² représente un atome d'hydrogéne ou de fluor, chlore, brome, iode, le groupe méthyle, éthyle, méthoxy, éthoxy, méthylthio ou éthylthio et
R³ représente un atome d'hydrogène, le groupe méthyle, éthyle, hydroxyméthyle, hydroxyéthyle, trifluorométhyle, difluorométhyle ou phényle,
ou
A représente un radical de formule 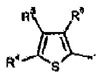 dans laquelle
R⁴ et R⁵ représentent, indépendamment l'un de l'autre, un atome d'hydrogène, de fluor, chlore, brome, le groupe méthyle, éthyle, difluorométhyle, trifluorométhyle, difluorochlorométhyle ou trichlorométhyle et
R⁶ représente un atome de fluor, chlore, brome, le groupe cyano, méthyle, trifluorométhyle, trifluorométhoxy, difluorométhoxy, difluorochlorométhoxy ou trichlorométhoxy,
ou
A représente un radical de formule 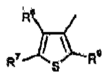 dans laquelle
R⁷ et R⁸ représentent, indépendamment l'un de l'autre, un atome d'hydrogène, de fluor, chlore, brome, le groupe méthyle, éthyle, difluorométhyle, trifluorométhyle, difluorochlorométhyle ou trichlorométhyle et
R⁹ représente un atome d'hydrogène ou de fluor, chlore, brome, le groupe méthyle ou éthyle,
ou
A représente un radical de formule 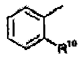 dans laquelle
R¹⁰ représente un atome d'hydrogène, de fluor, chlore, brome, iode, le groupe hydroxy, cyano, méthyle, éthyle, n-propyle, isopropyle, n-butyle, isobutyle, sec-butyle, tert-butyle, difluorométhyle, trifluorométhyle, difluorochlorométhyle, trichlorométhyle, trifluorométhoxy, difluorométhoxy, difluorochlorométhoxy, trichlorométhoxy, trifluorométhylthio, difluorométhylthio, difluorochlorométhylthio ou trichlorométhylthio,
ou
A représente un radical de formule 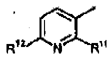 dans laquelle
R¹¹ représente un atome de fluor, chlore, brome, iode, le groupe hydroxy, cyano, méthyle, éthyle, n-propyle, isopropyle, n-butyle, isobutyle, sec-butyle, tert-butyle, méthoxy, éthoxy, méthylthio, éthylthio, trifluorométhyle, difluorométhyle, difluorochlorométhyle, trichlorométhyle, trifluorométhoxy, difluorométhoxy, difluorochlorométhoxy, trichlorométhoxy, difluorométhylthio, trifluorométhylthio et
R¹² représente un atome d'hydrogène, de fluor, chlore, brome, iode, le groupe cyano, méthyle, éthyle, n-propyle, isopropyle, n-butyle, isobutyle, sec-butyle, tert-butyle, méthoxy, éthoxy, méthylthio, éthylthio, méthyl-sulfinyle, méthylsulfonyle, trifluorométhyle, difluorométhyle, difluorochlorométhyle, trichlorométhyle, trifluorométhoxy, difluorométhoxy, difluorochlorométhoxy ou trichlorométhoxy,
ou
A représente un radical de formule 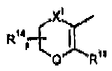 dans laquelle
R¹³ représente le groupe méthyle, éthyle, trifluorométhyle, difluorométhyle, difluorochlorométhyle ou trichlorométhyle et
R¹⁴ représente le groupe méthyle ou éthyle,
X¹ représente un atome de S (soufre), SO, SO₂ ou CH₂ et
p représente 0, 1 ou 2,
ou
A représente un radical de formule 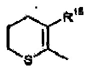 dans laquelle
R¹⁵ représente le groupe méthyle, éthyle, trifluoromethyle, difluorométhyle, difluorochlorométhyle ou trichlorométhyle,
ou
A représente un radical de formule 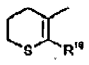 dans laquelle
R¹⁶ représente le groupe méthyle, éthyle, trifluorométhyle, difluorométhyle, difluorochlorométhyle ou trichlorométhyle,
ou
A représente un radical de formule 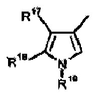 dans laquelle
R¹⁷ représente un atome de fluor, chlore, brome ou le groupe cyano, méthyle, éthyle, isopropyle, trifluorométhyle, difluorométhyle, difluorochlorométhyle ou trichloxométhyle,
R¹⁸ représente un atome d'hydrogène, de fluor, chlore, brome, le groupe méthyle, éthyle, trifluorométhyle, difluorométhyle ou trichlorométhyle et
R¹⁹ représente un atome d'hydrogène, le groupe méthyle, éthyle, trifluorométhyle, méthoxyméthyle, éthoxyméthyle, hydroxyméthyle ou hydroxyéthyle,
ou
A représente un radical de formule 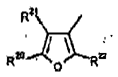 dans laquelle
R²⁰ et R²¹ représentent, indépendamment l'un de l'autre, un atome d'hydrogène, de fluor, chlore, brome, le groupe méthyle, éthyle, trifluorométhyle, difluorométhyle, difluorochlorométhyle ou trichlorométhyle et
R²² représente un atome d'hydrogène ou de fluor, chlore, brome, le groupe méthyle, éthyle, trifluorométhyle, difluorométhyle, difluorochlorométhyle ou trichlorométhyle,
ou
A représente un radical de formule 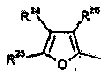 dans laquelle
R²³ et R²⁴ représentent, indépendamment l'un de l'autre, un atome d'hydrogène, de fluor, chlore, brome, le groupe nitro, méthyle, éthyle, trifluorométhyle, difluorométhyle, difluorochlorométhyle ou trichlorométhyle et
R²⁵ représente un atome d'hydrogène, de fluor, chlore, brome, le groupe méthyle, éthyle, trifluorométhyle, difluorométhyle, difluorochlorométhyle ou trichlorométhyle,
ou
A représente un radical de formule 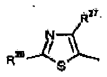 dans laquelle
R²⁶ représente un atome d'hydrogène, de fluor, chlore, brome, le groupe amino, méthylamino, diméthylamino, cyano, méthyle, éthyle, trifluorométhyle, difluorométhyle, difluorochlorométhyle ou trichlorométhyle et
R²⁷ représente un atome de fluor, chlore, brome, le groupe méthyle, éthyle, trifluoromethyle, difluorométhyle, difluorochlorométhyle ou trichlorométhyle,
ou
A représente un radical de formule 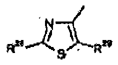 dans laquelle
R²⁸ représente un atome d'hydrogène, de fluor, chlore, brome, le groupe amino, méthylamino, diméthylamino, cyano, méthyle, éthyle, trifluorométhyle, difluorométhyle, difluorochlorométhyle ou trichlorométhyle et
R²⁹ représente un atome de fluor, chlore, brome, le groupe méthyle, éthyle, trifluorométhyle, difluorométhyle, difluorochlorométhyle ou trichlorométhyle,
ou
A représente un radical de formule 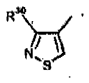 dans laquelle
R³⁰ représente un atome de fluor, chlore, brome, le groupe méthyle, éthyle, trifluorométhyle, difluorométhyle, difluorochlorométhyle ou trichlorométhyle,
ou
A représente un radical de formule 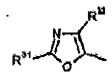 dans laquelle
R³¹ représente un atome d'hydrogène, le groupe méthyle ou éthyle et
R³² représente un atome de fluor, chlore, brome, le groupe méthyle ou éthyle,
ou
A représente un radical de formule 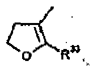 dans laquelle
R³³ représente le groupe méthyle, éthyle, trifluorométhyle, difluorométhyle, difluorochlorométhyle ou trichlorométhyle,
ou
A représente un radical de formule 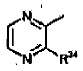 dans laquelle
R³⁴ représente un atome d'hydrogène, de fluor, chlore, brome, le groupe méthyle, éthyle ou trifluorométhyle.

4. Biphénylcarboxamides de formule (I-1) 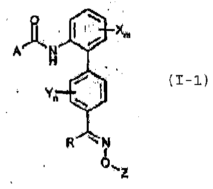 dans laquelle
R, Z, X, Y, m, n et A ont les significations indiquées dans l'une quelconque des revendications 1 à 3.

5. Procédé pour la préparation de biphénylcarboxamides de formule (I) selon la revendication 1, **caractérisé en ce que**
a) on fait réagir des dérivés d'acides carboxyliques de formule (II) 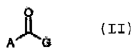 dans laquelle
A a les significations indiquées dans la revendication 1 et
G représente un atome d'halogène, un groupe hydroxy ou alcoxy en C₁-C₆,
avec des dérivés d'aniline de formule (III) 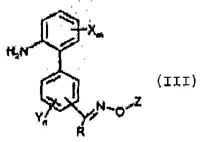 dans laquelle
R, Z, X, Y, m et n ont les significations indiquées dans la revendication 1,
éventuellement en présence d'un catalyseur, éventuellement en présence d'un capteur d'acide et éventuellement en présence d'un diluant,
ou
b) on fait réagir des dérivés de carboxamides de formule (IV) 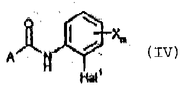 dans laquelle
A, X et m ont les significations indiquées dans la revendication 1,
Hal¹ représente le brome ou l'iode,
avec des dérivés d'acide boronique de formule (V) 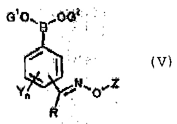 dans laquelle
R, Z, Y et n ont les significations indiquées dans la revendication 1 et
G¹ et G² représentent chacun un atome d'hydrogène ou ensemble le groupe tétraméthyléthyléne,
en présence d'un catalyseur, éventuellement en présence d'un capteur d'acide et éventuellement en présence d'un diluant,
ou
c) on fait réagir des dérivés de carboxamide-acide boronique de formule (VI) 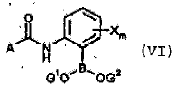 dans laquelle
A, X et m ont les significations indiquées dans la revendication 1 et
G¹ et G² représentent chacun un atome d'hydrogène ou ensemble le groupe tétraméthyléthylène,
avec des dérivés de phényloxime de formule (VII) 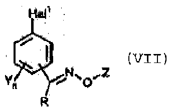 dans laquelle
R, Z, Y et n ont les significations indiquées dans la revendication 1,
Hal¹ représente le brome ou l'iode,
en présence d'un catalyseur, éventuellement en présence d'un capteur d'acide et éventuellement en présence d'un diluant,
ou
d) on fait réagir des dérivés biphénylacyle de formule (VIII) 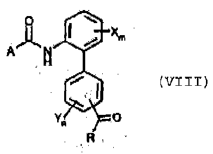 dans laquelle
A, R, X, Y, m et n ont les significations indiquées dans la revendication 1,
avec des dérivés d'hydroxylamine de formule (IX)
Z-O-NH₂ x HCl (IX)
dans laquelle
z a les significations indiquées dans la revendication 1,
éventuellement en présence d'un capteur d'acide et éventuellement en présence d'un diluant,
ou
e) on fait réagir des dérivées hydroxyimino de formule (I-a) 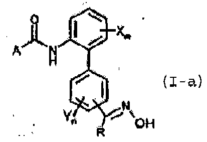 dans laquelle
A, R, X, Y, m et n ont les significations indiquées dans la revendication 1,
avec des composés de formule (X)
z-E (X)
dans laquelle
Z a les significations indiquées dans la revendication 1,
E représente un atome de chlore, brome, iode, le groupe méthanesulfonyle ou p-toluène-sulfonyle,
éventuellement en présence d'un capteur d'acide et éventuellement en présence d'un diluant,
ou
f) on fait réagir des dérivés de carboxamides de formule (IV) 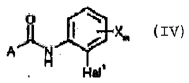 dans laquelle
A, X et m ont les significations indiquées dans la revendication 1,
Hal¹ représente le brome ou l'iode,
avec des dérivés de phényloxime de formule (VII) 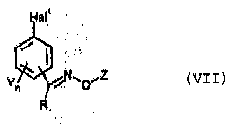 dans laquelle
R, Z, Y et n ont les significations indiquées dans la revendication 1,
Hal¹ représente le brome ou l'iode,
en présence d'un catalyseur au palladium ou au platine et en présence de 4,4,4',4',5,5,5',5'-octaméthyl-2,2'-bis-1,3,2-dioxohorolane, éventuellement en présence d'un capteur d'acide et éventuellement en présence d'un diluant.

6. Composition destinée à la lutte contre des micro-organismes indésirables, **caractérisée par** une teneur en au moins un biphénylcarboxamide de formule (I) selon la revendication 1, en plus d'excipients et/ou de substances tensioactives.

7. Utilisation de biphénylcarboxamides de formule (I) selon la revendication 1, pour la lutte contre des micro-organismes indésirables dans la protection des plantes et la protection des matériaux.

8. Procédé pour la lutte contre des micro-organismes indésirables dans la protection des plantes et la protection des matériaux, **caractérisé en ce qu'**on applique sur les micro-organismes et/ou leur habitat des biphénylcarboxamides de formule (I) selon la revendication 1.

9. Procédé pour la préparation de compositions destinées à la lutte contre des micro-organismes indésirables, **caractérisé en ce qu'**on mélange des biphénylcarboxamides de formule (I) selon la revendication 1 avec des excipients et/ou des substances tensioactives.

10. Dérivés d'aniline de formule (III) 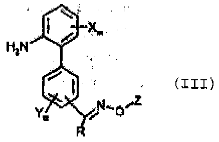 dans laquelle R, Z, X, Y, m et n ont les significations indiquées dans la revendication 1.

11. Dérivés d'acide boronique de formule (V) 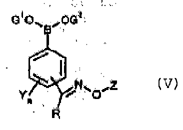 dans laquelle
R, Z, Y et n ont les significations indiquées dans la revendication 1 et
G¹ et G² représentent chacun un atome d'hydrogène ou ensemble le groupe tétraméthylène.
